(19) Europäisches Patentamt
European Patent Office
Office européen des brevets

(11) Veröffentlichungsnummer: **0 191 736 B1**

# (12) EUROPÄISCHE PATENTSCHRIFT

(45) Veröffentlichungstag der Patentschrift: **17.07.91**

(51) Int. Cl.⁵: **A01N 25/32**, C07D 215/28, //C07D215/26,C07D405/12, C07D409/12,C07D403/12, C07D213/64

(21) Anmeldenummer: **86810072.8**

(22) Anmeldetag: **10.02.86**

Die Akte enthält technische Angaben, die nach dem Eingang der Anmeldung eingereicht wurden und die nicht in dieser Patentschrift enthalten sind.

## (54) Verwendung von Chinolinderivaten zum Schützen von Kulturpflanzen.

(30) Priorität: **14.02.85 CH 682/85**
**02.12.85 CH 5132/85**

(43) Veröffentlichungstag der Anmeldung:
**20.08.86 Patentblatt 86/34**

(45) Bekanntmachung des Hinweises auf die Patenterteilung:
**17.07.91 Patentblatt 91/29**

(84) Benannte Vertragsstaaten:
**AT BE CH DE FR GB IT LI NL SE**

(56) Entgegenhaltungen:
**EP-A- 0 086 750**
**EP-A- 0 094 349**
**DE-A- 2 546 845**

(73) Patentinhaber: **CIBA-GEIGY AG**
**Klybeckstrasse 141**
**CH-4002 Basel(CH)**

(72) Erfinder: **Nyffeler, Andreas, Dr.**
**Gründlerstrasse 4**
**CH-4312 Magden(CH)**
Erfinder: **Hubele, Adolf, Dr.**
**Obere Egg 9**
**CH-4312 Magden(CH)**

**EP 0 191 736 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft die Verwendung von Chinolinderivaten zum Schützen von Kulturpflanzen gegen schädigende Wirkungen herbizid wirksamer 2-[4-(5-Chlor-3-fluorpyridin-2-yloxy)-phenoxy]-propionsäure-Derivate sowie herbizide Mittel, welche eine Kombination von Herbizid und schützendem Chinolinderivat enthält. Ferner betrifft die Erfindung auch neue Chinolinderivate.

Beim Einsatz von Herbiziden wie beispielsweise den vorstehend genannten Propionsäure-Derivaten können in Abhängigkeit von Faktoren wie beispielsweise Dosis des Herbizids und Applikationsart, Art der Kulturpflanze, Bodenbeschaffenheit und klimatischen Bedingungen, wie beispielsweise Belichtungsdauer, Temperatur und Niederschlagsmengen, die Kulturpflanzen in erheblichem Masse geschädigt werden. Insbesondere kann es zu starken Schädigungen kommen, wenn im Rahmen der Fruchtfolge nach Kulturpflanzen, die gegen die Herbizide resistent sind, andere Kulturpflanzen angebaut werden, welche keine oder nur unzureichende Resistenz gegenüber den Herbiziden aufweisen.

Es ist aus den europäischen Patentpublikationen 86 750 und 94 349 bekannt, dass sich Chinolinderivate zum Schützen von Kulturpflanzen gegen schädigende Wirkungen aggressiver Agrarchemikalien einsetzen lassen.

Es wurde nun gefunden, dass überraschenderweise ein Schutz von Kulturpflanzen gegen Schäden, welche durch herbizid wirksame 2-[4-(5-Chlor-3-fluorpyridin-2-yloxy)-phenoxy]-propionsäure-Derivate verursacht werden, durch Behandlung der Kulturpflanzen, von Teilen dieser Pflanzen oder von für den Anbau der Kulturpflanzen bestimmten Böden mit einem Safener aus einer Gruppe von Chinolinderivaten erzielt werden kann. Die herbizide Wirkung gegenüber Unkräutern und Ungräsern wird durch die Chinolinderivate nicht aufgehoben.

Chinolinderivate, welche zum Schützen von Kulturpflanzen vor schädigenden Wirkungen herbizid wirksamer 2-[4-(5-Chlor-3-fluorpyridin-2-yloxy)-phenoxy]-propionsäure Derivate geeignet sind, entsprechen der Formel I

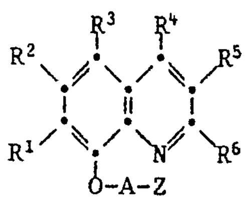

(I),

worin $R^1$, $R^2$ und $R^3$ unabhängig voneinander Wasserstoff, Halogen, Nitro, Cyano, $C_1$-$C_3$-Alkyl oder $C_1$-$C_3$-Alkoxy,

$R^4$, $R^5$ und $R^6$ unabhängig voneinander Wasserstoff, Halogen oder $C_1$-$C_3$-Alkyl,

A eine der Gruppen $-CH_2-$, $-CH_2-CH_2-$ oder $-CH(CH_3)-$ und

Z

a) Cyan oder Amidoxim, welches am Sauerstoffatom acyliert sein kann, oder

b) eine Carboxylgruppe oder ein Salz davon, eine Mercaptocarbonylgruppe oder ein Salz davon, eine Carbonsäureestergruppe, eine Carbonsäurethiolestergruppe, eine unsubstituierte oder substituierte Carbonsäureamidgruppe, ein cyclisiertes, unsubstituiertes oder substituiertes Derivat einer Carbonsäureamidgruppe oder eine Carbonsäurehydrazidgruppe, oder

A und Z zusammen einen unsubstituierten oder substituierten Tetrahydrofuran-2-on-Ring

bedeuten, unter Einschluss ihrer Säureadditionssalze und Metallkomplexe.

Unter Amidoxim ist die Gruppe $-C(NH_2)=N-OH$ zu verstehen. Das Amidoxim kann am Sauerstoffatom acyliert sein. Als am Sauerstoffatom acylierte Amidoxime kommen solche der Formel $-C(NH_2)=N-O-CO-E$ in Betracht, in denen E für $-R^7$, $-OR^8$, $-SR^9$ oder $-NR^{10}R^{11}$ steht, wobei

$R^7$ $C_1$-$C_7$-Alkyl, welches unsubstituiert oder durch Halogen oder $C_1$-$C_4$-Alkoxy substituiert ist, $C_3$-$C_6$-Cycloalkyl, $C_2$-$C_4$-Alkenyl, Phenyl, welches unsubstituiert oder durch Halogen, Nitro oder $C_1$-$C_3$-Alkyl substituiert ist, Benzyl, welches unsubstituiert oder durch Halogen, Nitro oder $C_1$-$C_3$-Alkyl substituiert ist, oder einen 5- bis 6-gliedrigen heterocyclischen Ring, welcher ein oder zwei Heteroatome aus der Gruppe N, O oder S enthält und unsubstituiert oder durch Halogen substituiert ist,

$R^8$, $R^9$ und $R^{10}$ unabhängig voneinander $C_1$-$C_8$-Alkyl, welches unsubstituiert oder durch Halogen substituiert ist, $C_2$-$C_4$-Alkenyl, $C_3$-$C_6$-Alkinyl, Phenyl, welches unsubstituiert oder durch Halogen, $C_1$-$C_3$-Alkyl, $C_1$-$C_3$-Alkoxy, Trifluormethyl oder Nitro substituiert ist, oder Benzyl, welches unsubstituiert oder durch Halogen

oder Nitro substituiert ist,
$R^{11}$ Wasserstoff, $C_1$-$C_8$-Alkyl oder $C_1$-$C_3$-Alkoxy, oder
$R^{10}$ und $R^{11}$ gemeinsam mit dem Stickstoffatom, an das sie gebunden sind, einen 5- bis 6-gliedrigen Heterocyclus, welcher noch ein weiteres Heteroatom aus der Gruppe N, O und S enthalten kann, bedeuten.

Bei $R^7$ als Heterocyclus kann es sich um gesättigte, teilgesättigte oder ungesättigte Heterocyclen handeln, wie beispielsweise Thiophen, Furan, Tetrahydrofuran und Pyrimidin.

Als Heterocyclen, welche von $R^{10}$ und $R^{11}$ gemeinsam mit dem Stickstoffatom, an das sie gebunden sind, gebildet werden, kommen gesättigte, teilgesättigte oder ungesättigte Heterocyclen in Betracht. Beispiele für solche Heterocyclen sind Pyrrolidin, Pyrrolin, Pyrrol, Imidazolidin, Imidazolin, Imidazol, Piperazin, Pyridin, Pyrimidin, Pyrazin, Thiazin, Oxazol, Thiazol und insbesondere Piperidin und Morpholin.

Unter Alkyl als Bestandteil des acylierten Amidoxims Z kommen im Rahmen der jeweils angegebenen Anzahl von Kohlenstoffatomen alle geradkettigen und alle verzweigten Alkylgruppen in Betracht.

In der Bedeutung von $R^7$ steht $C_3$-$C_6$-Cycloalkyl für Cyclopropyl, Cyclobutyl, Cyclopentyl und Cyclohexyl.

Von den $C_2$-$C_4$-Alkenyl- und $C_3$-$C_6$-Alkinylgruppen als Bestandteile des acylierten Amidoxims Z sind vor allem Vinyl, Allyl, 1-Propenyl, Methallyl und Propargyl zu erwähnen.

Für Z als Carbonsäureestergruppe oder Carbonsäurethiolestergruppe kommt ein entsprechender Säurerest in Betracht, der beispielsweise durch einen gegebenenfalls substituierten, aliphatischen Rest oder einen gegebenenfalls über einen aliphatischen Rest gebundenen und gegebenenfalls substituierten cycloaliphatischen, aromatischen oder heterocyclischen Rest verestert ist.

Als Carbonsäureesterrest bevorzugt ist der Rest $-COOR^{12}$ und als Carbonsäurethiolesterrest bevorzugt ist der Rest $-COSR^{13}$, wobei $R^{12}$ und $R^{13}$ die nachfolgend angegebenen Bedeutungen haben: gegebenenfalls substituierter Alkyl-, Alkenyl-, Alkinyl-, Cycloalkyl-, Phenyl- oder Naphthylrest oder gegebenenfalls substituierter heterocyclischer Rest. Die Reste $-COOR^{12}$ und $-COSR^{13}$ schliessen auch die freien Säuren ein, wobei $R^{12}$ und $R^{13}$ für Wasserstoff stehen, sowie die Salze davon, wobei $R^{12}$ und $R^{13}$ für ein Kation stehen. Als Salzbildner eignen sich hier besonders Metalle und organische Stickstoffbasen, vor allem quaternäre Ammoniumbasen. Hierbei kommen als zur Salzbildung geeignete Metalle Erdalkalimetalle, wie Magnesium oder Calcium, vor allem aber die Alkalimetalle in Betracht, wie Lithium und insbesondere Kalium und Natrium. Ferner sind als Salzbildner auch Uebergangsmetalle wie beispielsweise Eisen, Nickel, Kobalt, Kupfer, Zink, Chrom oder Mangan geeignet. Beispiele für zur Salzbildung geeignete Stickstoffbasen sind primäre, sekundäre oder tertiäre, aliphatische und aromatische, gegebenenfalls am Kohlenwasserstoffrest hydroxylierte Amine, wie Methylamin, Aethylamin, Propylamin, Isopropylamin, die vier isomeren Butylamine, Dimethylamin, Diäthylamin, Dipropylamin, Diisopropylamin, Di-n-butylamin, Pyrrolidin, Piperidin, Morpholin, Trimethylamin, Triäthylamin, Tripropylamin, Chinuclidin, Pyridin, Chinolin, Isochinolin sowie Methanolamin, Aethanolamin, Propanolamin, Dimethanolamin, Diäthanolamin oder Triäthanolamin. Als organische Stickstoffbasen kommen auch quaternäre Ammoniumbasen in Betracht. Beispiele für quaternäre Ammoniumbasen sind Tetraalkylammoniumkationen, in den die Alkylreste unabhängig voneinander geradkettige oder verzweigte $C_1$-$C_6$-Alkylgruppen sind, wie das Tetramethylammoniumkation, das Tetraäthylammoniumkation oder das Trimethyläthylammoniumkation, sowie weiterhin das Trimethylbenzylammoniumkation, das Triäthylbenzylammoniumkation und das Trimethyl-2-hydroxyäthylammoniumkation. Besonders bevorzugt als Salzbildner sind das Ammoniumkation und Trialkylammoniumkationen, in denen die Alkylreste unabhängig voneinander geradkettige oder verzweigte, gegebenenfalls durch eine Hydroxylgruppe substituierte $C_1$-$C_6$-Alkylgruppen, insbesondere $C_1$-$C_2$-Alkylgruppen, sind, wie beispielsweise das Trimethylammoniumkation, das Triäthylammoniumkation und das Tri-(2-hydroxyäthylen)-ammoniumkation.

Für Z als Carbonsäureamidgruppe kommt ein entsprechender Amidrest in Betracht, welcher unsubstituiert oder am Stickstoffatom mono- oder disubstituiert sein kann oder in welchem das Stickstoffatom Bestandteil eines gegebenenfalls substituierten heterocyclischen Restes ist. Als Substituenten der Amidgruppe sind beispielsweise ein gegebenenfalls substituierter und gegebenenfalls über ein Sauerstoffatom gebundener aliphatischer Rest, ein gegebenenfalls über einen aliphatischen Rest gebundener und gegebenenfalls substituierter cycloaliphatischer, aromatischer oder heterocyclischer Rest oder eine gegebenenfalls mono- oder disubstituierte Aminogruppe zu nennen.

Als Carbonsäureamidrest bevorzugt ist der Rest $-CONR^{14}R^{15}$, worin $R^{14}$ für Wasserstoff, einen gegebenenfalls substituierten Alkyl-, Alkenyl-, Alkinyl- Cycloalkyl-, Phenyl- oder Naphthylrest, einen gegebenenfalls substituierten heterocyclischen Rest oder einen Alkoxyrest, $R^{15}$ für Wasserstoff, Amino, mono- oder disubstituiertes Amino oder einen gegebenenfalls substituierten Alkyl-, Alkenyl-, Cycloalkyl- oder Phenylrest oder $R^{14}$ und $R^{15}$ gemeinsam mit dem Stickstoffatom, an das sie gebunden sind, für einen gegebenenfalls substituierten heterocyclischen Rest stehen.

Als Substituenten der organischen Reste $R^{12}$, $R^{13}$, $R^{14}$ und $R^{15}$ kommen beispielsweise Halogen, Nitro,

Cyan, Hydroxy, Alkyl, Halogenalkyl, Alkoxy, welches durch ein oder mehrere Sauerstoffatome unterbrochen sein kann, Alkylthio, Halogenalkoxy, Hydroxyalkoxy, welches durch ein oder mehrere Sauerstoffatome unterbrochen sein kann, Hydroxyalkylthio, Alkoxycarbonyl, Amino, Alkylamino, Dialkylamino, Hydroxyalkylamino, Di-(hydroxyalkyl)-amino, Aminoalkylamino, Cycloalkyl, gegebenenfalls substituiertes Phenyl, gegebenenfalls substituiertes Phenoxy oder ein gegebenenfalls substituierter heterocyclischer Rest in Betracht.

Unter heterocyclischen Resten als Bestandteile des Carbonsäureesterrestes, des Carbonsäurethiolesterrestes und des Carbonsäureamidrestes sind vorzugsweise 5- bis 6-gliedrige, gesättigte oder ungesättigte, gegebenenfalls substituierte monocyclische Heterocyclen mit 1 bis 3 Heteroatomen aus der Gruppe H, O und S zu verstehen, wie beispielsweise Furan, Tetrahydrofuran, Tetrahydropyran, Tetrahydropyrimidin, Pyridin, Piperidin, Morpholin und Imidazol.

Unter Cycloalkylresten als Bestandteile des Carbonsäureesterrestes, des Carbonsäurethiolesterrestes und des Carbonsäureamidrestes sind insbesondere solche mit 3 bis 8, vor allem 3 bis 6, Kohlenstoffatomen, zu verstehen,

Im Substituenten Z als Bestandteil des Carbonsäureesterrestes, des Carbonsäurethiolesterrestes und des Carbonsäureamidrestes vorliegende aliphatische, acyclische Reste können geradkettig oder verzweigt sein und enthalten zweckmässigerweise bis maximal 18 Kohlenstoffatome. Eine geringere Anzahl von Kohlenstoffatomen ist häufig, insbesondere bei zusammengesetzten Substituenten, von Vorteil.

Für Z als cyclisiertes Derivat einer Carbonsäureamidgruppe kommt insbesondere ein gegebenenfalls substituierter Oxazolin-2-yl-Rest, vorzugsweise ein unsubstituierter Oxazolin-2-yl-Rest, in Betracht.

A und Z können zusammen einen gegebenenfalls substituierten Tetrahydrofuran-2-on-Ring bilden, wobei der unsubstituierte Tetrahydrofuran-2-on-Ring bevorzugt ist, insbesondere der unsubstituierte Tetrahydrofuran-2-on-3-yl-Ring.

In den Verbindungen der Formel I bedeutet Halogen Fluor, Chlor, Brom und Jod, insbesondere Chlor, Brom und Jod.

Als Salzbildner für Säureadditionssalze kommen organische und anorganische Säuren in Betracht. Beispiele organischer Säuren sind Essigsäure, Trichloressigsäure, Oxalsäure, Benzolsulfonsäure und Methansulfonsäure. Beispiele anorganischer Säuren sind Chlorwasserstoffsäure, Bromwasserstoffsäure, Jodwasserstoffsäure, Schwefelsäure, Phosphorsäure, phosphorige Säure und Salpetersäure.

Als Metallkomplexbildner eignen sich beispielsweise Elemente der 3. und 4. Hauptgruppe, wie Alumium, Zinn und Blei, sowie der 1. bis 8. Nebengruppe, wie beispielsweise Chrom. Mangan, Eisen, Kobalt, Nickel, Zirkon, Zink, Kupfer, Silber und Quecksilber. Bevorzugt sind die Nebengruppenelemente der 4. Periode.

Wenn in den Verbindungen der Formel I A für $-CH(CH_3)-$ steht, der Rest Z ein asymmetrisches Kohlenstoffatom enthält oder A und Z zusammen einen Tetrahydrofuran-2-on-Ring bilden, existieren optisch isomere Verbindungen. Im Rahmen der vorliegenden Erfindung sind unter den entsprechenden Verbindungen der Formel I sowohl die optisch reinen Isomere wie auch die Isomerengemische zu verstehen. Ist bei Vorhandensein eines oder mehrerer asymmetrischer Kohlenstoffatome die Struktur nicht näher angegeben, so ist stets das Isomerengemisch gemeint.

Besonders geeignet zur erfindungsgemässen Verwendung sind Verbindungen der Formel I, in denen $R^1$, $R^2$, $R^4$, $R^5$ und $R^6$ Wasserstoff bedeuten, $R^3$ für Wasserstoff oder Chlor und der Rest -A-Z für eine Gruppe $-CH_2-COOR^{16}$ oder $-CH(CH_3)-COOR^{16}$ steht, worin $R^{16}$ $C_1$-$C_{12}$-Alkyl, $C_3$-$C_6$-Alkenyl, Phenyl-$C_1$-$C_4$-alkyl oder Phenoxy-$C_1$-$C_4$-alkyl steht.

Als bevorzugte Einzelverbindungen der Formel I zur erfindungsgemässen Verwendung sind zu nennen:
2-Chinolin-8-yloxy-essigsäureisopropylester,
2-(5-Chlorchinolin-8-yloxy)-essigsäure-n-dodecylester,
2-(5-Chlorchinolin-8-yloxy)-essigsäure-n-butylester,
2-(5-Chlorchinolin-8-yloxy)-essigsäure-n-oktylester,
2-Chinolin-8-yloxy-essigsäure-s-butylester,
2-(5-Chlorchinolin-8-yloxy)-thioessigsäure-n-oktylester,
2-(5-Chlorchinolin-8-yloxy)-essigsäure-(2-butenyl)-ester,
2-(5-Chlorchinolin-8-yloxy)-essigsäuremethallylester,
2-(5-Chlorchinolin-8-yloxy)-essigsäure-(2-isopropyloxyäthyl)-ester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäurecyclohexylester,
2-(5-Chlorchinolin-8-yloxy)-essigsäure-s-butylester,
2-(5-Chlorchinolin-8-yloxy)-essigsäure-(2-methylpentyl)-ester,
2-(5-Chlorchinolin-8-yloxy)-thioessigsäure-n-butylester,

2-(5-Chlorchinolin-8-yloxy)-essigsäure-(3,6-dioxadecyl)-ester,
2-(5-Chlorchinolin-8-yloxy)-essigsäure-(3-methoxybutyl)-ester,
2-(5-Chlorchinolin-8-yloxy)-essigsäure-(1-äthylbutyl)-ester,
2-(5-Chlorchinolin-8-yloxy)-essigsäure-(2-äthylbutyl)-ester,
2-(5-Chlorchinolin-8-yloxy)-essigsäure-(1-methylisopentyl)-ester,
2-(5-Chlorchinolin-8-yloxy)-essigsäure-n-undecylester,
2-(5-Chlorchinolin-8-yloxy)-essigsäure-(2-methylbutyl)-ester,
2-(5-Chlorchinolin-8-yloxy)-thioessigsäure-s-butylester,
2-(5-Chlorchinolin-8-yloxy)-essigsäure-(3,6-dioxaheptyl)-ester,
2-(5-Chlorchinolin-8-yloxy)-essigsäure-n-heptylester,
2-(5-Chlorchinolin-8-yloxy)-thioessigsäure-n-dodecylester,
2-(5-Chlorchinolin-8-yloxy)-thioessigsäure-n-decylester,
2-(5-Chlorchinolin-8-yloxy)-essigsäure-(1-propylpropargyl)-ester,
2-(5-Chlorchinolin-8-yloxy)-essigsäure-(1-methylisobutyl)-ester,
2-(5-Chlorchinolin-8-yloxy)-thioessigsäure-tert.butylester,
2-(5-Chlorchinolin-8-yloxy)-essigsäure-neopentylester,
2-(5-Chlorchinolin-8-yloxy)-thioessigsäure-n-propylester,
2-(5-Chlorchinolin-8-yloxy)-essigsäure-(1-methylhexyl)-ester,
2-(5-Chlorchinolin-8-yloxy)-thioessigsäureäthylester,
2-(5-Chlorchinolin-8-yloxy)-essigsäure-(2-äthylhexyl)-ester,
2-(5-Chlorchinolin-8-yloxy)-thioessigsäure-i-butylester,
2-Chinolin-8-yloxy-thioessigsäure-n-decylester,
2-(5-Chlorchinolin-8-yloxy)-essigsäure-i-pentylester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-thioessigsäure-n-hexylester,
2-(5-Chlorchinolin-8-yloxy)-essigsäure-n-hexylester,
2-(5-Chlorchinolin-8-yloxy)-thioessigsäure-i-propylester,
2-(5-Chlorchinolin-8-yloxy)-essigsäure-(1-pentylallyl)-ester,
2-(5-Chlorchinolin-8-yloxy)-essigsäure-(1-methylpentyl)-ester,
2-(5-Chlorchinolin-8-yloxy)-essigsäure-(1,1-dimethylpropargyl)-ester,
2-(5-Chlorchinolin-8-yloxy)-essigsäure-(1-äthyl-1-methylpropargyl)-ester,
2-(5-Chlorchinolin-8-yloxy)-essigsäure-n-butyloxycarbonylmethyl-ester,
2-(5-Chlorchinolin-8-yloxy)-essigsäure-(1-n-butyloxycarbonyläthyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-[1-methyl-2-(2-methylphenoxy)-äthyl]-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-[1-methyl-2-(2-isopropyl-phenoxy)-äthyl]-ester,
2-(5-Chlorchinolin-8-yloxy)-essigsäure-(1-phenylpropyl)-ester,
2-(5-Chlorchinolin-8-yloxy)-essigsäure-[1-methyl-2-(2-äthylphenoxy)-äthyl]-ester,
2-(5-Chlorchinolin-8-yloxy)-essigsäure-[1-methyl-2-(3-äthylphenoxy)-äthyl]-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenylpropyl)-ester,
2-(5-Chlorchinolin-8-yloxy)-essigsäure-[1-methyl-2-(3-methylphenoxy)-äthyl]-ester,
2-(5-Chlorchinolin-8-yloxy)-essigsäure-[1-methyl-2-(4-isopropylphenoxy)-äthyl]-ester und
2-(5-Chlorchinolin-8-yloxy)-essigsäure-[1-methyl-2-(4-methylphenoxy)-äthyl]-ester.

Hervorzuheben ist im Rahmen der vorliegenden Erfindung besonders die Verwendung von:
2-(5-Chlorchinolin-8-yloxy)-essigsäuremethallylester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäure-(1-äthylbutyl)-ester,
2-(5-Chlorchinolin-8-yloxy)-essigsäure-(1-methylisopentyl)-ester,
2-(5-Chlorchinolin-8-yloxy)-essigsäure-(1-methylhexyl)-ester,
2-(5-Chlorchinolin-8-yloxy)-essigsäure-(1-äthylpentyl)-ester,
2-(5-Chlorchinolin-8-yloxy)-essigsäure-(1-propylbutyl)-ester,

2-(5-Chlorchinolin-8-yloxy)-essigsäure-(1-pentylallyl)-ester,
2-(5-Chlorchinolin-8-yloxy)-essigsäure-(1-methylpentyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-(1-phenylpropyl)-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenylpropyl)-ester und
2-(5-Chlorchinolin-8-yloxy)-essigsäure-[1-methyl-2-(4-methylphenoxy)-äthyl]-ester.

Als ganz besonders wirksam haben sich für diesen Zweck die folgenden Verbindungen erwiesen:
2-(5-Chlorchinolin-8-yloxy)-essigsäuremethallylester,
2-(5-Chlorchinolin-8-yloxy)-essigsäure-(2-phenoxyäthyl)-ester,
2-(5-Chlorchinolin-8-yloxy)-essigsäure-(1-methylisopentyl-)ester und
2-(5-Chlorchinolin-8-yloxy)-essigsäure-(1-methylhexyl)-ester.

Folgende bisher noch nicht offenbarte Einzelwirkstoffe der Formel I wurden speziell zur Verwendung als Gegenmittel gegen die phytotoxische Wirkung von 2-[4-(5-Chlor-3-fluorpyridin-2-yloxy)-phenoxy]-propionsäure-Derivaten synthetisiert. Sie bilden einen weiteren Gegenstand der vorliegenden Erfindung:
2-(5-Chlorchinolin-8-yloxy)-essigsäure(R-1-methylisopentyl)-ester,
2-(5-Chlorchinolin-8-yloxy)-essigsäure-(S-1-methylisopentyl)-ester,
2-(5-Chlorchinolin-8-yloxy)-essigsäure-(R-1-methylhexyl)-ester,
2-(5-Chlorchinolin-8-yloxy)-essigsäure-(S-1-methylhexyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-(1-phenylpropyl)-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenylpropyl)-ester und
2-(5-Chlorchinolin-8-yloxy)-essigsäure[1-methyl-2-(4-methylphenoxy)-äthyl]-ester.

Diese neuen Verbindungen werden in an sich bekannter Weise aus einem 2-(5-Chlorchinolin-8-yloxy)-essigsäure-Derivat und einem geeigneten Alkohol durch Veresterung oder aus 5-Chlor-8-hydroxychinolin und einem geeigneten α-Halogenessigsäureester in Gegenwart einer Base hergestellt. Weitere geeignete Herstellungsverfahren sind in der publizierten Europäischen Patentanmeldung EP-A-94 349 beschrieben.

Die vorliegende Erfindung betrifft auch ein Verfahren zur Herstellung einer Verbindung aus der Gruppe
2-(5-Chlorchinolin-8-yloxy)-essigsäure-(R-1-methylisopentyl)-ester,
2-(5-Chlorchinolin-8-yloxy)-essigsäure-(S-1-methylisopentyl)-ester,
2-(5-Chlorchinolin-8-yloxy)-essigsäure-(R-1-methylhexyl)-ester,
2-(5-Chlorchinolin-8-yloxy)-essigsäure-(S-1-methylhexyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-(1-phenylpropyl)-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenylpropyl)-ester und
2-(5-Chlorchinolin-8-yloxy)-essigsäure-[1-methyl-2-(4-methylphenoxy)-äthyl]-ester,
welches dadurch charakterisiert ist, dass man 5-Chlor-8-hydroxychinolin in Gegenwart eines säurebindenden Mittels mit einer Verbindung aus der Reihe
Bromessigsäure-(R-1-methylisopentyl)-ester,
Bromessigsäure-(S-1-methylisopentyl)-ester,
Bromessigsäure-(R-1-methylhexyl)-ester,

Bromessigsäure-(S-1-methylhexyl)-ester,
Bromessigsäure-(1-methylisohexyl)-ester,
Bromessigsäure-(1-phenylisobutyl)-ester,
Bromessigsäure-(1-phenyläthyl)-ester,
Bromessigsäure-[1-methyl-2-(4-äthylphenoxy)-äthyl]-ester,
Bromessigsäure-(1-methyl-2-phenyläthyl)-ester,
Bromessigsäure(1-phenylpropyl)-ester,
Bromessigsäure-(1-methyl-2-phenoxyäthyl)-ester,
Bromessigsäure-(1-methyl-3-phenylpropyl)-ester und
Bromessigsäure-[1-methyl-2-(4-methylphenoxy)-äthyl]-ester umsetzt.

Optisch aktive Isomere der Verbindungen der Formel I können aus den Isomerengemischen durch übliche Isomerentrennungsverfahren erhalten werden. Mit Vorteil stellt man aber die reinen Isomeren durch eine gezielte Synthese aus bereits optisch aktiven Zwischenprodukten her. Beispielsweise kann man ein geeignetes 2-(5-Chlorchinolin-8-yloxy)-essigsäure-Derivat mit einem optisch aktiven Alkohol verestern oder man führt die Koppelung von 5-Chlor-8-hydroxychinolin mit einem optisch aktiven $\alpha$-Halogenessigsäureester aus.

Beispiele für erfindungsgemäss zu verwendende Verbindungen mit Schutzwirkung gegen herbizid wirksame 2-[4-(5-Chlor-3-fluorpyridin-2-yloxy)-phenoxy]-propionsäure-Derivate zeigt die nachfolgende Tabelle 1.

<u>Tabelle 1</u>:

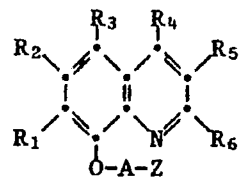

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.1 | H | H | H | H | H | H | $-CH_2-$ | $-CN$ | Smp. 118-119°C |
| 1.2 | H | H | H | H | H | H | $-CH_2-$ | 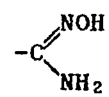 | Smp. 201-204°C (Zers.) |
| 1.3 | H | H | H | H | H | $CH_3$ | $-CH_2-$ | $-CN$ | Smp. 114-116°C |
| 1.4 | H | H | H | H | H | $CH_3$ | $-CH_2-$ | 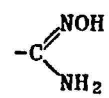 | Smp. 209-210°C (Zers.) |
| 1.5 | H | H | Cl | H | H | H | $-CH_2-$ | 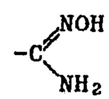 | Smp. 203-205°C (Zers.) |
| 1.6 | H | H | H | H | H | H | $-CH_2-$ | 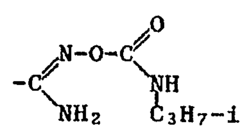 | Smp. 136-138°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.7 | H | H | Cl | H | H | H | $-CH_2-$ | -CN | Smp. 159-160°C |
| 1.8 | H | H | H | H | H | H | $-CH_2-$ | 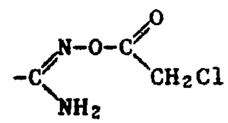 | Smp. 129-130°C |
| 1.9 | Br | H | Cl | H | H | H | $-CH_2-$ | 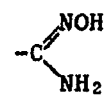 | Smp. 197-198°C (Zers.) |
| 1.10 | Br | H | Cl | H | H | H | $-CH_2-$ | -CN | Smp. 150-151°C |
| 1.11 | H | H | H | H | H | H | $-CH_2-$ | 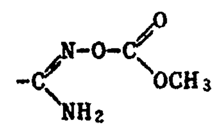 | Smp. 143-145°C |
| 1.12 | J | H | Cl | H | H | H | $-CH_2-$ | 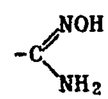 | Smp. 195-196°C (Zers.) |
| 1.13 | J | H | Cl | H | H | H | $-CH_2-$ | -CN | Smp. 150,5-152°C |
| 1.14 | Br | H | Cl | H | H | H | $-CH_2-$ | 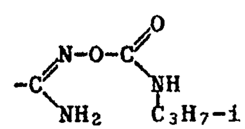 | Smp. 162-165°C |

**Tabelle 1** (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.15 | Cl | H | Cl | H | H | $CH_3$ | $-CH_2-$ | 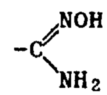 | Smp. 205-207°C (Zers.) |
| 1.16 | Cl | H | Cl | H | H | H | $-CH_2-$ | -CN | Smp. 150-152°C |
| 1.17 | J | H | Cl | H | H | H | $-CH_2-$ | 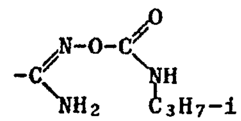 | Smp. 163-167°C |
| 1.18 | Cl | H | Cl | H | H | $CH_3$ | $-CH_2-$ | -CN | Smp. 157-158°C |
| 1.19 | Cl | H | Cl | H | H | $CH_3$ | $-CH_2-$ | 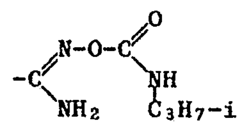 | Smp. 149-152°C |
| 1.20 | H | H | H | H | H | H | 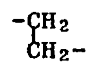 | -CN | Smp. 108-112°C |
| 1.21 | H | H | H | H | H | H | 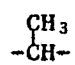 | -CN | Smp. 121-124°C |
| 1.22 | H | H | H | H | H | H | 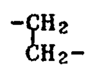 | 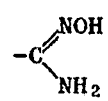 | Smp. 186-189°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.23 | H | H | Cl | H | H | H | 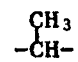 | -CN | Smp. 143-145° |
| 1.24 | H | H | H | H | H | H | 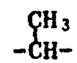 | 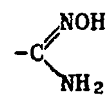 | Smp. 191-194°C (Zers.) |
| 1.25 | H | H | Cl | H | H | H | 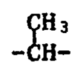 | 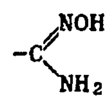 | Smp. 186-189°C (Zers.) |
| 1.26 | H | H | $NO_2$ | H | H | H | 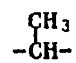 | -CN | Smp. 154-156°C |
| 1.27 | Cl | H | $NO_2$ | H | H | H | $-CH_2-$ | 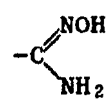 | Smp. 214-216°C (Zers.) |
| 1.28 | Cl | H | $NO_2$ | H | H | H | $-CH_2-$ | -CN | Smp. 166-169°C |
| 1.29 | H | H | H | H | H | H | $-CH_2-$ | 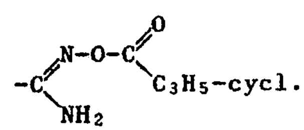 | Smp. 165-166°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.30 | H | H | H | H | H | H | $-CH_2-$ | 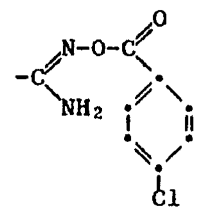 | Smp. 139-141°C |
| 1.31 | H | H | Cl | H | H | H | $-CH_2-$ | 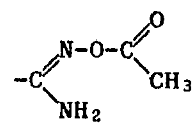 | Smp. 141-143°C |
| 1.32 | H | H | $NO_2$ | H | H | H | $-CH_2-$ | $-CN$ | Smp. 162-164°C |
| 1.33 | H | H | $NO_2$ | H | H | H | $-CH_2-$ | 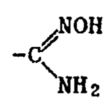 | Smp. 212-215°C (Zers.) |
| 1.34 | H | H | Cl | H | H | H | $-CH_2-$ | 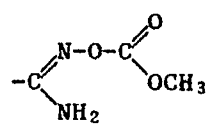 | Smp. 148-149°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.35 | H | H | Cl | H | H | H | $-CH_2-$ | 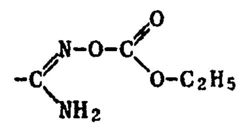 | Smp. 139-140°C |
| 1.36 | H | H | H | H | H | H | $-CH_2-$ | 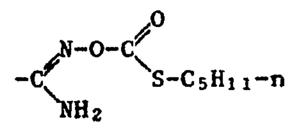 | Smp. 111-114°C |
| 1.37 | H | H | H | H | H | H | $-CH_2-$ | 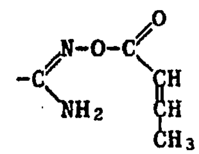 | Smp. 158-162°C |
| 1.38 | H | H | H | H | H | H | $-CH_2-$ | 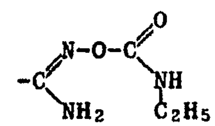 | Smp. 123-125°C |
| 1.39 | H | H | H | H | H | H | $-CH_2-$ | 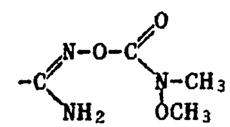 | Smp. 138-139°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.40 | H | H | H | H | H | H | $-CH_2-$ | 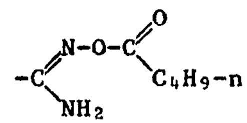 | Smp. 120-122°C |
| 1.41 | H | H | Cl | H | H | H | $-CH_2-$ | 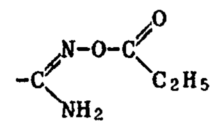 | Smp. 157-158°C (Zers.) |
| 1.42 | H | H | H | H | H | H | $-CH_2-$ | 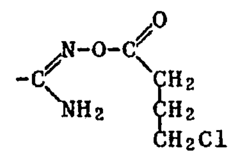 | Smp. 144-146°C |
| 1.43 | H | H | H | H | H | H | $-CH_2-$ | 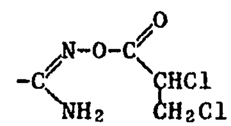 | Smp. 112-114°C |
| 1.44 | H | H | Cl | H | H | H | $-CH_2-$ | 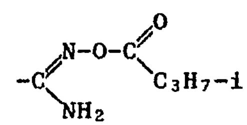 | Smp. 173-174°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.45 | H | H | H | H | H | H | $-CH_2-$ | 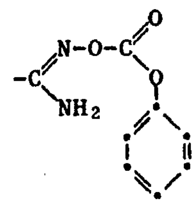 | Smp. 155-156°C |
| 1.46 | H | H | H | H | H | H | $-CH_2-$ | 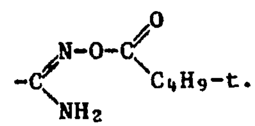 | Smp. 107-110,5°C |
| 1.47 | H | H | H | H | H | H | $-CH_2-$ | 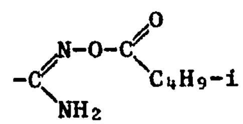 | Smp. 124-126°C |
| 1.48 | H | H | H | H | H | H | $-CH_2-$ | 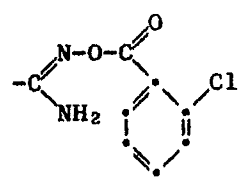 | Smp. 131-132°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.49 | H | H | H | H | H | H | $-CH_2-$ | 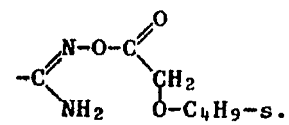 | Smp. 84-86°C |
| 1.50 | H | H | H | H | H | H | $-CH_2-$ | 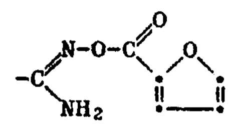 | Smp. 168-169°C |
| 1.51 | H | H | H | H | H | H | $-CH_2-$ | 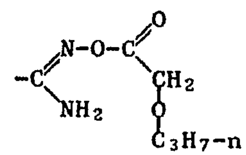 | Smp. 100-103°C |
| 1.52 | H | H | Cl | H | H | H | $-CH_2-$ | 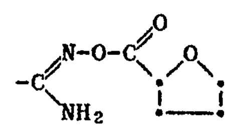 | Smp. 156-157°C (Zers.) |
| 1.53 | H | H | H | H | H | H | $-CH_2-$ | 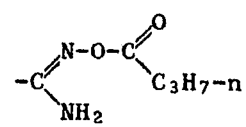 | Smp. 82-85°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.54 | H | H | H | H | H | H | $-CH_2-$ | 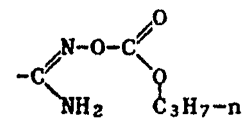 | Smp. 144-147°C |
| 1.55 | H | H | H | H | H | H | $-CH_2-$ | 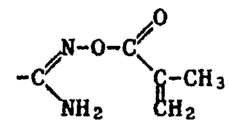 | Smp. 128-130°C |
| 1.56 | H | H | H | H | H | H | $-CH_2-$ | 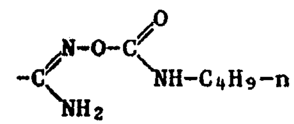 | Smp. 104-107°C |
| 1.57 | H | H | H | H | H | H | $-CH_2-$ | 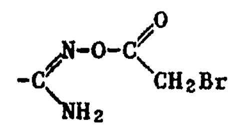 | Smp. 132-134°C |
| 1.58 | H | H | H | H | H | H | $-CH_2-$ | 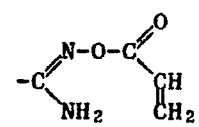 | Smp. 138-140°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.59 | H | H | H | H | H | H | $-CH_2-$ | 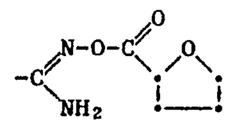 | Smp. 129–131°C |
| 1.60 | H | H | H | H | H | H | $-CH_2-$ | 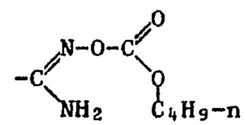 | Smp. 121–123°C |
| 1.61 | H | H | H | H | H | H | $-CH_2-$ | 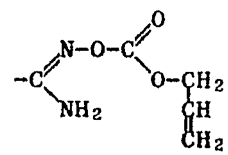 | Smp. 123–125°C |
| 1.62 | H | H | H | H | H | H | $-CH_2-$ | 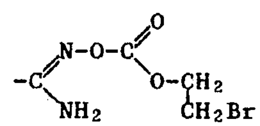 | Smp.127–128°C (Zers.) |
| 1.63 | H | H | Cl | H | H | H | $-CH_2-$ | 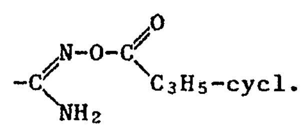 | Smp. 173–175°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.64 | H | H | H | H | H | H | $-CH_2-$ | 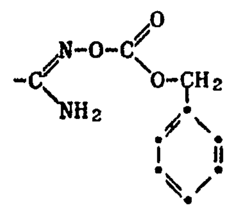 | Smp. 135-137°C |
| 1.65 | H | H | Cl | H | H | H | $-CH_2-$ | 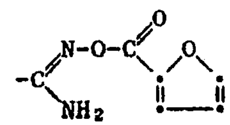 | Smp.191-192°C (Zers.) |
| 1.66 | H | H | H | H | H | H | $-CH_2-$ | 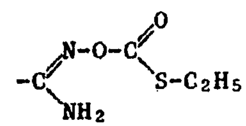 | Smp. 120-121°C |
| 1.67 | H | H | H | H | H | H | $-CH_2-$ | 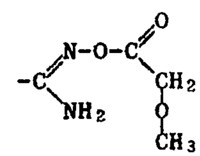 | Smp. 118-120°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.68 | H | H | Cl | H | H | H | $-CH_2-$ | 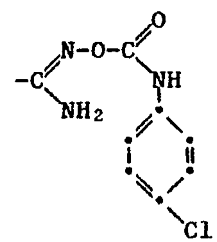 | Smp.191-192°C (Zers.) |
| 1.69 | H | H | H | H | H | H | $-CH_2-$ | 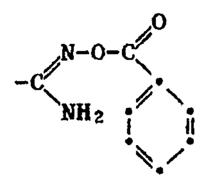 | Smp. 158-159°C |
| 1.70 | H | H | H | H | H | H | $-CH_2-$ | 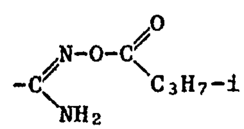 | Smp. 115-117,5°C |
| 1.71 | H | H | H | H | H | H | $-CH_2-$ | 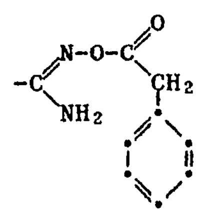 | Smp. 140-142°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.72 | H | H | H | H | H | H | $-CH_2-$ | 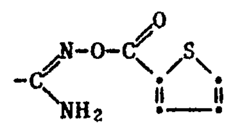 | Smp. 164-165°C |
| 1.73 | H | H | H | H | H | H | $-CH_2-$ | 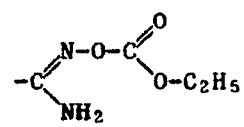 | Smp. 129-132°C |
| 1.74 | H | H | H | H | H | H | $-CH_2-$ | 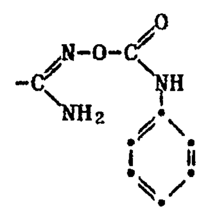 | Smp. 155-157,5°C |
| 1.75 | H | H | H | H | H | H | $-CH_2-$ | 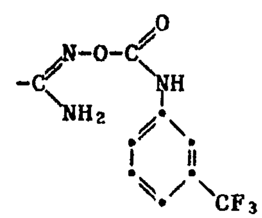 | Smp. 158-160°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.76 | H | H | Cl | H | H | H | $-CH_2-$ | 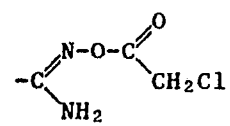 | Smp. 155-158°C (Zers.) |
| 1.77 | H | H | H | H | H | H | $-CH_2-$ | 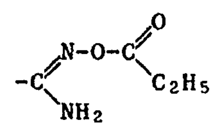 | Smp. 144-146°C |
| 1.78 | H | H | H | H | H | H | $-CH_2-$ | 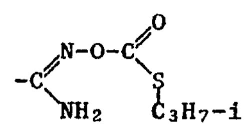 | Smp. 123-124°C |
| 1.79 | H | H | H | H | H | H | $-CH_2-$ | 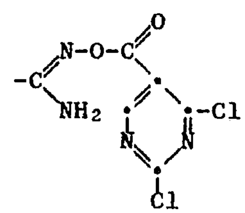 | Smp. 173-176°C (Zers.) |
| 1.80 | H | H | H | H | H | H | $-CH_2-$ | 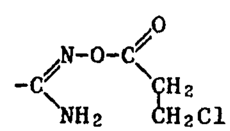 | Smp. 134-136°C (Zers.) |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.81 | H | H | H | H | H | H | $-CH_2-$ | 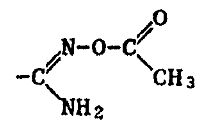 | Smp. 100-102°C |
| 1.82 | H | H | H | H | H | H | $-CH_2-$ | 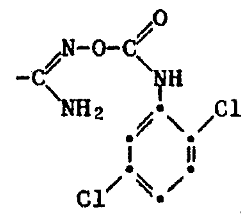 | Smp. 197-199°C |
| 1.83 | H | H | H | H | H | H | $-CH_2-$ | 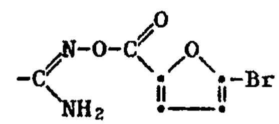 | Smp. 170-171°C |
| 1.84 | Cl | H | Cl | H | H | H | $-CH-$<br>$CH_3$ | $-COOCH_3$ | Smp. 65-66°C |
| 1.85 | H | H | H | H | H | H | $-CH-$<br>$CH_3$ | $-COOCH_3$ | Smp. 70-72°C |
| 1.86 | H | H | H | H | H | H | $-CH_2-$ | $-COOH \cdot H_2O$ | Smp. 184-185°C |
| 1.87 | H | H | H | H | H | H | $-CH_2-$ | $-COOCH_2CH_2OCH_3$ | Smp. 80-82°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.88 | H | H | H | H | H | H | $-CH_2-$ | $-COOCH_3$ | Smp. 46,5-67,0°C |
| 1.89 | H | H | H | H | H | H | $-CH_2-$ | $-COOC_2H_5 \cdot H_2O$ | Smp. 56-59°C |
| 1.90 | H | H | H | H | H | H | $-CH(CH_3)-$ | $-CONH(CH_2)_3OC_2H_5$ | Smp. 54-56°C |
| 1.91 | H | H | H | H | H | H | $-CH(CH_3)-$ | $-CONHC_2H_5$ | Smp. 86-88°C |
| 1.92 | H | H | H | H | H | H | $-CH_2-$ | $-COOC_3H_7$-n | Smp. 28-31°C |
| 1.93 | H | H | H | H | H | H | $-CH_2-$ | $-COOC_3H_7$-i | $n_D^{23}$ = 1.5696 |
| 1.94 | H | H | H | H | H | H | $-CH_2-$ | $-CONHCH_3 \cdot H_2O$ | Smp. 74-81°C |
| 1.95 | H | H | H | H | H | H | $-CH_2-$ | $-CON(CH_3)_2$ | Smp. 142-145°C |
| 1.96 | H | H | H | H | H | H | $-CH_2-$ | $-CONHC_2H_5$ | $n_D^{22,5}$ = 1.6002 |
| 1.97 | H | H | H | H | H | H | $-CH(CH_3)-$ | $-CONH(CH_2)_3OH$ | Smp. 120-122°C |
| 1.98 | H | H | H | H | H | H | $-CH_2-$ | $-COOCH_2CH_2OC_2H_5$ | $n_D^{24}$ = 1.5673 |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.99 | H | H | H | H | H | H | $-CH-$<br>$CH_3$ | $-CONHCH_2-$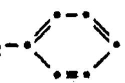 | Smp. 88-90°C |
| 1.100 | H | H | H | H | H | H | $-CH_2-$ | $-CONH(CH_2)_3CH_3$ | Smp. 66-68°C |
| 1.101 | H | H | H | H | H | H | $-CH-$<br>$CH_3$ | 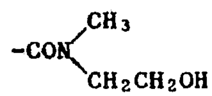 | $n_D^{22}$ = 1.6054 |
| 1.102 | H | H | H | H | H | H | $-CH_2-$ | 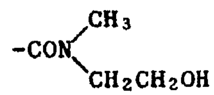 | Smp. 146-149°C |
| 1.103 | H | H | H | H | H | H | $-CH_2-$ | $-COOCH_2-$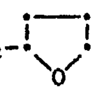 | zähe Masse |
| 1.104 | H | H | H | H | H | H | $-CH-$<br>$CH_3$ | $-CONH(CH_2)_3CH_3 \cdot H_2O$ | Smp. 73-76°C |
| 1.105 | H | H | H | H | H | H | $-CH-$<br>$CH_3$ | $-CO-$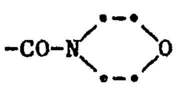 | Smp. 120-121°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.106 | H | H | H | H | H | H | 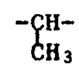 | 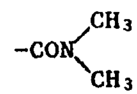 | Smp. 105-111°C |
| 1.107 | H | H | Cl | H | H | H | $-CH_2-$ | $-COOH$ | Smp. 232-233°C |
| 1.108 | H | H | Cl | H | H | H | $-CH_2-$ | $-COOCH_2CH_2OCH_3$ | Smp. 97-98°C |
| 1.109 | H | H | Cl | H | H | H | $-CH_2-$ | $-COOCH_3$ | Smp. 104-105,5°C |
| 1.110 | H | H | Cl | H | H | H | $-CH_2-$ | $-COOC_2H_5$ | Smp. 116-117°C |
| 1.111 | H | H | Cl | H | H | H | $-CH_2-$ | $-COOC_3H_7$-n | Smp. 108-109°C |
| 1.112 | H | H | Cl | H | H | H | $-CH_2-$ | 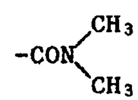 | Smp. 135-136°C |
| 1.113 | H | H | H | H | H | $CH_3$ | $-CH_2-$ | $-COOCH_3$ | Smp. 58-66°C |
| 1.114 | H | H | H | H | H | $CH_3$ | $-CH_2-$ | $-COOC_2H_5$ | $n_D^{22,5}$ = 1.5762 |
| 1.115 | H | H | Cl | H | H | H | $-CH_2-$ | $-COOC_4H_9$-t | Smp. 63-69°C |
| 1.116 | H | H | H | H | H | H | $-CH_2-$ | $-COOC_4H_9$-t | Smp. 68-70°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.117 | H | H | Cl | H | H | H | $-CH_2-$ | $-COOCH_2-C{\equiv}CH$ | Smp. 115-116°C |
| 1.118 | H | H | Cl | H | H | H | $-CH_2-$ | $-COOC_3H_7-i$ | Smp. 147-148°C |
| 1.119 | H | H | Cl | H | H | H | $-CH_2-$ | $-COOCH_2CH_2OC_2H_5$ | Smp. 102-104°C |
| 1.120 | H | H | Cl | H | H | H | $-CH_2-$ | $-COOCH_2-$ 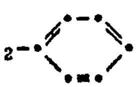 | Smp. 110-112°C |
| 1.121 | H | H | Cl | H | H | H | $-CH_2-$ | $-COOCH_2-CH{=}CH_2$ | Smp. 98-99°C |
| 1.122 | H | H | Cl | H | H | H | $-CH_2-$ | $-COO(CH_2)_{11}CH_3$ | Smp. 76-77°C |
| 1.123 | H | H | Cl | H | H | H | $-CH_2-$ | $-COOC_4H_9-s$ | Smp. 110-111°C |
| 1.124 | H | H | H | H | H | H | $-CH_2-$ | $-COO(CH_2)_7CH_3$ | $n_D^{24} = 1.5419$ |
| 1.125 | H | H | Cl | H | H | H | $-CH_2-$ | $-COOC_4H_9n$ | Smp. 90,5-92°C |
| 1.126 | H | H | H | H | H | H | $-CH_2-$ | $-COO(CH_2)_{11}CH_3$ | $n_D^{23} = 1.5232$ |
| 1.127 | H | H | H | H | H | H | $-CH_2-$ | $-COOCH_2-CH{=}CH_2$ | $n_D^{23} = 1.5885$ |
| 1.128 | H | H | Cl | H | H | H | $-CH_2-$ | $-COO(CH_2)_7CH_3$ | Smp. 87-88°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.129 | H | H | H | H | H | H | $-CH_2-$ | $-COOC_4H_9$-n | $n_D^{22}$ = 1.5642 |
| 1.130 | H | H | H | H | H | H | $-CH_2-$ | $-COOC_4H_9$-s | rotes Oel |
| 1.131 | H | H | Cl | H | H | H | $-CH_2-$ | $-COOCH_2CH_2Cl$ | Smp. 125-126°C |
| 1.132 | H | H | H | H | H | H | $-CH_2-$ | $-COOCH_2-$ 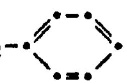 | $n_D^{23,5}$ = 1.6099 |
| 1.133 | H | H | Cl | H | H | H | $-CH_2-$ | $-COOCH_2-$ 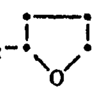 | Smp. 101-103°C |
| 1.134 | H | H | Cl | H | H | H | $-CH_2-$ | $-COS(CH_2)_7CH_3$ | Smp. 53-54°C |
| 1.135 | H | H | H | H | H | H | $-CH_2-$ | $-COOCH_2CH_2Cl$ | Smp. 109-110°C |
| 1.136 | J | H | Cl | H | H | H | $-CH_2-$ | $-COOC_4H_9$-t | Smp. 81-97°C |
| 1.137 | J | H | Cl | H | H | H | $-CH_2-$ | $-COOC_2H_5$ | Smp. 92-94°C |
| 1.138 | J | H | Cl | H | H | H | $-CH_2-$ | $-COO(CH_2)_{11}CH_3$ | Smp. 51-53°C |
| 1.139 | J | H | Cl | H | H | H | $-CH_2-$ | $-COOCH_3$ | Smp. 121-126°C |

<u>Tabelle 1</u> (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.140 | J | H | Cl | H | H | H | $-CH_2-$ | $-COOCH_2CH_2Cl$ | Smp. 44-45°C |
| 1.141 | J | H | Cl | H | H | H | $-CH_2-$ | $-COOCH_2-$ 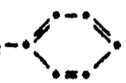 | Smp. 112-113°C |
| 1.142 | J | H | Cl | H | H | H | $-CH_2-$ | $-COOC_3H_7-n$ | Smp. 71-73°C |
| 1.143 | H | H | H | H | H | H | $-CH_2-$ | $-COOC_4H_9-i$ | $n_D^{22}$ = 1.5632 |
| 1.144 | H | H | H | H | H | H | $-CH_2-$ | $-COOCHCH_2CH_2CH_3$<br>$CH_3$ | $n_D^{22}$ = 1.5391 |
| 1.145 | H | H | H | H | H | H | $-CH_2-$ | $-COOCH(CH_2)_5CH_3$<br>$CH_3$ | $n_D^{22}$ = 1.5342 |
| 1.146 | H | H | H | H | H | H | $-CH_2-$ | $-CONH(CH_2)_{11}CH_3$ | Smp. 56-61°C |
| 1.147 | H | H | H | H | H | H | $-CH_2-$ | $-CONHCH_2CH_2-$ 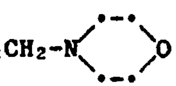 | Smp. 94-99°C |
| 1.148 | H | H | H | H | H | H | $-CH_2-$ | $-CONHCH_2CH_2CH_2OH$ | Smp. 138-139°C |
| 1.149 | H | H | H | H | H | H | $-CH_2-$ | $-CONH-$ 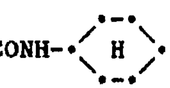 | Smp. 104-106°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.150 | H | H | H | H | H | H | $-CH_2-$ | 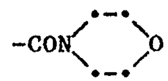 | Smp. 99-103°C |
| 1.151 | H | H | H | H | H | H | $-CH_2-$ | 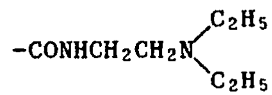 | $n_D^{23}$ = 1.5686 |
| 1.152 | H | H | H | H | H | H | $-CH_2-$ | 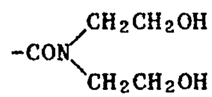 | Smp. 144-146°C |
| 1.153 | H | H | H | H | H | H | $-CH_2-$ | 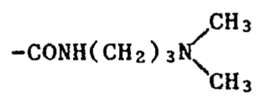 | $n_D^{23}$ = 1.5766 |
| 1.154 | H | H | H | H | H | H | $-CH_2-$ | 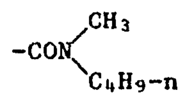 | $n_D^{22}$ = 1.5840 |
| 1.155 | H | H | H | H | H | H | $-CH_2-$ | 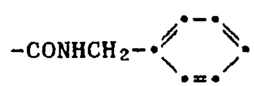 • $H_2O$ | Smp. 70,5-73,5°C |
| 1.156 | H | H | H | H | H | H | $-CH_2-$ | 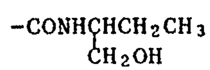 | Smp. 150-151°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.157 | H | H | H | H | H | H | $-CH_2-$ | 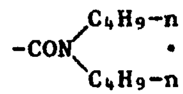 • $2H_2O$ | Smp. 105-106°C |
| 1.158 | H | H | H | H | H | H | $-CH_2-$ | 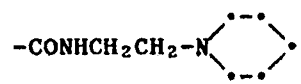 | $n_D^{26}$ = 1.5821 |
| 1.159 | H | H | H | H | H | H | $-CH_2-$ | 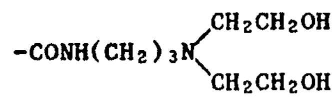 | Smp. 109-110°C |
| 1.160 | H | H | H | H | H | H | $-CH_2-$ | $-CONHCH_2-CH=CH_2$ • $H_2O$ | Smp. 71-75°C |
| 1.161 | H | H | H | H | H | H | $-CH_2-$ | 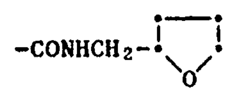 • $H_2O$ | Smp. 57-58°C |
| 1.162 | H | H | H | H | H | H | $-CH_2-$ | $-CONH(CH_2)_3OC_2H_5$ | Smp. 51-61°C |
| 1.163 | H | H | H | H | H | H | $-CH_2-$ | $-CONHCH_2CH_2NHCH_2CH_2OH$ | Smp. 70-91°C |
| 1.164 | H | H | Cl | H | H | H | $-CH_2-$ | $CONH(CH_2)_3OC_2H_5$ | Smp. 85-88°C |
| 1.165 | H | H | Cl | H | H | H | $-CH_2-$ | 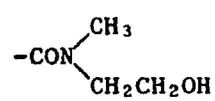 | Smp. 187-189°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.166 | H | H | Cl | H | H | H | $-CH_2-$ | 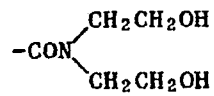 | Smp. 177-179°C |
| 1.167 | H | H | Cl | H | H | H | $-CH_2-$ | 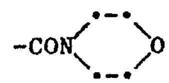 | Smp. 148-150°C |
| 1.168 | H | H | Cl | H | H | H | $-CH_2-$ | $-CONHCH_2CH_2CH_2OH$ | Smp. 157-160°C |
| 1.169 | H | H | Cl | H | H | H | $-CH_2-$ | $-CONHC_4H_9-n \cdot H_2O$ | Smp. 87-90°C |
| 1.170 | H | H | Cl | H | H | H | $-CH_2-$ | $-CONHC_2H_5$ | Smp. 94-98°C |
| 1.171 | H | H | Cl | H | H | H | $-CH_2-$ | $-CONHCH_2-$ 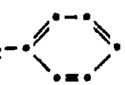 $\cdot \frac{1}{2} H_2O$ | Smp. 146-149°C |
| 1.172 | H | H | H | H | H | $CH_3$ | $-CH_2-$ | $-CONH_2$ | Smp. 193-196°C |
| 1.173 | H | H | H | H | H | H | $-CH_2-$ | $-CONHNH_2 \cdot H_2O$ | Smp. 121-124°C |
| 1.174 | H | H | H | H | H | H | $-CH_2-$ | $-COONa \cdot H_2O$ | Smp. 140-142°C |
| 1.175 | H | H | H | H | H | H | $-CH_2-$ | $-COOK \cdot H_2O$ | Smp. > 200°C |
| 1.176 | H | H | H | H | H | H | $-CH_2-$ | $-COO^{\ominus}\ \overset{\oplus}{H}N(CH_3)_3$ | Smp. 176-178°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.177 | H | H | H | H | H | H | $-CH_2-$ | $-COO^{\ominus}\ \overset{\oplus}{H}N(CH_2CH_2OH)_3$ | Smp. 97-98°C |
| 1.178 | H | H | Cl | H | H | H | $-CH_2-$ | $-COOK \cdot H_2O$ | Smp. > 260°C |
| 1.179 | H | H | Cl | H | H | H | $-CH_2-$ | $-COONa \cdot H_2O$ | Smp. > 260°C |
| 1.180 | H | H | H | H | H | H | $-CH_2-$ | $-COO^{\ominus}\ \overset{\oplus}{H}N(C_2H_5)_3$ | Smp. 255-257°C (Zers.) |
| 1.181 | H | H | Cl | H | H | H | $-CH_2-$ | $-COO^{\ominus}\ {}^{\oplus}NH_4$ | Smp. 227-228°C (Zers.) |
| 1.182 | H | H | Cl | H | H | H | $-CH_2-$ | $-COO^{\ominus}\ \overset{\oplus}{H}N(CH_2CH_2OH)_3$ | Smp. 132-156°C (Zers.) |
| 1.183 | H | H | Cl | H | H | H | $-CH-$<br>$CH_3$ | 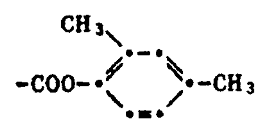 | Smp. 120-122°C |
| 1.184 | H | H | Cl | H | H | H | $-CH_2-$ | $CH_3$<br>$-COOCH(CH_2)_5CH_3$ | Smp. 65-67°C |
| 1.185 | H | H | Cl | H | H | H | $-CH_2-$ | $-COOCH_2CH=CH-CH_3$ | Smp. 100-102°C |
| 1.186 | H | H | Cl | H | H | H | $-CH_2-$ | $CH_3$<br>$-COOCH_2-C=CH_2$ | Smp. 94-95°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.187 | H | H | Cl | H | H | H | $-CH_2-$ | $-COOCH_2CH_2OC_3H_7$-i | Smp. 70-72°C |
| 1.188 | H | H | Cl | H | H | H | $-CH_2-$ | $-COOCH_2CH_2-O-$ 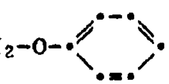 | Smp. 79-80,5°C |
| 1.189 | Br | H | Br | H | H | H | $-CH_2-$ | $-COOCH_3$ | Smp. 143-145°C |
| 1.190 | Br | H | Cl | H | H | H | $-CH_2-$ | $-COOC_3H_7$-i | Smp. 71-73°C |
| 1.191 | Br | H | Br | H | H | H | $-CH_2-$ | $-COOC_3H_7$-i | Smp. 47-51°C |
| 1.192 | Cl | H | Cl | H | H | H | $-CH_2-$ | $-COOC_4H_9$-n | Smp. 42-43,5°C |
| 1.193 | Br | H | Cl | H | H | H | $-CH_2-$ | $-COOC_4H_9$-n | Smp. ca. 28°C |
| 1.194 | Cl | H | Cl | H | H | H | $-CH_2-$ | $-COO(CH_2)_7CH_3$ | Smp. ca. 30°C |
| 1.195 | Br | H | Cl | H | H | H | $-CH_2-$ | $-COO(CH_2)_7CH_3$ | Smp. 41-42°C |
| 1.196 | Br | H | Cl | H | H | H | $-CH_2-$ | $CH_3$<br>$-COOCH(CH_2)_5CH_3$ | Smp. 46-48°C |
| 1.197 | Cl | H | Cl | H | H | H | $-CH_2-$ | $-COO(CH_2)_{11}CH_3$ | Smp. 49-50°C |
| 1.198 | Br | H | Cl | H | H | H | $-CH_2-$ | $-COO(CH_2)_{11}CH_3$ | Smp. 50-52°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.199 | Cl | H | Cl | H | H | H | $-CH_2-$ | $-COOCH_2-$ 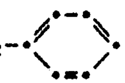 | Smp. 79-80°C |
| 1.200 | Br | H | Cl | H | H | H | $-CH_2-$ | $-COOCH_2-$ 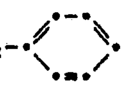 | Smp. 100-102°C |
| 1.201 | Br | H | Br | H | H | H | $-CH_2-$ | $-COOCH_2-$ 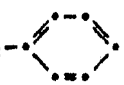 | Smp. 101-104°C |
| 1.202 | Br | H | Cl | H | H | H | $-CH_2-$ | $-COOCH_2CH_2OCH_3$ | Smp. 68-70°C |
| 1.203 | Cl | H | Cl | H | H | H | $-CH_2-$ | $-COOCH_2CH_2OC_2H_5$ | Smp. 81-82°C |
| 1.204 | Br | H | Cl | H | H | H | $-CH_2-$ | $-COOCH_2CH_2OC_2H_5$ | Smp. 71-72°C |
| 1.205 | Br | H | Cl | H | H | H | $-CH_2-$ | $-COOCH_2CH_2OC_3H_7$-i | $n_D^{25} = 1.5763$ |
| 1.206 | Br | H | Cl | H | H | H | $-CH_2-$ | $-COOCH_2CH_2O-$ 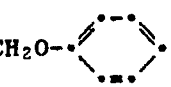 | Smp. 80-82°C |
| 1.207 | Cl | H | Cl | H | H | H | $-CH_2-$ | $-COOCH_2-$ 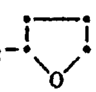 | Smp. 77-78°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.208 | Br | H | Cl | H | H | H | $-CH_2-$ | $-COOCH_2-$ 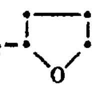 | Smp. 79-80°C |
| 1.209 | Cl | H | Cl | H | H | H | $-CH_2-$ | $-COOCH_2CH=CH_2$ | Smp. 72-73°C |
| 1.210 | Br | H | Cl | H | H | H | $-CH_2-$ | $-COOCH_2CH=CH_2$ | Smp. 66-68,5°C |
| 1.211 | Br | H | Br | H | H | H | $-CH_2-$ | $-COOCH_2CH=CH_2$ | Smp. 78-79°C |
| 1.212 | Br | H | Cl | H | H | H | $-CH_2-$ | $-COOCH_2CH=CH-CH_3$ | Smp. 60-64°C |
| 1.213 | Cl | H | Cl | H | H | H | $-CH_2-$ | $-COOCH_2-C(CH_3)=CH_2$ | Smp. 62-65°C |
| 1.214 | Br | H | Cl | H | H | H | $-CH_2-$ | $-COOCH_2-C(CH_3)=CH_2$ | Smp. 62-64°C |
| 1.215 | Br | H | Cl | H | H | H | $-CH_2-$ | $-COO-$ 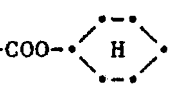 | Smp. 52-54°C |
| 1.216 | H | H | Cl | H | H | H | $-CH(CH_3)-$ | $-COOC_3H_7-i$ | $n_D^{24}$ = 1.5642 |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.217 | H | H | Cl | H | H | H | $-CH-$<br>$CH_3$ | $-COO(CH_2)_7CH_3$ | $n_D^{23}$ = 1.5356 |
| 1.218 | H | H | Cl | H | H | H | $-CH-$<br>$CH_3$ | $CH_3$<br>$-COOCH(CH_2)_5CH_3$ | $n_D^{25}$ = 1.5370 |
| 1.219 | H | H | Cl | H | H | H | $-CH-$<br>$CH_3$ | $-COO(CH_2)_{11}CH_3$ | Smp. 54-55°C |
| 1.220 | H | H | Cl | H | H | H | $-CH-$<br>$CH_3$ | $-COOCH_2-$ 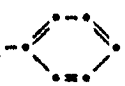 | Smp. 57-59°C |
| 1.221 | H | H | Cl | H | H | H | $-CH-$<br>$CH_3$ | $-COOCH_2CH_2OC_3H_7$-i | $n_D^{32}$ = 1.5403 |
| 1.222 | H | H | Cl | H | H | H | $-CH-$<br>$CH_3$ | $-COOCH_2CH_2O-$ 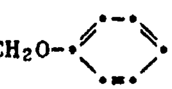 | $n_D^{29}$ = 1.5962 |
| 1.223 | H | H | Cl | H | H | H | $-CH-$<br>$CH_3$ | $-COOCH_2CH=CH_2$ | Smp. 40-41°C |
| 1.224 | H | H | Cl | H | H | H | $-CH-$<br>$CH_3$ | $-COOCH_2CH=CH-CH_3$ | Smp. 39-40°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.225 | H | H | Cl | H | H | H | $-CH(CH_3)-$ | $-COOCH_2-C(CH_3)=CH_2$ | Smp. 62-63°C |
| 1.226 | H | H | Cl | H | H | H | $-CH(CH_3)-$ | -COO- 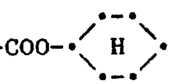 | $n_D^{30}$ = 1.5677 |
| 1.227 | Br | H | Cl | H | H | H | $-CH(CH_3)-$ | $-COO(CH_2)_7CH_3$ | $n_D^{28}$ = 1.5439 |
| 1.228 | Cl | H | Cl | H | H | H | $-CH(CH_3)-$ | $-COOCH(CH_3)(CH_2)_5CH_3$ | $n_D^{25}$ = 1.5408 |
| 1.229 | Br | H | Cl | H | H | H | $-CH(CH_3)-$ | $-COOCH(CH_3)(CH_2)_5CH_3$ | $n_D^{25}$ = 1.5527 |
| 1.230 | Br | H | Cl | H | H | H | $-CH(CH_3)-$ | $-COO(CH_2)_{11}CH_3$ | $n_D^{30}$ = 1.5347 |
| 1.231 | Br | H | Cl | H | H | H | $-CH(CH_3)-$ | $-COOCH_2-$ 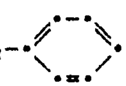 | Smp. 55-56°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.232 | Br | H | Cl | H | H | H | $-\underset{CH_3}{CH}-$ | $-COOCH_2-$ 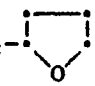 | $n_D^{30} = 1.5886$ |
| 1.233 | Br | H | Cl | H | H | H | $-\underset{CH_3}{CH}-$ | $-COOCH_2CH_2OC_3H_7-i$ | $n_D^{28} = 1.5642$ |
| 1.234 | Br | H | Cl | H | H | H | $-\underset{CH_3}{CH}-$ | $-COOCH_2CH_2O-$ 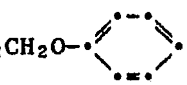 | $n_D^{20} = 1.6031$ |
| 1.235 | Br | H | Cl | H | H | H | $-\underset{CH_3}{CH}-$ | $-COOCH_2CH=CH_2$ | Smp. 55-56°C |
| 1.236 | Cl | H | Cl | H | H | H | $-\underset{CH_3}{CH}-$ | $-COOCH_2CH=CH-CH_3$ | Smp. 38-39°C |
| 1.237 | Br | H | Cl | H | H | H | $-\underset{CH_3}{CH}-$ | $-COOCH_2CH=CH-CH_3$ | Smp. 38-40°C |
| 1.238 | Br | H | Cl | H | H | H | $-\underset{CH_3}{CH}-$ | $-COOCH_2\overset{CH_3}{C}=CH_2$ | $n_D^{28} = 1.5824$ |
| 1.239 | H | H | Cl | H | H | H | $-CH_2-$ | $-COO-$ 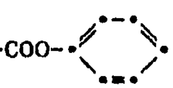 | Smp. 165-170°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.240 | H | H | Cl | H | H | H | $-CH_2-$ | -COO- 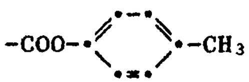 | Smp. 143-145°C |
| 1.241 | H | H | Cl | H | H | H | $-CH_2-$ | -COO- 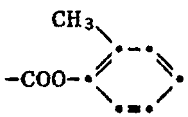 | Smp. 111-116°C |
| 1.242 | H | H | Cl | H | H | H | $-CH_2-$ | -COO- 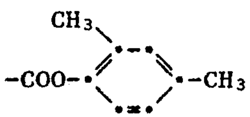 | Smp. 108-119°C |
| 1.243 | H | H | Cl | H | H | H | $-CH(CH_3)-$ | -COO- 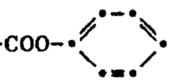 | Smp. 102-105°C |

Tabelle 1 (Fortsetzung)

| Nr. | $R^1$ | $R^2$ | $R^3$ | $R^4$ | $R^5$ | $R^6$ | A + Z | physikal. Konstante |
|---|---|---|---|---|---|---|---|---|
| 1.244 | H | H | Cl | H | H | H | 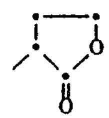 | Smp. 140-141,5°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.245 | H | H | Cl | H | H | H | $-CH_2-$ | $-COO\overset{CH_3}{C}HCH_2CH_2CH_3$ | Smp. 65-70°C |
| 1.246 | H | H | H | H | H | H | $-CH_2-$ | $-COOCH_2-\overset{CH_3}{C}H(CH_2)_2CH_3$ | $n_D^{22}$ = 1.5525 |
| 1.247 | H | H | Cl | H | H | H | $-CH_2-$ | -COO- 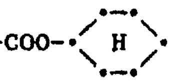 | Smp. 112-113°C |
| 1.248 | H | H | Cl | H | H | H | $-CH_2-$ | $-COOCH_2\overset{CH_3}{C}H-CH_3$ | Smp. 113-114°C |
| 1.249 | H | H | H | H | H | H | $-CH_2-$ | $-COO(CH_2)_2\overset{OCH_3}{C}HCH_3$ | $n_D^{22}$ =1.5580 |
| 1.250 | H | H | H | H | H | H | $-CH_2-$ | $-COOCH_2CH_2OCH_2CH_2O(CH_2)_3CH_3$ | $n_D^{22}$ =1.5389 |
| 1.251 | H | H | H | H | H | H | $-CH_2-$ | $-COS(CH_2)_3CH_3$ | $n_D^{23}$ =1.6096 |
| 1.252 | H | H | H | H | H | H | $-CH_2-$ | -COO- 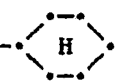 | $n_D^{23}$ =1.5755 |
| 1.253 | H | H | H | H | H | H | $-CH_2-$ | $-COO(CH_2)_4CH_3$ | $n_D^{23}$ =1.5591 |
| 1.254 | H | H | H | H | H | H | $-CH_2-$ | $-COS(CH_2)_7CH_3$ | $n_D^{22}$ =1.5697 |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.255 | H | H | Cl | H | H | H | $-CH_2-$ | $-COOCH_2-\overset{CH_3}{CH}(CH_2)_2CH_3$ | Smp. 74-75°C |
| 1.256 | H | H | Cl | H | H | H | $-CH_2-$ | $-COS(CH_2)_3CH_3$ | $n_D^{22}$ =1.6076 |
| 1.257 | H | H | H | H | H | H | $-CH_2-$ | $-COOCH_2CH=CH-CH_3$ | $n_D^{22}$ =1.5833 |
| 1.258 | H | H | H | H | H | H | $-CH_2-$ | $-COOCH_2-\overset{C_2H_5}{CH}-C_2H_5$ | $n_D^{23}$ =1.5530 |
| 1.259 | H | H | Cl | H | H | H | $-CH_2-$ | $-COOCH_2CH_2OCH_2CH_2O(CH_2)_3CH_3$ | Smp. 39-41°C |
| 1.260 | H | H | Cl | H | H | H | $-CH_2-$ | $-COO(CH_2)_2\overset{OCH_3}{CH}CH_3$ | Smp. 72-73°C |
| 1.261 | H | H | Cl | H | H | H | $-CH_2-$ | $-COO(CH_2)_4CH_3$ | Smp. 78-79°C |
| 1.262 | H | H | Cl | H | H | H | $-CH_2-$ | $-COO\overset{C_2H_5}{CH}-(CH_2)_2CH_3$ | Smp. 37-46°C |
| 1.263 | H | H | H | H | H | H | $-CH_2-$ | $-COOCH_2CH_2OC_3H_7-i$ | $n_D^{22}$= 1.5546 |
| 1.264 | H | H | Cl | H | H | H | $-CH_2-$ | $-COO(CH_2)_{13}CH_3$ | Smp. 75-76°C |
| 1.265 | H | H | H | H | H | H | $-CH_2-$ | $-COO\overset{C_2H_5}{CH}-C_2H_5$ | Smp. 47-50°C |
| 1.266 | H | H | H | H | H | H | $-CH_2-$ | -COO- 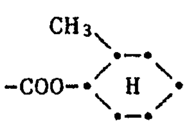 | Smp. 29-31°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.267 | H | H | Cl | H | H | H | $-CH_2-$ | $-COOCH_2-\overset{C_2H_5}{CH}-C_2H_5$ | Smp. 58-63°C |
| 1.268 | H | H | H | H | H | H | $-CH_2-$ | $-COOCH_2CH_2OCH_2CH_2OC_2H_5$ | $n_D^{22}$ =1.5489 |
| 1.269 | H | H | H | H | H | H | $-CH_2-$ | $-COOCH_2CH_2O-$ 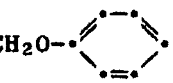 | Smp. 80-81°C |
| 1.270 | H | H | Cl | H | H | H | $-CH_2-$ | $-COO\overset{C_2H_5}{CH}-C_2H_5$ | Smp. 55-80°C |
| 1.271 | H | H | H | H | H | H | $-CH_2-$ | $-COO\overset{CH_3}{CH}CH_2\overset{CH_3}{CH}-CH_3$ | $n_D^{22}$ =1.5463 |
| 1.272 | H | H | H | H | H | H | $-CH_2-$ | $-COO(CH_2)_{13}CH_3$ | Smp. 35-36°C |
| 1.273 | H | H | H | H | H | H | $-CH_2-$ | $-COOCH_2CH_2O(CH_2)_3CH_3$ | $n_D^{22}$=1.5495 |
| 1.274 | H | H | Cl | H | H | H | $-CH_2-$ | $-COOCH_2CH_2OCH_2CH_2OC_2H_5$ | Smp.42-43°C |
| 1.275 | H | H | H | H | H | H | $-CH_2-$ | $-COOCH_2-\overset{CH_3}{CH}-C_2H_5$ | $n_D^{22}$ =1.5566 |
| 1.276 | H | H | Cl | H | H | H | $-CH_2-$ | $-COO\overset{CH_3}{CH}CH_2\overset{CH_3}{CH}-CH_3$ | Smp.63-64°C |
| 1.277 | H | H | H | H | H | H | $-CH_2-$ | $-COS\overset{CH_3}{CH}-C_2H_5$ | $n_D^{22}$ =1.5973 |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.278 | H | H | Cl | H | H | H | $-CH_2-$ | 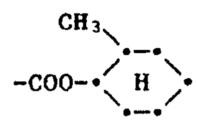 | Smp. 98-101°C |
| 1.279 | H | H | H | H | H | H | $-CH_2-$ | 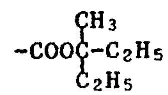 | $n_D^{22}$=1.5551 |
| 1.280 | H | H | H | H | H | H | $-CH_2-$ | 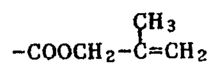 | $n_D^{22}$ =1.5805 |
| 1.281 | H | H | H | H | H | H | $-CH_2-$ | 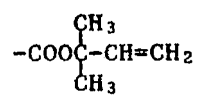 | $n_D^{22}$=1.5793 |
| 1.282 | H | H | H | H | H | H | $-CH_2-$ | $-COOCH_2CH_2OCH_2CH_2OCH_3$ | $n_D^{23}$ =1.5560 |
| 1.283 | H | H | Cl | H | H | H | $-CH_2-$ | 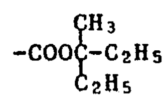 | $n_D^{22}$ =1.5632 |
| 1.284 | H | H | Cl | H | H | H | $-CH_2-$ | $-COO(CH_2)_{10}CH_3$ | Smp. 70-71°C |
| 1.285 | H | H | Cl | H | H | H | $-CH_2-$ | 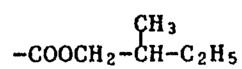 | Smp. 78-79°C |
| 1.286 | H | H | H | H | H | H | $-CH_2-$ | 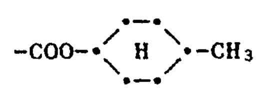 | Smp. 40-42°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.287 | H | H | H | H | H | H | $-CH_2-$ | $-COO(CH_2)_6CH_3$ | $n_D^{23}$=1.5469 |
| 1.288 | H | H | H | H | H | H | $-CH_2-$ | $CH_3$<br>$-COOC-C_2H_5$<br>$CH_3$ | $n_D^{22}$=1.5581 |
| 1.289 | H | H | Cl | H | H | H | $-CH_2-$ | $-COOCH_2CH_2O(CH_2)_3CH_3$ | Smp. 69-70°C |
| 1.290 | H | H | Cl | H | H | H | $-CH_2-$ | $CH_3$<br>$-COSCH-C_2H_5$ | Smp. 55-56°C |
| 1.291 | H | H | Cl | H | H | H | $-CH_2-$ | $CH_3$<br>$-COOC-CH=CH_2$<br>$CH_3$ | Smp. 83-87°C |
| 1.292 | H | H | H | H | H | H | $-CH_2-$ | $-COSCH_3$ | Smp. 41-44°C |
| 1.293 | H | H | Cl | H | H | H | $-CH_2-$ | $-COOCH_2CH_2OCH_2CH_2OCH_3$ | $n_D^{23}$= 1.5633 |
| 1.294 | H | H | Cl | H | H | H | $-CH_2-$ | $-COSCH_3$ | Smp. 89-91°C |
| 1.295 | H | H | Cl | H | H | H | $-CH_2-$ | $CH_3$<br>$-COOC-C_2H_5$<br>$CH_3$ | Smp. 53-54°C |
| 1.296 | H | H | H | H | H | H | $-CH_2-$ | $-COO(CH_2)_{10}CH_3$ | $n_D^{23}$=1.5310 |
| 1.297 | H | H | Cl | H | H | H | $-CH_2-$ | $-COO(CH_2)_6CH_3$ | Smp.74-76°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.298 | H | H | H | H | H | H | $-CH_2-$ | $CH_3CH_3$<br>$-COOCH-CH-CH_3$ | $n_D^{23}$=1.5554 |
| 1.299 | H | H | Cl | H | H | H | $-CH_2-$ | -COO- 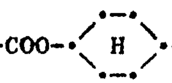 $-CH_3$ | Smp. 103-105°C |
| 1.300 | H | H | H | H | H | H | $-CH_2-$ | $CH_3$<br>$-COSC-CH_3$<br>$CH_3$ | $n_D^{23}$=1.5987 |
| 1.301 | H | H | Cl | H | H | H | $-CH_2-$ | $-COS(CH_2)_{11}CH_3$ | Smp.26-28°C |
| 1.302 | H | H | Cl | H | H | H | $-CH_2-$ | $-COS(CH_2)_9CH_3$ | Smp.29-31°C |
| 1.303 | H | H | Cl | H | H | H | $-CH_2-$ | $-COO(CH_2)_9CH_3$ | Smp.73-74°C |
| 1.304 | H | H | H | H | H | H | $-CH_2-$ | $CH_3$<br>$-COOCH(CH_2)_4CH_3$ | $n_D^{23}$=1.5433 |
| 1.305 | H | H | Cl | H | H | H | $-CH_2-$ | $C_3H_7$-n<br>$-COOCH-C{\equiv}CH$ | Smp. 81-82°C |
| 1.306 | H | H | H | H | H | H | $-CH_2-$ | $C_5H_{11}$-n<br>$-COOCH-CH-CH_2$ | $n_D^{23}$=1.5472 |
| 1.307 | H | H | Cl | H | H | H | $-CH_2-$ | $CH_3$ $CH_3$<br>$-COOCH-CH-CH_3$ | Smp.70-74°C |
| 1.308 | H | H | Cl | H | H | H | $-CH_2-$ | $CH_3$<br>$-COSC-CH_3$<br>$CH_3$ | $n_D^{22}$=1.5996 |

**Tabelle 1** (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.309 | H | H | H | H | H | H | $-CH_2-$ | $-COOCH(CH_3)-C\equiv CH$ | $n_D^{23}=1.5837$ |
| 1.310 | H | H | H | H | H | H | $-CH_2-$ | $-COS(CH_2)_{11}CH_3$ | $n_D^{23}=1.5523$ |
| 1.311 | H | H | H | H | H | H | $-CH_2-$ | $-COOCH_2-C(CH_3)_2-CH_3$ | $n_D^{22}=1.5524$ |
| 1.312 | H | H | H | H | H | H | $-CH_2-$ | $-COSC_2H_5$ | $n_D^{23}=1.6310$ |
| 1.313 | H | H | Cl | H | H | H | $-CH_2-$ | $-COOCH_2-C(CH_3)_2-CH_3$ | Smp.76-81°C |
| 1.314 | H | H | Cl | H | H | H | $-CH_2-$ | $-COSC_3H_7$-n | $n_D^{22}=1.6136$ |
| 1.315 | H | H | H | H | H | H | $-CH_2-$ | $-COO(CH_2)_9CH_3$ | $n_D^{22}=1.5308$ |
| 1.316 | H | H | Cl | H | H | H | $-CH_2-$ | $-COOCH(CH_3)(CH_2)_4CH_3$ | Smp.65-67°C |
| 1.317 | H | H | H | H | H | H | $-CH_2-$ | $-COO(CH_2)_2CH(CH_3)-CH_3$ | $n_D^{23}=1.5568$ |
| 1.318 | H | H | H | H | H | H | $-CH_2-$ | $-COOCH(C_2H_5)(CH_2)_3CH_3$ | $n_D^{22}=1.5454$ |
| 1.319 | H | H | Cl | H | H | H | $-CH_2-$ | $-COO(CH_2)_8CH_3$ | Smp.78-79°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.320 | H | H | H | H | H | H | $-CH_2-$ | $CH_3$<br>$-COSCH_2CHCH_3$ | $n_D^{23}=1.6049$ |
| 1.321 | H | H | Cl | H | H | H | $-CH_2-$ | $-COSC_2H_5$ | Smp. 55-57°C |
| 1.322 | H | H | H | H | H | H | $-CH_2-$ | $-COO(CH_2)_8CH_3$ | $n_D^{24}=1.5436$ |
| 1.323 | H | H | Cl | H | H | H | $-CH_2-$ | $C_2H_5$<br>$-COOCH_2-CH(CH_2)_3CH_3$ | Smp. 45-47°C |
| 1.324 | H | H | Cl | H | H | H | $-CH_2-$ | $CH_3$<br>$-COSCH_2CH-CH_3$ | $n_D^{23}=1.6045$ |
| 1.325 | H | H | H | H | H | H | $-CH_2-$ | $-COS(CH_2)_9CH_3$ | $n_D^{23}=1.5630$ |
| 1.326 | H | H | Cl | H | H | H | $-CH_2-$ | $CH_3$<br>$-COO(CH_2)_2CH-CH_3$ | Smp. 72-74°C |
| 1.327 | H | H | Cl | H | H | H | $-CH_2-$ | $C_2H_5$<br>$-COOCH(CH_2)_3CH_3$ | $n_D^{22}=1.5542$ |
| 1.328 | H | H | H | H | H | H | $-CH_2-$ | $-COO(CH_2)_5CH_3$ | $n_D^{22}=1.5512$ |
| 1.329 | H | H | H | H | H | H | $-CH_2-$ | $C_3H_7$-n<br>$-COOCH(CH_2)_2CH_3$ | Smp. 48-50°C |
| 1.330 | H | H | H | H | H | H | $-CH_2-$ | $-COS(CH_2)_4CH_3$ | $n_D^{22}=1.5937$ |
| 1.331 | H | H | H | H | H | H | $-CH_2-$ | $-COSC_3H_7$-iso | $n_D^{23}=1.5821$ |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.332 | H | H | H | H | H | H | $-CH_2-$ | $C_2H_5$<br>$-COOCH_2CH-(CH_2)_3CH_3$ | $n_D^{22}$=1.5395 |
| 1.333 | H | H | Cl | H | H | H | $-CH_2-$ | $C_3H_7$-n<br>$-COOCH(CH_2)_2CH_3$ | Smp.55-57°C |
| 1.334 | H | H | Cl | H | H | H | $-CH_2-$ | $COS(CH_2)_5CH_3$ | $n_D^{22}$=1.5882 |
| 1.335 | H | H | Cl | H | H | H | $-CH_2-$ | $-COS(CH_2)_4CH_3$ | $n_D^{23}$=1.5990 |
| 1.336 | H | H | Cl | H | H | H | $-CH_2-$ | $-COO(CH_2)_5CH_3$ | Smp.71-72°C |
| 1.337 | H | H | Cl | H | H | H | $-CH_2-$ | $-COSC_3H_7$-iso | Smp.62-64°C |
| 1.338 | H | H | Cl | H | H | H | $-CH_2-$ | $C_2H_5$ $CH_3$<br>$-COOCH-CH_2CHC_2H_5$ | Smp.25-29°C |
| 1.339 | H | H | H | H | H | H | $-CH_2-$ | $C_3H_7$-i<br>$-COOCH-C_3H_7$-i | $n_D^{22}$=1.5468 |
| 1.340 | H | H | H | H | H | H | $-CH_2-$ | $CH_3$<br>$-COOCH-(CH_2)_3CH_3$ | $n_D^{23}$=1.5531 |
| 1.341 | H | H | Cl | H | H | H | $-CH_2-$ | $C_5H_{11}$-n<br>$-COOCH-CH=CH_2$ | $n_D^{23}$=1.5579 |
| 1.342 | H | H | H | H | H | H | $-CH_2-$ | $C_2H_5$<br>$-COOCH-(CH_2)_2CH_3$ | Smp.42-44°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.343 | H | H | H | H | H | H | $-CH_2-$ | $-COSC_3H_7$-n | $n_D^{22}$ =1.6108 |
| 1.344 | H | H | Cl | H | H | H | $-CH_2-$ | $CH_3$<br>$-COOCH-(CH_2)_3CH_3$ | Smp.68-71°C |
| 1.345 | H | H | H | H | H | H | $-CH_2-$ | $C_2H_5$ $CH_3$<br>$-COOCHCH_2CHC_2H_5$ | $n_D^{23}$=1.5472 |
| 1.346 | H | H | Cl | H | H | H | $-CH_2-$ | $C_3H_7$-i<br>$-COOCH-C_3H_7$-i | Smp.88-89°C |
| 1.347 | H | H | H | H | H | H | $-CH_2-$ | $-COS(CH_2)_5CH_3$ | $n_D^{22}$=1.5804 |
| 1.348 | H | H | H | H | H | H | $-CH_2-$ | $-COO(CH_2)_9-CH=CH_2$ | $n_D^{22}$=1.5386 |
| 1.349 | H | H | H | H | H | H | $-CH_2-$ | $C_3H_7$-n<br>$-COOCH-C\equiv CH$ | $n_D^{22}$=1.5659 |
| 1.350 | H | H | Cl | H | H | H | $-CH_2-$ | $CH_3$<br>$-COOCH-C\equiv CH$ | Smp.97-100°C |
| 1.351 | H | H | H | H | H | H | $-CH_2-$ | $CH_3$<br>$-COOC-C\equiv CH$<br>$C_2H_5$ | $n_D^{22}$=1.5688 |
| 1.352 | H | H | Cl | H | H | H | $-CH_2-$ | $-COO(CH_2)_9-CH=CH_2$ | Smp.66-67°C |
| 1.353 | H | H | Cl | H | H | H | $-CH_2-$ | $CH_3$<br>$-COO-C-C\equiv CH$<br>$CH_3$ | Smp.76-81°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.354 | H | H | H | H | H | H | $-CH_2$ | $CH_3$<br>$-COO-C-C\equiv CH$<br>$CH_3$ | $n_D^{23}=1.5740$ |
| 1.355 | H | H | Cl | H | H | H | $-CH_2-$ | $CH_3$<br>$-COO-C-C\equiv CH$<br>$C_2H_5$ | Smp. 78-79°C |
| 1.356 | H | H | Cl | H | H | H | $-CH_2-$ | $CH_3\ CH_3$<br>$-COO-CH-CH-C_2H_5$ | Smp. 71-73°C |
| 1.357 | Br | H | Cl | H | H | H | $-CH_2-$ | $-COOCH_3$ | Smp. 126-128°C |
| 1.358 | Br | H | Cl | H | H | H | $-CH-$<br>$CH_3$ | $-COOC_3H_7-i$ | Smp. 66-68°C |
| 1.359 | Cl | H | Cl | H | H | H | $-CH_2-$ | $-COOCH_2CH_2OCH_3$ | Smp. 68-70°C |
| 1.360 | Cl | H | Cl | H | H | H | $-CH_2-$ | $-COOC_3H_7-i$ | Smp. 60-63°C |
| 1.361 | H | H | Cl | H | H | H | $-CH-$<br>$CH_3$ | $-COOCH_2-$ 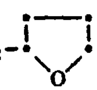 | $n_D^{30}=1.5734$ |
| 1.362 | H | H | Cl | H | H | H | $-CH_2-$ | $-COOCH_2COOC_4H_9-n$ | Smp. 52-54°C |
| 1.363 | H | H | Cl | H | H | H | $-CH_2-$ | $-COOCH-COOC_4H_9-n$<br>$CH_3$ | $n_D^{22}=1.5508$ |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.364 | H | H | Cl | H | H | H | $-CH_2-$ | 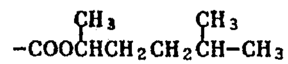 | Smp. 55-59°C |
| 1.365 | H | H | Cl | H | H | H | $-CH_2-$ | 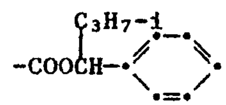 | Smp. 43-47°C |
| 1.366 | H | H | Cl | H | H | H | $-CH_2-$ | 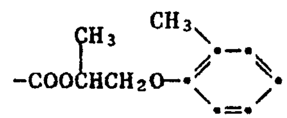 | Smp. 75-78°C |
| 1.367 | H | H | Cl | H | H | H | $-CH_2-$ | 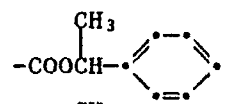 | Smp. 117-122°C |
| 1.368 | H | H | Cl | H | H | H | $-CH_2-$ | 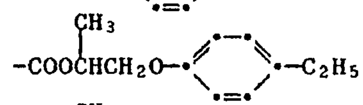 | Smp. 63-68°C |
| 1.369 | H | H | Cl | H | H | H | $-CH_2-$ | 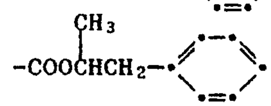 | Smp. 116-118°C |
| 1.370 | H | H | Cl | H | H | H | $-CH_2-$ | 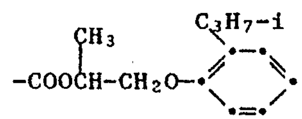 | Smp. 41-43°C |

Tabelle 1 (Fortsetzung)

| Nr. | $R^1$ | $R^2$ | $R^3$ | $R^4$ | $R^5$ | $R^6$ | A | Z | physikal. Konstante |
|---|---|---|---|---|---|---|---|---|---|
| 1.371 | H | H | Cl | H | H | H | $-CH_2-$ | 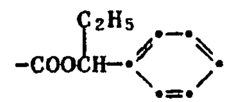 | Smp. 74-76°C |
| 1.372 | H | H | Cl | H | H | H | $-CH_2-$ | 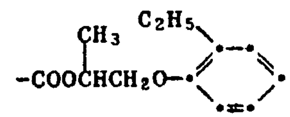 | Smp. 96-98°C |
| 1.373 | H | H | Cl | H | H | H | $-CH_2-$ | 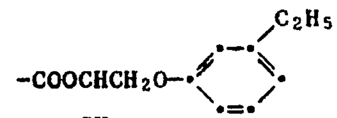 | Smp. 82-85°C |
| 1.374 | H | H | Cl | H | H | H | $-CH_2-$ | 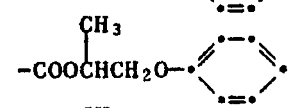 | Smp. 42-44°C |
| 1.375 | H | H | Cl | H | H | H | $-CH_2-$ | 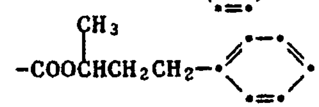 | Smp. 78-79°C |
| 1.376 | H | H | Cl | H | H | H | $-CH_2-$ | 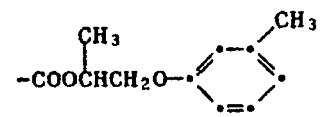 | Smp. 58-61°C |
| 1.377 | H | H | Cl | H | H | H | $-CH_2-$ | 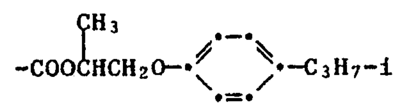 | Smp. 35-38°C |
| 1.378 | H | H | Cl | H | H | H | $-CH_2-$ | 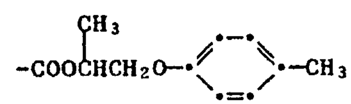 | Smp. 82-84°C |

Die Verbindungen der Formel I lassen sich nach bekannten Methoden herstellen, wie sie beispielsweise in den europäischen Patentpublikationen 86 750 und 94 349 beschrieben sind, oder sind analog bekannten Methoden herstellbar.

Die Chinolinderivate der Formel I besitzen in hervorragendem Masse die Eigenschaften, Kulturpflanzen gegen schädigende Wirkungen von herbizid wirksamen 2-[4-(5-Chlor-3-fluorpyridin-2-yloxy)-phenoxy]-propionsäure Derivaten zu schützen. Die vorgenannten herbiziden Wirkstoffe sind aus den publizierten

Europäischen Patentanmeldungen EP-A-83556 und EP-A-97460 bekannt und können nach den dort angegebenen Methoden hergestellt werden. Besonders wirkungsvolle und gemäss der erfindungsgemässen Lehre einsetzbare 2-[4-(5-Chlor-3-fluorpyridin-2-yloxy)-phenoxy]-propionsäure-Derivate entsprechen der Formel II

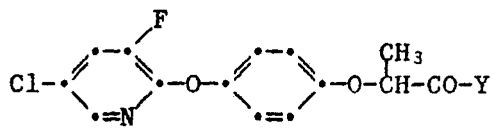

(II)

worin Y für eine Gruppe $-NR^{16}R^{17}$, $-O-R^{18}$, $-S-R^{18}$ oder $-O-N=CR^{19}R^{20}$ steht,

$R^{16}$ und $R^{17}$ unabhängig voneinander Wasserstoff, $C_1$-$C_8$-Alkoxy, $C_1$-$C_8$-Alkyl, Phenyl oder Benzyl,

$R^{16}$ uns $R^{17}$ zusammen mit dem sie tragenden Stickstoffatom einen 5-bis 6-gliedrigen gesättigten Stickstoffheterocyclus, der durch ein Sauerstoff- oder Schwefelatom unterbrochen sein kann,

$R^{18}$ Wasserstoff oder das Aequivalent eines Alkalimetall-, Erdalkalimetall-, Kupfer- oder Eisen-Ions; einen quaternären $C_1$-$C_4$-Alkylammonium- oder $C_1$-$C_4$-Hydroxyalkylammonium-Rest; einen gegebenenfalls ein- oder mehrfach durch Amino, Halogen, Hydroxyl, Cyan, Nitro, Phenyl, $C_1$-$C_4$-Alkoxy, Polyäthoxy mit 2 bis 6 Aethylenoxideinheiten, $-COOR^{21}$, $-COSR^{21}$, $-CONH_2$-, $-CON(C_1$-$C_4$-alkoxy)-$C_1$-$C_4$-alkyl, -CO-N-di-$C_1$-$C_4$-alkyl, -CONH-$C_1$-$C_4$-alkyl, -N($C_1$-$C_4$-alkoxy)-$C_1$-$C_4$-alkyl oder Di-$C_1$-$C_4$-alkylamino substituierten $C_1$-$C_9$-Alkylrest; einen gegebenenfalls durch Halogen oder $C_1$-$C_4$-Alkoxy substituierten $C_3$-$C_9$-Alkenylrest; einen gegebenenfalls durch Halogen oder $C_1$-$C_4$-Alkoxy substituierten $C_3$-$C_9$-Alkinylrest; $C_3$-$C_9$-Cycloalkyl; oder gegebenenfalls durch Cyan, $C_1$-$C_4$-Alkyl, $C_1$-$C_4$-Alkoxy, Acetyl, $-COOR^{21}$, $-COSR^{21}$, $-CONH_2$, -CON($C_1$-$C_4$-alkoxy)-$C_1$-$C_4$-alkyl, -CO-N-di-$C_1$-$C_4$-alkyl oder -CONH-$C_1$-$C_4$-alkyl substituiertes Phenyl,

$R^{19}$ und $R^{20}$ unabhängig voneinander $C_1$-$C_4$-Alkyl oder zusammen eine 3-bis 6-gliedrige Alkylenkette und

$R^{21}$ Wasserstoff, $C_1$-$C_6$-Alkyl, $C_1$-$C_6$-Halogenalkyl, $C_2$-$C_6$-Alkoxyalkyl, $C_3$-$C_6$-Alkenyl, $C_3$-$C_6$-Halogenalkenyl, $C_3$-$C_6$-Alkinyl oder $C_3$-$C_6$-Halogenalkinyl bedeuten.

In den Verbindungen der Formel II bedeutet Halogen als selbstständiger Substituent oder Teil eines anderen Substituenten, wie Halogenalkyl, Halogenalkoxy, Halogenalkenyl oder Halogenalkinyl, Fluor, Chlor, Brom oder Jod, worunter Fluor oder Chlor bevorzugt sind.

Alkyl steht Je nach der Anzahl der vorhandenen Kohlenstoffatome für Methyl, Aethyl, n-Propyl, i-Propyl sowie die isomere Butyl, Pentyl, Hexyl, Heptyl oder Oktyl. Die in den Resten Alkoxy, Alkoxyalkyl, Halogenalkyl oder Halogenalkoxy enthaltenen Alkylgruppen haben die gleiche Bedeutung. Bevorzugt sind jeweils Alkylgruppe mit niedriger Anzahl von Kohlenstoffatomen.

Bevorzugte Halogenalkylreste, bzw. Halogenalkylteile in Halogenalkoxyresten sind: Fluormethyl, Difluormethyl, Trifluormethyl, Chlormethyl, Trichlormethyl, 2-Fluoräthyl, 2,2,2-Trifluoräthyl, 1,1,2,2-Tetrafluoräthyl, Perfluoräthyl, 2-Chloräthyl, 2,2,2-Trichloräthyl, 2-Bromäthyl und 1,1,2,3,3,3-Hexafluorpropyl.

Cycloalkyl steht für mono-, und bi-cyclische gesättigte Kohlenwasserstoffringsysteme wie Cyclopropyl, Cyclobutyl, Cyclopentyl, Cyclohexyl, Cycloheptyl, Cyclooktyl, Cyclononyl, Bicyclo[4.3.0]nonyl, Bicyclo-[5.2.0]nonyl oder Bicyclo[2.2.2.]oktyl.

Besonders bemerkenswert ist die Schutzwirkung von Chinolin-Derivaten der Formel I gegenüber solchen Herbiziden der Formel II, in denen Y für die Gruppen $-O-R^{18}$, $-S-R^{18}$, oder $-O-N=CR^{19}R^{20}$ steht, wobei $R^{18}$ Wasserstoff, $C_1$-$C_4$-Alkyl, $C_3$-$C_4$-Alkinyl oder durch $C_1$-$C_4$-Alkoxycarbonyl oder Di-$C_1$-$C_4$-alkylamino substituiertes $C_1$-$C_4$-Alkyl und

$R^{19}$ und $R^{20}$ unabhängig voneinander $C_1$-$C_4$-Alkyl oder

$R^{19}$ und $R^{20}$ zusammen eine $C_4$-$C_7$-Alkylenkette bedeuten.

Besonders hervorzuhebende Einzelbedeutungen für Y sind dabei Methoxy, Aethoxy, Propyloxy, Isopropyloxy, Butyloxy, Dimethylaminoäthoxy, Propargyloxy, 1-Cyano-1-methyläthoxy, Methoxycarbonylmethylthio, 1-Aethoxycarbonyläthoxy, Butyloxycarbonyl, $-O-N-C(CH_3)_2$, $-O-N=C(CH_3)C_2H_5$ oder $-O-N=C(CH_2)_5$.

Das optisch aktive Kohlenstoffatom der Propionsäuregruppe hat üblicherweise sowohl R- als auch S-Konfiguration. Ohne besondere Angabe sind hierin die racemischen Gemische gemeint. Bevorzugte Herbizide der Formel II sind 2R-konfiguriert.

Beispiele für herbizid wirksame 2-[4-(5-Chlor-3-fluorpyridin-2-yloxy)-phenoxy]-propionsäure-Derivate, gegen deren Wirkung Kulturpflanzen erfindungsgemäss geschützt werden können, sind in der nachfolgenden Tabelle 2 aufgeführt.

Tabelle 2:

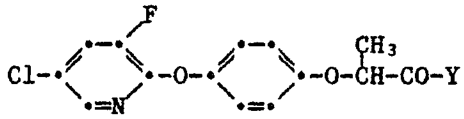

(II)

| Nr. | Y | physikalische Konstante |
|---|---|---|
| 2.1 | $-OCH_3$ | Smp. 63-64°C |
| 2.2 | $-OC_4H_9-n$ | $n_D^{35}$ = 1.5275 |
| 2.3 | $-O-N=C(CH_3)_2$ | $n_D^{35}$ = 1.5488 |
| 2.4 | $-OC_2H_5$ | $n_D^{35}$ = 1.5358 |
| 2.5 | $-O-CH_2-CH_2-N(CH_3)_2$ | $n_D^{35}$ = 1.5334 |
| 2.6 | $-O-CH_2-C\equiv CH$ | $n_D^{35}$ = 1.5492 |
| 2.7 | -O-C-CN<br>CH₃ CH₃ | $n_D^{35}$ = 1.5330 |
| 2.8 | $-S-CH_2-COOCH_3$ | $n_D^{35}$ = 1.5607 |
| 2.9 | $-O-CH-COOC_2H_5$<br>$CH_3$ | $n_D^{35}$ = 1.5227 |
| 2.10 | $-O-CH_2-COOC_4H_9-n$ | $n_D^{35}$ = 1.5223 |
| 2.11 | $-OC_3H_7-n$ | $n_D^{35}$ = 1.5319 |
| 2.12 | $-OC_3H_7-i$ | $n_D^{35}$ = 1.5284 |
| 2.13 | $-O-N=C-C_2H_5$<br>$CH_3$ | $n_D^{35}$ = 1.5340 |
| 2.14 | -O-N=C 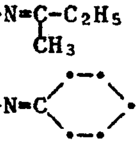 | $n_D^{35}$ = 1.5360 |
| 2.15 | $-OCH_3$ (2R) | $n_D^{35}$ = 1.5359 |
| 2.16 | -OH | Smp. 95-97°C |
| 2.17 | $-S-CH_2-COOCH_3$ (2R) | $n_D^{35}$ = 1.5623 |

Tabelle 2 (Fortsetzung)

| Nr. | Y | physikalische Konstante |
|---|---|---|
| 2.18 | $-O-CH(CH_3)-COOC_2H_5$ (2R,S) | $n_D^{35}$ = 1,5223 |
| 2.19 | $-O-CH_2-C\equiv CH$ (2R) | Smp. 55-56°C |
| 2.20 | $-NH-OCH_3$ | Smp. 103-105°C |

Als Kulturpflanzen, welche durch Chinolinderivate der Formel I gegen schädigende Wirkungen von Herbiziden der Formel II geschützt werden können, kommen insbesondere diejenigen in Betracht, die auf dem Nahrungs- oder Textilsektor von Bedeutung sind, beispielsweise Zuckerrohr und insbesondere Kulturhirse, Mais, Reis und andere Getreidearten (Weizen, Roggen, Gerste, Hafer). Ganz besonders ist an dieser Stelle die Verwendung in Weizen, Roggen, Gerste und Reis herauszustellen.

Eine bevorzugte Ausführungsform des erfindungsgemässen Verfahrens besteht in der Verwendung von
2-Chinolin-8-yloxy-essigsäureisopropylester,
2-(5-Chlorchinolin-8-yloxy)-essigsäure-n-dodecylester,
2-(5-Chlorchinolin-8-yloxy)-essigsäure-n-butylester,
2-(5-Chlorchinolin-8-yloxy)-essigsäure-n-oktylester,
2-Chinolin-8-yloxy-essigsäure-s-butylester,
2-(5-Chlorchinolin-8-yloxy)-thioessigsäure-n-oktylester,
2-(5-Chlorchinolin-8-yloxy)-essigsäure-(2-butenyl)-ester,
2-(5-Chlorchinolin-8-yloxy)-essigsäuremethallylester,
2-(5-Chlorchinolin-8-yloxy)-essigsäure-(2-isopropyloxyäthyl)-ester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäurecyclohexylester,
2-(5-Chlorchinolin-8-yloxy)-essigsäure-s-butylester,
2-(5-Chlorchinolin-8-yloxy)-essigsäure-(2-methylpentyl)-ester,
2-(5-Chlorchinolin-8-yloxy)-thioessigsäure-n- butylester,
2-(5-Chlorchinolin-8-yloxy)-essigsäure-(3,6-dioxadecyl)-ester,
2-(5-Chlorchinolin-8-yloxy)-essigsäure-(3-methoxybutyl)-ester,
2-(5-Chlorchinolin-8-yloxy)-essigsäure-(1-äthylbutyl)-ester,
2-(5-Chlorchinolin-8-yloxy)-essigsäure-(2-äthylbutyl)-ester,
2-(5-Chlorchinolin-8-yloxy)-essigsäure-(1-methylisopentyl)-ester,
2-(5-Chlorchinolin-8-yloxy)-essigsäure-n-undecylester,
2-(5-Chlorchinolin-8-yloxy)-essigsäure-(2-methylbutyl)-ester,
2-(5-Chlorchinolin-8-yloxy)-thioessigsäure-s-butylester,
2-(5-Chlorchinolin-8-yloxy)-essigsäure-(3,6-dioxaheptyl)-ester,
2-(5-Chlorchinolin-8-yloxy)-essigsäure-n-heptylester,
2-(5-Chlorchinolin-8-yloxy)-thioessigsäure-n-dodecylester,
2-(5-Chlorchinolin-8-yloxy)-thioessigsäure-n-decylester,
2-(5-Chlorchinolin-8-yloxy)-essigsäure-(1-propylpropargyl)-ester,
2-(5-Chlorchinolin-8-yloxy)-essigsäure-(1-methylisobutyl)-ester,
2-(5-Chlorchinolin-8-yloxy)-thioessigsäure-tert.butylester,
2-(5-Chlorchinolin-8-yloxy)-essigsäure-neopentylester,
2-(5-Chlorchinolin-8-yloxy)-thioessigsäure-n-propylester,
2-(5-Chlorchinolin-8-yloxy)-essigsäure-(1-methylhexyl)-ester,
2-(5-Chlorchinolin-8-yloxy)-thioessigsäureäthylester,
2-(5-Chlorchinolin-8-yloxy)-essigsäure-(2-äthylhexyl)-ester,
2-(5-Chlorchinolin-8-yloxy)-thioessigsäure-i-butylester,
2-Chinolin-8-yloxy-thioessigsäure-n-decylester,

2-(5-Chlorchinolin-8-yloxy)-essigsäure-i-pentylester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-thioessigsäure-n-hexylester,
2-(5-Chlorchinolin-8-yloxy)-essigsäure-n-hexylester,
2-(5-Chlorchinolin-8-yloxy)-thioessigsäure-i-propylester,
2-(5-Chlorchinolin-8-yloxy)-essigsäure-(1-pentylallyl)-ester,
2-(5-Chlorchinolin-8-yloxy)-essigsäure-(1-methylpentyl)-ester,
2-(5-Chlorchinolin-8-yloxy)-essigsäure-(1,1-dimethylpropargyl)-ester,
2-(5-Chlorchinolin-8-yloxy)-essigsäure-(1-äthyl-1-methylpropargyl)-ester,
2-(5-Chlorchinolin-8-yloxy)-essigsäure-n-butyloxycarbonylmethylester,
2-(5-Chlorchinolin-8-yloxy)-essigsäure-(1-n-butyloxycarbonyläthyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-[1-methyl-2-(2-methylphenoxy)-äthyl]-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-[1-methyl-2-( 2-isopropylphenoxy)-äthyl]-ester,
2-(5-Chlorchinolin-8-yloxy)-essigsäure-(1-phenylpropyl)-ester,
2-(5-Chlorchinolin-8-yloxy)-essigsäure-[1-methyl-2-(äthylphenoxy)-äthyl]-ester,
2-(5-Chlorchinolin-8-yloxy)-essigsäure-[1-methyl-2-(3-äthylphenoxy)-äthyl]-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenylpropyl)-ester,
2-(5-Chlorchinolin-8-yloxy)-essigsäure-[1-methyl-2-(3-methylphenoxy)-äthyl]-ester,
2-(5-Chlorchinolin-8-yloxy)-essigsäure-[1-methyl-2-(4-isopropylphenoxy)-äthyl]-ester oder
2-(5-Chlorchinolin-8-yloxy)-essigsäure-[1-methyl-2-(4-methylphenoxy)-äthyl]-ester
zum Schützen von Kulturpflanzen, insbesondere Getreide, gegen die schädigende Wirkung von 2-[4-(5-Chlor-3-fluorpyridin-2-yloxy)-phenoxy]-propionsäuremethylester, 2-[4-(5-Chlor-3-fluorpyridin-2-yloxy)-phenoxy]-propionsäurepropargylester, 2-[4-(5-Chlor-3-fluorpyridin-2-yloxy)-phenoxy]-thiopropionsäure-S-methoxycarbonylmethylester oder 2-[4-(5-Chlor-3-fluorpyridin-2-yloxy)-phenoxy]-propionsäure-(1-äthoxycarbonyläthyl)-ester.

Wegen des hervorragenden erzielbaren Ergebnisses wird der Anwender vorzugsweise die Verbindungen
2-(5-Chlorchinolin-8-yloxy)-essigsäuremethallylester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäure-(1-äthylbutyl)-ester,
2-(5-Chlorchinolin-8-yloxy)-essigsäure-(1-methylisopentyl)-ester,
2-(5-Chlorchinolin-8-yloxy)-essigsäure-(1-methylhexyl)-ester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-essigsäure-(1-pentylallyl)-ester,
2-(5-Chlorchinolin-8-yloxy)-essigsäure-(1-methylpentyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-(1-phenylpropyl)-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenylpropyl)-ester oder
2-(5-Chlorchinolin-8-yloxy)-essigsäure-[1-methyl-2-(4-methylphenoxy)-äthyl]-ester
zum Schützen von Kulturpflanzen, insbesondere Getreide, gegen die schädigende Wirkung von 2-[4-(5-Chlor-3-fluorpyridin-2-yloxy)-phenoxy]-propionsäuremethylester, 2-[4-(5-Chlor-3-fluorpyridin-2-yloxy)-phenoxy]-propionsäurepropargylester, 2-[4-(5-Chlor-3-fluorpyridin-2-yloxy)-phenoxy]-thiopropionsäure-S-methoxycarbonylmethylester oder 2-[4-(5-Chlor-3-fluorpyridin-2-yloxy)-phenoxy]-propionsäure-(1-äthoxycarbonyläthyl)-ester einsetzen.

Ein geeignetes Verfahren zum Schützen von Kulturpflanzen unter Verwendung von Verbindungen der Formel I besteht darin, dass man Kulturpflanzen, Teile dieser Pflanzen oder für den Anbau der Kulturpflanzen bestimmte Böden vor oder nach dem Einbringen des pflanzlichen Materials in den Boden mit einer Verbindung der Formel I oder einem Mittel, welches eine solche Verbindung enthält, behandelt. Die Behandlung kann vor, gleichzeitig mit oder nach dem Einsatz des Herbizids der Formel II erfolgen. Als Pflanzenteile kommen insbesondere diejenigen in Betracht, die zur Neubildung einer Pflanze befähigt sind, wie beispielsweise Samen, Früchte, Stengelteile und Zweige (Stecklinge) sowie auch Wurzeln, Knollen und Rhizome.

Die Erfindung betrifft auch ein Verfahren zur selektiven Bekämpfung von Unkräutern in Kulturpflanzenbeständen, wobei die Kulturpflanzenbestände, Teile der Kulturpflanzen oder Anbauflächen für Kulturpflanzen mit einem Herbizid der Formel II und einer Verbindung der Formel I oder einem Mittel, welches eine Kcmbination aus einem solchen Herbizid und einer Verbindung der Formel I enthält, behandelt.

Gegenstand der vorliegenden Erfindung sind ebenfalls herbizide Mittel, welche eine Kombination der antagonistischen Komponente I und der herbiziden Komponente II enthalten.

Vorzugsweise enthalten solche Mittel als antagonistische Komponente eine Verbindung aus der Reihe
2-Chinolin-8-yloxy-essigsäureisopropylester,
2-(5-Chlorchinolin-8-yloxy)-essigsäure-n-dodecylester,
2-(5-Chlorchinolin-8-yloxy)-essigsäure-n-butylester,
2-(5-Chlorchinolin-8-yloxy)-essigsäure-n-oktylester,
2-Chinolin-8-yloxy-essigsäure-s-butylester,
2-(5-Chlorchinolin-8-yloxy)-thioessigsäure-n-oktylester,
2-(5-Chlorchinolin-8-yloxy)-essigsäure-(2-butenyl)-ester,
2-(5-Chlorchinolin-8-yloxy)-essigsäuremethallylester,
2-(5-Chlorchinolin-8-yloxy)-essigsäure-(2-isopropyloxyäthyl)-ester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäurecyclohexylester,
2-(5-Chlorchinolin-8-yloxy)-essigsäure-s-butylester,
2-(5-Chlorchinolin-8-yloxy)-essigsäure-(2-methylpentyl)-ester,
2-(5-Chlorchinolin-8-yloxy)-thioessigsäure-n-butylester,
2-(5-Chlorchinolin-8-yloxy)-essigsäure-(3,6-dioxadecyl)-ester,
2-(5-Chlorchinolin-8-yloxy)-essigsäure-(3-methoxybutyl)-ester,
2-(5-Chlorchinolin-8-yloxy)-essigsäure-(1-äthylbutyl)-ester,
2-(5-Chlorchinolin-8-yloxy)-essigsäure-(2-äthylbutyl)-ester,
2-(5-Chlorchinolin-8-yloxy)-essigsäure-(1-methylisopentyl)-ester,
2-(5-Chlorchinolin-8-yloxy)-essigsäure-n-undecylester,
2-(5-Chlorchinolin-8-yloxy)-essigsäure-(2-methylbutyl)-ester,
2-(5-Chlorchinolin-8-yloxy)-thioessigsäure-s-butylester,
2-(5-Chlorchinolin-8-yloxy)-essigsäure-(3,6-dioxaheptyl)-ester,
2-(5-Chlorchinolin-8-yloxy)-essigsäure-n-heptylester,
2-(5-Chlorchinolin-8-yloxy)-thioessigsäure-n-dodecylester,
2-(5-Chlorchinolin-8-yloxy)-thioessigsäure-n-decylester,
2-(5-Chlorchinolin-8-yloxy)-essigsäure-(1-propylpropargyl)-ester,
2-(5-Chlorchinolin-8-yloxy)-essigsäure-(1-methylisobutyl)-ester,
2-(5-Chlorchinolin-8-yloxy)-thioessigsäure-tert.butylester,
2-(5-Chlorchinolin-8-yloxy)-essigsäure-neopentylester,
2-(5-Chlorchinolin-8-yloxy)-thioessigsäure-n-propylester,
2-(5-Chlorchinolin-8-yloxy)-essigsäure-(1-methylhexyl)-ester,
2-(5-Chlorchinolin-8-yloxy)-thioessigsäureäthylester,
2-(5-Chlorchinolin-8-yloxy)-essigsäure-(2-äthylhexyl)-ester,
2-(5-Chlorchinolin-8-yloxy)-thioessigsäure-i-butylester,
2-Chinolin-8-yloxy-thioessigsäure-n-decylester,
2-(5-Chlorchinolin-8-yloxy)-essigsäure-i-pentylester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-thioessigsäure-n-hexylester,
2-(5-Chlorchinolin-8-yloxy)-essigsäure-n-hexylester,
2-(5-Chlorchinolin-8-yloxy)-thioessigsäure-i-propylester,

2-(5-Chlorchinolin-8-yloxy)-essigsäure-(1-pentylallyl)-ester,
2-(5-Chlorchinolin-8-yloxy)-essigsäure-(1-methypentyl)-ester,
2-(5-Chlorchinolin-8-yloxy)-essigsäure-(1,1-dimethylpropargyl)-ester,
2-(5-Chlorchinolin-8-yloxy)-essigsäure-(1-äthyl-1-methylpropargyl)-ester,
2-(5-Chlorchinolin-8-yloxy)-essigsäure-n-butyloxycarbonylmethylester,
2-(5-Chlorchinolin-8-yloxy)-essigsäure-(1-n-butyloxycarbonyläthyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-[1-methyl-2-(2-methylphenoxy)-äthyl]-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-[1-methyl-2-(2-isopropylphenoxy)-äthyl]-ester,
2-(5-Chlorchinolin-8-yloxy)-essigsäure-(1-phenylpropyl)-ester,
2-(5-Chlorchinolin-8-yloxy)-essigsäure-[1-methyl-2-(2-äthylphenoxy)-äthyl]-ester,
2-(5-Chlorchinolin-8-yloxy)-essigsäure-[1-methyl-2-(3-äthylphenoxy)-äthyl]-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenylpropyl)-ester,
2-(5-Chlorchinolin-8-yloxy)-essigsäure-[1-methyl-2-(3-methylphenoxy)-äthyl]-ester,
2-(5-Chlorchinolin-8-yloxy)-essigsäure-[1-methyl-2-(4-isopropylphenoxy)-äthyl]-ester oder
2-(5-Chlorchinolin-8-yloxy)-essigsäure-[1-methyl-2-(4-methylphenoxy)-äthyl]-ester
und als Herbizidkomponente eine Verbindung aus der Reihe
2-[4-(5-Chlor-3-fluorpyridin-2-yloxy)-phenoxy]-propionsäurmethylester, 2-[4-(5-Chlor-3-fluorpyridin-2-yloxy)-phenoxy]-propionsäurepropargylester, 2-[4-(5-Chlor-3-fluorpyridin-2-yloxy)-phenoxy]-thiopropionsäure-S-methoxycarbonylmethylester oder 2-[4-(5-Chlor-3-fluorpyridin-2-yloxy)-phenoxy]-propionsäure-(1-äthoxycarbonyläthyl)-ester.

Besonders bevorzugt sind unter diesem Mittel diejenigen, die als antagonistische Komponente die Verbindungen
2-(5-Chlorchinolin-8-yloxy)-essigsäuremethallylester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäure-(1-äthylbutyl)-ester,
2-(5-Chlorchinolin-8-yloxy)-essigsäure-(1-methylisopentyl)-ester,
2-(5-Chlorchinolin-8-yloxy)-essigsäure-(1-methylhexyl)-ester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-essigsäure-(1-pentylallyl)-ester,
2-(5-Chlorchinolin-8-yloxy)-essigsäure-(1-methylpentyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-(1-phenylpropyl)-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenylpropyl)-ester oder
2-(5-Chlorchinolin-8-yloxy)-essigsäure-[1-methyl-2-(4-methylphenoxy)-äthyl]-ester
und als herbizide Komponente die Verbindungen
2-[4-(5-Chlor-3-fluorpyridin-2-yloxy)-phenoxy]-propionsäurmethylester, 2-[4-(5-Chlor-3-fluorpyridin-2-yloxy)-phenoxy]-propionsäurepropargylester, 2-[4-(5-Chlor-3-fluorpyridin-2-yloxy)-phenoxy]-thiopropionsäure-S-methoxycarbonylmethylester oder 2-[4-(5-Chlor-3-fluorpyridin-2-yloxy)-phenoxy]-propionsäure-(1-äthoxycarbonyläthyl)-ester enthalten.

Von diesen Mitteln geniessen weiterhin solche Mittel den Vorzug, die die als antagonistischen Wirkstoff
2-(5-Chlorchinolin-8-yloxy)-essigsäuremethallylester,
2-(5-Chlorchinolin-8-yloxy)-essigsäure-(2-phenoxyäthyl)-ester,
2-(5-Chlorchinolin-8-yloxy)-essigsäure-(1-methylisopentyl)-ester
oder
2-(5-Chlorchinolin-8-yloxy)-essigsäure-(1-methylhexyl)-ester

enthalten.

Bei den zu bekämpfenden Unkräutern kann es sich sowohl um monokotyle wie um dikotyle Unkräuter handeln.

Als Kulturpflanzen oder Teile dieser Pflanzen kommen beispielsweise die vorstehend genannten in Betracht. Als Anbauflächen gelten die bereits mit den Kulturpflanzen bewachsenen oder mit dem Saatgut dieser Kulturpflanzen beschickten Bodenareale, wie auch die zur Bebauung mit diesen Kulturpflanzen bestimmten Böden.

Die zu applizierende Aufwandmenge Antidot im Verhältnis zum Herbizid richtet sich weitgehend nach der Anwendungsart. Bei einer Feldbehandlung, welche entweder unter Verwendung einer Tankmischung mit einer Kombination von Antidot und Herbizid oder durch getrennte Applikation von Antidot und Herbizid erfolgt, liegt in der Regel ein Verhältnis von Antidot zu Herbizid von 1:100 bis 10:1, bevorzugt 1:20 bis 1:1, und insbesondere 1:1, vor. Dagegen werden bei der Samenbeizung weit geringere Mengen Antidot im Verhältnis zur Aufwandmenge an Herbizid pro Hektar Anbaufläche benötigt.

In der Regel werden bei der Feldbehandlung 0,01 bis 10 kg Antidot/ha, vorzugsweise 0,05 bis 0,5 kg Antidot/ha, appliziert,

Bei der Samenbeizung werden im allgemeinen 0,01 bis 10 g Antidot/kg Samen, vorzugsweise 0,05 bis 2 g Antidot/kg Samen, appliziert. Wird das Antidot in flüssiger Form kurz vor der Aussaat unter Samenquellung appliziert, so werden zweckmässigerweise Antidot-Lösungen verwendet, welche den Wirkstoff in einer Konzentration von 1 bis 10 000, vorzugsweise von 100 bis 1 000 ppm, enthalten.

Zur Applikation werden die Verbindungen der Formel I oder Kombinationen von Verbindungen der Formel I mit den zu antagonisierenden Herbiziden zweckmässigerweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen, in bekannter Weise verarbeitet. Die Anwendungsverfahren wie Versprühen, Vernebeln, Verstäuben, Verstreuen, Bestreichen oder Giessen werden gleich wie die Art der zu verwendenden Mittel den angestrebten Zielen und den gegebenen Verhältnissen entsprechend gewählt.

Die Formulierungen, d.h. die den Wirkstoff der Formel I oder eine Kombination von Wirkstoff der Formel I mit zu antagonisierendem Herbizid und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmiteln, wie z.B. mit Lösungsmitteln, festen Trägerstoffen, und gegebenenfalls oberflächenaktiven Verbindungen (Tensiden).

Als Lösungsmittel können in Frage kommen; Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder

Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon,Dimethylsulfoxid oder Dimethylformamid, sowie gegebenenfalls epoxidierte Pflanzenöle wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffs der Formel I und gegebenenfalls auch dem zu antagonisierenden Herbizid nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood New Jersey, 1981.
Stache, H., "Tensid-Taschenbuch",
Carl Hanser Verlag, München/Wien, 1981.

Die agrochemischen Zubereitungen enthalten in der Regel 0,1 bis 99 Gewichtsprozent, insbesondere 0,1 bis 95 Gew.-%, Wirkstoff der Formel I oder Wirkstoffgemisch Antidot/Herbizid, 1 bis 99,9 Gew.-%, insbesondere 5 bis 99,8 Gew.-%, eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Für die Verwendung von Verbindungen der Formel I oder sie enthaltender Mittel zum Schützen von Kulturpflanzen gegen schädigende Wirkungen von Herbiziden der Formel II kommen verschiedene Methoden und Techniken in Betracht, wie beispielsweise die folgenden:

i) Samenbeizung

a) Beizung der Samen mit einem als Spritzpulver formulierten Wirkstoff der Formel I durch Schütteln in einem Gefäss bis zur gleichmässigen Verteilung auf der Samenoberfläche (Trockenbeizung). Man verwendet dabei etwa 1 bis 500 g Wirkstoff der Formel I (4 g bis 2 kg Spritzpulver) pro 100 kg Saatgut.

b) Beizung der Samen mit einem Emulsionskonzentrat des Wirksoffs der Formel I nach der Methode a) (Nassbeizung).

c) Beizung durch Tauchen des Saatguts in eine Brühe mit 50-3200 ppm Wirkstoff der Formel I während 1 bis 72 Stunden und gegebenenfalls nachfolgendes Trocknen der Samen (Tauchbeizung).

Die Beizung des Saatguts oder die Behandlung des angekeimten Sämlings sind naturgemäss die

bevorzugten Methoden der Applikation, weil die Wirkstoffbehandlung vollständig auf die Zielkultur gerichtet ist. Man verwendet in der Regel 1 bis 500 g Antidot, vorzugsweise 5 bis 250 g Antidot, pro 100 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

ii) Applikation aus Tankmischung

Eine flüssige Aufarbeitung eines Gemisches von Antidot und Herbizid (gegenseitiges Mengenverhältnis zwischen 10:1 und 1:100) wird verwendet, wobei die Aufwandmenge an Herbizid 0,1 bis 10 kg pro Hektar beträgt. Solche Tankmischung wird vor oder nach der Aussaat appliziert.

iii) Applikation in der Saatfurche

Das Antidot wird als Emulsionskonzentrat, Spritzpulver oder als Granulat in die offene besäte Saatfurche eingebracht und hierauf wird nach dem Decken der Saatfurche in normaler Weise das Herbizid im Vorauflaufverfahren appliziert.

iv) Kontrollierte Wirkstoffabgabe

Der Wirkstoff der Formel I wird in Lösung auf mineralische Granulatträger oder polymerisierte Granulate (Harnstoff/Formaldehyd) aufgezogen und trocknen gelassen. Gegebenenfalls kann ein Ueberzug aufgebracht werden (Umhüllungsgranulate), der es erlaubt, den Wirkstoff über einen bestimmten Zeitraum dosiert abzugeben.

Formulierungsbeispiel für flüssige Wirkstoffe der Formel I (% = Gewichtsprozent)

| 1. Emulsions-Konzentrate | a) | b) | c) |
|---|---|---|---|
| Wirkstoff aus Tabelle 1 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykol-äther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff aus Tabelle 1 | 80 % | 10 % | 5 % | 95 % |
| Aethylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°) | - | - | 94 % | - |

Die Lösungen sind zur Anwendung in Form kleinster Tropfen geeignet.

| 3. Granulate | a) | b) |
| --- | --- | --- |
| Wirkstoff aus Tabelle 1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 4. Stäubemittel | a) | b) |
| --- | --- | --- |
| Wirkstoff aus Tabelle 1 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemitel.

Formulierungsbeispiele für feste Wirkstoffe der Formel I (% = Gewichtsprozent)

| 5. Spritzpulver | a) | b) | c) |
| --- | --- | --- | --- |
| Wirkstoff aus Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

**6. Emulsions-Konzentrate**

| | |
|---|---|
| Wirkstoff aus Tabelle 1 | 10 % |
| Octylphenolpolyäthylenglykoläther (4-5 Mol AeO) | 3 % |
| Ca-Dodecylbenzolsulfonat | 3 % |
| Ricinusölpolyglykoläther (35 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoff aus Tabelle 1 | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

**8. Extruder-Granulate**

| | |
|---|---|
| Wirkstoff aus Tabelle 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

**9. Umhüllungs-Granulate**

| | |
|---|---|
| Wirkstoff aus Tabelle 1 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| 10. Suspensions-Konzentrate | |
|---|---|
| Wirkstoff aus Tabelle 1 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der feingemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Formulierungsbeispiele für Wirkstoffgemische (flüssig (% = Gewichtsprozent)

| 11. Emulsions-Konzentrate | a) | b) | c) |
|---|---|---|---|
| Wirkstoffgemisch:Antidot aus Tabelle 1 und ein Herbizid der Formel II im Verhältnis 1:1 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 12. Emulsions-Konzentrate | a) | b) | c) |
|---|---|---|---|
| Wirkstoffgemisch: Antidot aus Tabelle 1 und ein Herbizid der Formel II im Verhältnis 1:3 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 13. Emulsions-Konzentrate | a) | b) | c) |
|---|---|---|---|
| Wirkstoffgemisch: Antidot aus Tabelle 1 und ein Herbizid der Formel II im Verhältnis 2:1 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 14. Emulsions-Konzentrate | a) | b) | c) |
|---|---|---|---|
| Wirkstoffgemisch: Antidot aus Tabelle 1 und 2-[4-(5-Chlor-3-fluorpyridin-2-yloxy)-phenoxy]-propionsäure-methylester im Verhältnis 1:1 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 15. Emulsions-Konzentrat | a) | b) | c) |
|---|---|---|---|
| Wirkstoffgemisch: Antidot aus Tabelle 1 und 2-[4-(5-Chlor-3-fluorpyridin-2-yloxy)-phenoxy]-propionsäure-methylester im Verhältnis 1:3 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 16. Lösungen | a) | b) | c) | d) |
|---|---|---|---|---|
| Wirkstoffgemisch: Antidot aus Tabelle 1 und ein Herbizid der Formel II im Verhältnis 1:4 | 80 % | 10 % | 5 % | 95 % |
| Aethylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 17. Lösungen | a) | b) | c) | d) |
|---|---|---|---|---|
| Wirkstoffgemisch: Antidot aus Tabelle 1 und ein Herbizid der Formel II im Verhältnis 5:2 | 80 % | 10 % | 5 % | 95 % |
| Aethylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 18. Lösungen | a) | b) | c) | d) |
|---|---|---|---|---|
| Wirkstoffgemisch: Antidot aus Tabelle 1 und ein Herbizid der Formel II im Verhältnis 1:1 | 80 % | 10 % | 5 % | 95 % |
| Aethylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 19. Lösungen | a) | b) | c) | d) |
|---|---|---|---|---|
| Wirkstoffgemisch: Antidot aus Tabelle 1 und 2-[4-(5-Chlor-3-fluorpyridin-2-yloxy)-phenoxy]-propionsäure-methylester im Verhältnis 1:1 | 80 % | 10 % | 5 % | 95 % |
| Aethylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 20. Lösungen | a) | b) | c) | d) |
|---|---|---|---|---|
| Wirkstoffgemisch: Antidot aus Tabelle 1 und 2-[4-(5-Chlor-3-fluorpyridin-2-yloxy)-phenoxy]-propionsäure-methylester im Verhältnis 1:4 | 80 % | 10 % | 5 % | 95 % |
| Aethylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 21. Granulate | a) | b) |
|---|---|---|
| Wirkstoffgemisch: Antidot aus Tabelle 1 und ein Herbizid der Formel II im Verhältnis 1:1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 22. Granulate | a) | b) |
|---|---|---|
| Wirkstoffgemisch: Antidot aus Tabelle 1 und 2-[4-(5-Chlor-3-fluorpyridin-2-yloxy)-phenoxy]-propionsäure-methylester im Verhältnis 1:1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 23. Stäubemittel | a) | b) |
|---|---|---|
| Wirkstoffgemisch: Antidot aus Tabelle 1 und ein Herbizid der Formel II im Verhältnis 1:1 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für Wirkstoffgemische (fest) (% = Gewichtsprozent)

| 24. Spritzpulver | a) | b) | c) |
|---|---|---|---|
| Wirkstoffgemisch: Antidot aus Tabelle 1 und ein Herbizid der Formel II im Verhältnis 1:1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 25. Spritzpulver | a) | b) | c) |
|---|---|---|---|
| Wirkstoffgemisch: Antidot aus Tabelle 1 und ein Herbizid der Formel II im Verhältnis 1:4 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 26. Spritzpulver | a) | b) | c) |
|---|---|---|---|
| Wirkstoffgemisch: Antidot aus Tabelle 1 und ein Herbizid der Formel II im Verhältnis 3:1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 27. Emulsions-Konzentrate | |
|---|---|
| Wirkstoffgemisch: Antidot aus Tabelle 1 und ein Herbizid der Formel II im Verhältnis 1:1 | 10 % |
| Octylphenolpolyäthylenglykoläther (4-5 Mol AeO) | 3 % |
| Ca-Dodecylbenzolsulfonat | 3 % |
| Ricinusölpolyglykoläther (35 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

**28. Emulsions-Konzentrate**

| | |
|---|---|
| Wirkstoffgemisch: Antidot aus Tabelle 1 und ein Herbizid der Formel II im Verhältnis 5:2 | 10 % |
| Octylphenolpolyäthylenglykoläther (4-5 Mol AeO) | 3 % |
| Ca-Dodecylbenzolsulfonat | 3 % |
| Ricinusölpolyglykoläther (35 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

**29. Emulsions-Konzentrate**

| | |
|---|---|
| Wirkstoffgemisch: Antidot aus Tabelle 1 und ein Herbizid der Formel II im Verhältnis 1:4 | 10 % |
| Octylphenolpolyäthylenglykoläther (4-5 Mol AeO) | 3 % |
| Ca-Dodecylbenzolsulfonat | 3 % |
| Ricinusölpolyglykoläther (35 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 30. Stäubemittel | a) | b) |
|---|---|---|
| Wirkstoffgemisch: Antidot aus Tabelle 1 und ein Herbizid der Formel II im Verhältnis 1:1 | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

31. Extruder-Granulate

| | |
|---|---|
| Wirkstoffgemisch: Antidot aus Tabelle 1 und ein Herbizid der Formel II im Verhältnis 1:1 | 10 % |
| Na-Liginsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

32. Umhüllungs-Granulate

| | |
|---|---|
| Wirkstoffgemisch: Antidot aus Tabelle 1 und ein Herbizid der Formel II im Verhältnis 1:1 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

33. Suspensions-Konzentrate

| | |
|---|---|
| Wirkstoffgemisch: Antidot aus Tabelle 1 und ein Herbizid der Formel II im Verhältnis 1:1 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

34. Suspensions-Konzentrate

| | | |
|---|---|---|
| Wirkstoffgemisch: Antidot aus Tabelle 1 und ein Herbizid der Formel II im Verhältnis 1:4 | 40 | % |
| Aethylenglykol | 10 | % |
| Nonylphenolpolyäthylenglykoläther (15 Mol AeO) | 6 | % |
| Na-Ligninsulfonat | 10 | % |
| Carboxymethylcellulose | 1 | % |
| 37%ige wässrige Formaldehyd-Lösung | 0,2 | % |
| Silikonöl in Form einer 75%igen wässrigen Emulsion | 0,8 | % |
| Wasser | 32 | % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

35. Suspensions-Konzentrate

| | | |
|---|---|---|
| Wirkstoffgemisch: Antidot aus Tabelle 1 und ein Herbizid der Formel II im Verhältnis 3:1 | 40 | % |
| Aethylenglykol | 10 | % |
| Nonylphenolpolyäthylenglykoläther (15 Mol AeO) | 6 | % |
| Na-Ligninsulfonat | 10 | % |
| Carboxymethylcellulose | 1 | % |
| 37%ige wässrige Formaldehyd-Lösung | 0,2 | % |
| Silikonöl in Form einer 75%igen wässrigen Emulsion | 0,8 | % |
| Wasser | 32 | % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Testbeschreibung

Im Gewächshaus werden Plastiktöpfe, welche 0,5 1 Erde enthalten, mit Samen der zu testenden Pflanzen beschickt. Wenn die Pflanzen das 2-bis 3-Blattstadium erreicht haben, werden ein Safener der Formel I und ein Herbizid der Formel II zusammen als Tankmischung appliziert. 21 Tage nach der Applikation wird die Schutzwirkung des Safeners in Prozent bonitiert. Als Referenz dienen dabei mit dem Herbizid allein behandelte Pflanzen sowie die vollständig unbehandelte Kontrolle. Die Resultate sind in der nachfolgenden Tabelle 3 dargestellt.

Tabelle 3:

Relative Schutzwirkung in Prozent in Sommerweizen, Sorte "Besso" und Sommergerste, Sorte "Cornel".

| Safener Verb.Nr | Aufwand-menge g AS/ha | Herbizid Nr. | Aufwand-menge g AS/ha | Relative Schutzwirkung in Weizen in % | Relative Schutzwirkung in Gerste in % |
|---|---|---|---|---|---|
| 1.125 | 31 | 2.1 | 125 | 10 | 30 |
| 1.125 | 62 | 2.1 | 125 | 0 | 25 |
| 1.125 | 125 | 2.1 | 125 | 10 | 30 |
| 1.125 | 62 | 2.1 | 250 | 70 | 15 |
| 1.125 | 125 | 2.1 | 250 | 65 | 25 |
| 1.125 | 250 | 2.1 | 250 | 65 | 15 |
| 1.125 | 125 | 2.1 | 500 | 80 | 13 |
| 1.125 | 250 | 2.1 | 500 | 75 | 8 |
| 1.125 | 500 | 2.1 | 500 | 75 | 18 |
| 1.125 | 31 | 2.6 | 125 | 20 | 60 |
| 1.125 | 62 | 2.6 | 125 | 20 | 70 |
| 1.125 | 125 | 2.6 | 125 | 20 | 65 |
| 1.125 | 62 | 2.6 | 250 | 50 | 45 |
| 1.125 | 125 | 2.6 | 250 | 55 | 50 |
| 1.125 | 250 | 2.6 | 250 | 50 | 45 |
| 1.125 | 125 | 2.6 | 500 | 70 | 35 |
| 1.125 | 250 | 2.6 | 500 | 70 | 45 |
| 1.125 | 500 | 2.6 | 500 | 65 | 35 |
| 1.125 | 31 | 2.8 | 125 | 0 | 35 |
| 1.125 | 62 | 2.8 | 125 | 0 | 35 |
| 1.125 | 125 | 2.8 | 125 | 0 | 30 |
| 1.125 | 62 | 2.8 | 250 | 10 | 45 |
| 1.125 | 125 | 2.8 | 250 | 5 | 45 |
| 1.125 | 250 | 2.8 | 250 | 10 | 30 |
| 1.125 | 125 | 2.8 | 500 | 40 | 40 |
| 1.125 | 250 | 2.8 | 500 | 40 | 40 |
| 1.125 | 500 | 2.8 | 500 | 35 | 35 |
| 1.125 | 31 | 2.9 | 125 | 10 | 65 |
| 1.125 | 62 | 2.9 | 125 | 15 | 60 |
| 1.125 | 125 | 2.9 | 125 | 15 | 75 |
| 1.125 | 62 | 2.9 | 250 | 50 | 60 |
| 1.125 | 125 | 2.9 | 250 | 45 | 55 |
| 1.125 | 250 | 2.9 | 250 | 30 | 60 |
| 1.125 | 125 | 2.9 | 500 | 75 | 50 |
| 1.125 | 250 | 2.9 | 500 | 65 | 45 |
| 1.125 | 500 | 2.9 | 500 | 65 | 45 |

Tabelle 3 (Fortsetzung)

| Safener Verb.Nr | Aufwand-menge g AS/ha | Herbizid Nr. | Aufwand-menge g AS/ha | Relative Schutzwirkung in Weizen in % | Relative Schutzwirkung in Gerste in % |
|---|---|---|---|---|---|
| 1.130 | 31 | 2.1 | 125 | 5 | 5 |
| 1.130 | 62 | 2.1 | 125 | 10 | 5 |
| 1.130 | 125 | 2.1 | 125 | 0 | 5 |
| 1.130 | 62 | 2.1 | 250 | 70 | 0 |
| 1.130 | 125 | 2.1 | 250 | 60 | 0 |
| 1.130 | 250 | 2.1 | 250 | 70 | 0 |
| 1.130 | 125 | 2.1 | 500 | 70 | 8 |
| 1.130 | 250 | 2.1 | 500 | 75 | 8 |
| 1.130 | 500 | 2.1 | 500 | 80 | 8 |
| 1.130 | 31 | 2.6 | 125 | 15 | 5 |
| 1.130 | 62 | 2.6 | 125 | 20 | 5 |
| 1.130 | 125 | 2.6 | 125 | 20 | 5 |
| 1.130 | 62 | 2.6 | 250 | 65 | 0 |
| 1.130 | 125 | 2.6 | 250 | 65 | 0 |
| 1.130 | 250 | 2.6 | 250 | 65 | 0 |
| 1.130 | 125 | 2.6 | 500 | 65 | 0 |
| 1.130 | 250 | 2.6 | 500 | 75 | 0 |
| 1.130 | 500 | 2.6 | 500 | 80 | 0 |
| 1.130 | 31 | 2.8 | 125 | 0 | 15 |
| 1.130 | 62 | 2.8 | 125 | 0 | 0 |
| 1.130 | 125 | 2.8 | 125 | 0 | 0 |
| 1.130 | 62 | 2.8 | 250 | 15 | 5 |
| 1.130 | 125 | 2.8 | 250 | 15 | 0 |
| 1.130 | 250 | 2.8. | 250 | 5 | 5 |
| 1.130 | 125 | 2.8 | 500 | 40 | 0 |
| 1.130 | 250 | 2.8 | 500 | 40 | 0 |
| 1.130 | 500 | 2.8 | 500 | 40 | 0 |
| 1.130 | 31 | 2.9 | 125 | 15 | 35 |
| 1.130 | 62 | 2.9 | 125 | 15 | 35 |
| 1.130 | 125 | 2.9 | 125 | 15 | 40 |
| 1.130 | 62 | 2.9 | 250 | 50 | 5 |
| 1.130 | 125 | 2.9 | 250 | 50 | 10 |
| 1.130 | 250 | 2.9 | 250 | 45 | 10 |
| 1.130 | 125 | 2.9 | 500 | 55 | 5 |
| 1.130 | 250 | 2.9 | 500 | 60 | 5 |
| 1.130 | 500 | 2.9 | 500 | 70 | 5 |

Tabelle 3 (Fortsetzung)

| Safener Verb.Nr | Aufwand-menge g AS/ha | Herbizid Nr. | Aufwand-menge g AS/ha | Relative Schutzwirkung in Weizen in % | Relative Schutzwirkung in Gerste in % |
|---|---|---|---|---|---|
| 1.134 | 31 | 2.1 | 125 | 10 | 35 |
| 1.134 | 62 | 2.1 | 125 | 10 | 45 |
| 1.134 | 125 | 2.1 | 125 | 5 | 45 |
| 1.134 | 62 | 2.1 | 250 | 75 | 20 |
| 1.134 | 125 | 2.1 | 250 | 70 | 15 |
| 1.134 | 250 | 2.1 | 250 | 65 | 15 |
| 1.134 | 125 | 2.1 | 500 | 80 | 8 |
| 1.134 | 250 | 2.1 | 500 | 75 | 8 |
| 1.134 | 500 | 2.1 | 500 | 70 | 13 |
| 1.134 | 31 | 2.6 | 125 | 20 | 45 |
| 1.134 | 62 | 2.6 | 125 | 15 | 55 |
| 1.134 | 125 | 2.6 | 125 | 20 | 65 |
| 1.134 | 62 | 2.6 | 250 | 60 | 45 |
| 1.134 | 125 | 2.6 | 250 | 60 | 50 |
| 1.134 | 250 | 2.6 | 250 | 65 | 50 |
| 1.134 | 125 | 2.6 | 500 | 90 | 20 |
| 1.134 | 250 | 2.6 | 500 | 90 | 20 |
| 1.134 | 500 | 2.6 | 500 | 80 | 15 |
| 1.134 | 31 | 2.8 | 125 | 5 | 45 |
| 1.134 | 62 | 2.8 | 125 | 0 | 45 |
| 1.134 | 125 | 2.8 | 125 | 0 | 40 |
| 1.134 | 62 | 2.8 | 250 | 10 | 50 |
| 1.134 | 125 | 2.8 | 250 | 10 | 45 |
| 1.134 | 250 | 2.8 | 250 | 10 | 40 |
| 1.134 | 125 | 2.8 | 500 | 40 | 30 |
| 1.134 | 250 | 2.8 | 500 | 35 | 30 |
| 1.134 | 500 | 2.8 | 500 | 35 | 30 |
| 1.134 | 31 | 2.9 | 125 | 20 | 65 |
| 1.134 | 62 | 2.9 | 125 | 20 | 65 |
| 1.134 | 125 | 2.9 | 125 | 20 | 60 |
| 1.134 | 62 | 2.9 | 250 | 45 | 45 |
| 1.134 | 125 | 2.9 | 250 | 50 | 60 |
| 1.134 | 250 | 2.9 | 250 | 45 | 55 |
| 1.134 | 125 | 2.9 | 500 | 70 | 40 |
| 1.134 | 250 | 2.9 | 500 | 70 | 40 |
| 1.134 | 500 | 2.9 | 500 | 70 | 55 |

Tabelle 3 (Fortsetzung)

| Safener Verb.Nr | Aufwand-menge g AS/ha | Herbizid Nr. | Aufwand-menge g AS/ha | Relative Schutzwirkung in Weizen in % | Relative Schutzwirkung in Gerste in % |
|---|---|---|---|---|---|
| 1.186 | 31 | 2.1 | 125 | 10 | 45 |
| 1.186 | 62 | 2.1 | 125 | 15 | 35 |
| 1.186 | 125 | 2.1 | 125 | 15 | 45 |
| 1.186 | 62 | 2.1 | 250 | 75 | 15 |
| 1.186 | 125 | 2.1 | 250 | 65 | 20 |
| 1.186 | 250 | 2.1 | 250 | 70 | 15 |
| 1.186 | 125 | 2.1 | 500 | 85 | 13 |
| 1.186 | 250 | 2.1 | 500 | 85 | 13 |
| 1.186 | 500 | 2.1 | 500 | 75 | 13 |
| 1.186 | 31 | 2.6 | 125 | 20 | 50 |
| 1.186 | 62 | 2.6 | 125 | 20 | 60 |
| 1.186 | 125 | 2.6 | 125 | 20 | 60 |
| 1.186 | 62 | 2.6 | 250 | 50 | 35 |
| 1.186 | 125 | 2.6 | 250 | 55 | 45 |
| 1.186 | 250 | 2.6 | 250 | 55 | 50 |
| 1.186 | 125 | 2.6 | 500 | 90 | 25 |
| 1.186 | 250 | 2.6 | 500 | 85 | 20 |
| 1.186 | 500 | 2.6 | 500 | 70 | 20 |
| 1.186 | 31 | 2.8 | 125 | 0 | 35 |
| 1.186 | 62 | 2.8 | 125 | 0 | 45 |
| 1.186 | 125 | 2.8 | 125 | 0 | 35 |
| 1.186 | 62 | 2.8 | 250 | 0 | 35 |
| 1.186 | 125 | 2.8 | 250 | 0 | 45 |
| 1.186 | 250 | 2.8 | 250 | 0 | 40 |
| 1.186 | 125 | 2.8 | 500 | 35 | 25 |
| 1.186 | 250 | 2.8 | 500 | 35 | 25 |
| 1.186 | 500 | 2.8 | 500 | 25 | 25 |
| 1.186 | 31 | 2.9 | 125 | 20 | 40 |
| 1.186 | 62 | 2.9 | 125 | 20 | 65 |
| 1.186 | 125 | 2.9 | 125 | 20 | 60 |
| 1.186 | 62 | 2.9 | 250 | 50 | 35 |
| 1.186 | 125 | 2.9 | 250 | 40 | 45 |
| 1.186 | 250 | 2.9 | 250 | 50 | 55 |
| 1.186 | 125 | 2.9 | 500 | 70 | 40 |
| 1.186 | 250 | 2.9 | 500 | 60 | 45 |
| 1.186 | 500 | 2.9 | 500 | 55 | 50 |

Tabelle 3 (Fortsetzung)

| Safener Verb.Nr | Aufwand-menge g AS/ha | Herbizid Nr. | Aufwand-menge g AS/ha | Relative Schutzwirkung in Weizen in % | Relative Schutzwirkung in Gerste in % |
|---|---|---|---|---|---|
| 1.188 | 31 | 2.1 | 125 | 15 | 15 |
| 1.188 | 62 | 2.1 | 125 | 15 | 25 |
| 1.188 | 125 | 2.1 | 125 | 15 | 30 |
| 1.188 | 62 | 2.1 | 250 | 70 | 15 |
| 1.188 | 125 | 2.1 | 250 | 70 | 15 |
| 1.188 | 250 | 2.1 | 250 | 60 | 15 |
| 1.188 | 125 | 2.1 | 500 | 90 | 13 |
| 1.188 | 250 | 2.1 | 500 | 85 | 8 |
| 1.188 | 500 | 2.1 | 500 | 80 | 8 |
| 1.188 | 31 | 2.6 | 125 | 20 | 55 |
| 1.188 | 62 | 2.6 | 125 | 20 | 50 |
| 1.188 | 125 | 2.6 | 125 | 20 | 55 |
| 1.188 | 62 | 2.6 | 250 | 65 | 30 |
| 1.188 | 125 | 2.6 | 250 | 65 | 50 |
| 1.188 | 250 | 2.6 | 250 | 60 | 50 |
| 1.188 | 125 | 2.6 | 500 | 85 | 20 |
| 1.188 | 250 | 2.6 | 500 | 85 | 30 |
| 1.188 | 500 | 2.6 | 500 | 80 | 30 |
| 1.188 | 31 | 2.8 | 125 | 5 | 50 |
| 1.188 | 62 | 2.8 | 125 | 5 | 55 |
| 1.188 | 125 | 2.8 | 125 | 0 | 50 |
| 1.188 | 62 | 2.8 | 250 | 10 | 65 |
| 1.188 | 125 | 2.8 | 250 | 10 | 60 |
| 1.188 | 250 | 2.8 | 250 | 10 | 60 |
| 1.188 | 125 | 2.8 | 500 | 30 | 35 |
| 1.188 | 250 | 2.8 | 500 | 30 | 35 |
| 1.188 | 500 | 2.8 | 500 | 35 | 30 |
| 1.188 | 31 | 2.9 | 125 | 20 | 50 |
| 1.188 | 62 | 2.9 | 125 | 20 | 55 |
| 1.188 | 125 | 2.9 | 125 | 20 | 50 |
| 1.188 | 62 | 2.9 | 250 | 50 | 50 |
| 1.188 | 125 | 2.9 | 250 | 50 | 45 |
| 1.188 | 250 | 2.9 | 250 | 45 | 40 |
| 1.188 | 125 | 2.9 | 500 | 75 | 30 |
| 1.188 | 250 | 2.9 | 500 | 70 | 40 |
| 1.188 | 500 | 2.9 | 500 | 75 | 40 |

Tabelle 3 (Fortsetzung)

| Safener Verb.Nr | Aufwand-menge g AS/ha | Herbizid Nr. | Aufwand-menge g AS/ha | Relative Schutzwirkung in Weizen in % | Relative Schutzwirkung in Gerste in % |
|---|---|---|---|---|---|
| 1.245 | 250 | 2.1 | 500 | 70 | - |
| 1.245 | 500 | 2.1 | 500 | 65 | - |
| 1.245 | 250 | 2.1 | 1000 | 50 | - |
| 1.245 | 500 | 2.1 | 1000 | 45 | - |
| 1.245 | 62 | 2.8 | 250 | 55 | 50 |
| 1.245 | 125 | 2.8 | 250 | 65 | 55 |
| 1.245 | 125 | 2.8 | 500 | 75 | 58 |
| 1.245 | 250 | 2.8 | 500 | 90 | 48 |
| 1.247 | 250 | 2.1 | 500 | 65 | - |
| 1.247 | 500 | 2.1 | 500 | 75 | - |
| 1.247 | 250 | 2.1 | 1000 | 45 | - |
| 1.247 | 500 | 2.1 | 1000 | 65 | - |
| 1.247 | 62 | 2.8 | 250 | 70 | - |
| 1.247 | 125 | 2.8 | 250 | 70 | - |
| 1.247 | 125 | 2.8 | 500 | 80 | - |
| 1.247 | 250 | 2.8 | 500 | 80 | - |
| 1.248 | 250 | 2.1 | 500 | 65 | - |
| 1.248 | 500 | 2.1 | 500 | 65 | - |
| 1.248 | 250 | 2.1 | 1000 | 40 | - |
| 1.248 | 500 | 2.1 | 1000 | 50 | - |
| 1.248 | 62 | 2.8 | 250 | 70 | 60 |
| 1.248 | 125 | 2.8 | 250 | 70 | 75 |
| 1.248 | 125 | 2.8 | 500 | 90 | 68 |
| 1.248 | 250 | 2.8 | 500 | 90 | 73 |
| 1.255 | 62 | 2.8 | 250 | - | 70 |
| 1.255 | 125 | 2.8 | 250 | - | 70 |
| 1.255 | 125 | 2.8 | 500 | - | 35 |
| 1.255 | 250 | 2.8 | 500 | - | 50 |
| 1.256 | 250 | 2.1 | 500 | 65 | - |
| 1.256 | 500 | 2.1 | 500 | 65 | - |
| 1.256 | 250 | 2.1 | 1000 | 60 | - |
| 1.256 | 500 | 2.1 | 1000 | 50 | - |
| 1.256 | 62 | 2.8 | 250 | 60 | 65 |
| 1.256 | 125 | 2.8 | 250 | 65 | 60 |
| 1.256 | 125 | 2.8 | 500 | 85 | 43 |
| 1.256 | 250 | 2.8 | 500 | 80 | 73 |

Tabelle 3 (Fortsetzung)

| Safener Verb.Nr | Aufwand-menge g AS/ha | Herbizid Nr. | Aufwand-menge g AS/ha | Relative Schutzwirkung in Weizen in % | Relative Schutzwirkung in Gerste in % |
|---|---|---|---|---|---|
| 1.259 | 62 | 2.8 | 250 | - | 60 |
| 1.259 | 125 | 2.8 | 250 | - | 75 |
| 1.259 | 125 | 2.8 | 500 | - | 53 |
| 1.259 | 250 | 2.8 | 500 | - | 68 |
| 1.260 | 62 | 2.8 | 250 | - | 65 |
| 1.260 | 125 | 2.8 | 250 | - | 60 |
| 1.260 | 125 | 2.8 | 500 | - | 53 |
| 1.260 | 250 | 2.8 | 500 | - | 53 |
| 1.261 | 62 | 2.8 | 250 | - | 65 |
| 1.261 | 125 | 2.8 | 250 | - | 70 |
| 1.261 | 125 | 2.8 | 500 | - | 58 |
| 1.261 | 250 | 2.8 | 500 | - | 68 |
| 1.262 | 62 | 2.8 | 250 | - | 75 |
| 1.262 | 125 | 2.8 | 250 | - | 85 |
| 1.262 | 125 | 2.8 | 500 | - | 63 |
| 1.262 | 250 | 2.8 | 500 | - | 78 |
| 1.267 | 250 | 2.1 | 500 | 65 | - |
| 1.267 | 500 | 2.1 | 500 | 65 | - |
| 1.267 | 250 | 2.1 | 1000 | 55 | - |
| 1.267 | 250 | 2.1 | 1000 | 50 | - |
| 1.267 | 62 | 2.8 | 250 | 65 | 65 |
| 1.267 | 125 | 2.8 | 250 | 65 | 70 |
| 1.267 | 125 | 2.8 | 500 | 85 | 48 |
| 1.267 | 250 | 2.8 | 500 | 85 | 73 |
| 1.276 | 250 | 2.1 | 500 | 60 | - |
| 1.276 | 500 | 2.1 | 500 | 55 | - |
| 1.276 | 250 | 2.1 | 1000 | 35 | - |
| 1.276 | 500 | 2.1 | 1000 | 50 | - |
| 1.276 | 62 | 2.8 | 250 | 70 | 65 |
| 1.276 | 125 | 2.8 | 250 | 65 | 75 |
| 1.276 | 125 | 2.8 | 500 | 85 | 63 |
| 1.276 | 250 | 2.8 | 500 | 80 | 68 |

Tabelle 3 (Fortsetzung)

| Safener Verb.Nr | Aufwand-menge g AS/ha | Herbizid Nr. | Aufwand-menge g AS/ha | Relative Schutzwirkung in Weizen in % | Relative Schutzwirkung in Gerste in % |
|---|---|---|---|---|---|
| 1.284 | 250 | 2.1 | 500 | 60 | - |
| 1.284 | 500 | 2.1 | 500 | 65 | - |
| 1.284 | 250 | 2.1 | 1000 | 50 | - |
| 1.284 | 500 | 2.1 | 1000 | 45 | - |
| 1.284 | 62 | 2.8 | 250 | 70 | 60 |
| 1.284 | 125 | 2.8 | 250 | 65 | 55 |
| 1.284 | 125 | 2.8 | 500 | 75 | 63 |
| 1.284 | 250 | 2.8 | 500 | 70 | 73 |
| 1.285 | 250 | 2.1 | 500 | 55 | - |
| 1.285 | 500 | 2.1 | 500 | 65 | - |
| 1.285 | 250 | 2.1 | 1000 | 40 | - |
| 1.285 | 500 | 2.1 | 1000 | 50 | - |
| 1.285 | 62 | 2.8 | 250 | 65 | 65 |
| 1.285 | 125 | 2.8 | 250 | 65 | 65 |
| 1.285 | 125 | 2.8 | 500 | 80 | 68 |
| 1.285 | 250 | 2.8 | 500 | 85 | 78 |
| 1.290 | 250 | 2.1 | 500 | 60 | - |
| 1.290 | 500 | 2.1 | 500 | 60 | - |
| 1.290 | 250 | 2.1 | 1000 | 45 | - |
| 1.290 | 500 | 2.1 | 1000 | 60 | - |
| 1.290 | 62 | 2.8 | 250 | 50 | 70 |
| 1.290 | 125 | 2.8 | 250 | 65 | 75 |
| 1.290 | 125 | 2.8 | 500 | 80 | 63 |
| 1.290 | 250 | 2.8 | 500 | 85 | 73 |
| 1.293 | 250 | 2.1 | 500 | 60 | - |
| 1.293 | 500 | 2.1 | 500 | 45 | - |
| 1.293 | 250 | 2.1 | 1000 | 45 | - |
| 1.293 | 500 | 2.1 | 1000 | 70 | - |
| 1.293 | 62 | 2.8 | 250 | 50 | 60 |
| 1.293 | 125 | 2.8 | 250 | 55 | 65 |
| 1.293 | 125 | 2.8 | 500 | 55 | 48 |
| 1.293 | 250 | 2.8 | 500 | 80 | 53 |

Tabelle 3 (Fortsetzung)

| Safener Verb.Nr | Aufwand-menge g AS/ha | Herbizid Nr. | Aufwand-menge g AS/ha | Relative Schutzwirkung in Weizen in % | Relative Schutzwirkung in Gerste in % |
|---|---|---|---|---|---|
| 1.301 | 250 | 2.1 | 500 | 70 | - |
| 1.301 | 500 | 2.1 | 500 | 75 | - |
| 1.301 | 250 | 2.1 | 1000 | 50 | - |
| 1.301 | 500 | 2.1 | 1000 | 45 | - |
| 1.301 | 62 | 2.8 | 250 | 60 | - |
| 1.301 | 125 | 2.8 | 250 | 65 | - |
| 1.301 | 125 | 2.8 | 500 | 70 | - |
| 1.301 | 250 | 2.8 | 500 | 75 | - |
| 1.305 | 62 | 2.8 | 250 | - | 65 |
| 1.305 | 125 | 2.8 | 250 | - | 70 |
| 1.305 | 125 | 2.8 | 500 | - | 68 |
| 1.305 | 250 | 2.8 | 500 | - | 73 |
| 1.308 | 62 | 2.8 | 250 | - | 90 |
| 1.308 | 125 | 2.8 | 250 | - | 90 |
| 1.308 | 125 | 2.8 | 500 | - | 63 |
| 1.308 | 250 | 2.8 | 500 | - | 73 |
| 1.314 | 62 | 2.8 | 250 | - | 80 |
| 1.314 | 125 | 2.8 | 250 | - | 90 |
| 1.314 | 125 | 2.8 | 500 | - | 58 |
| 1.314 | 250 | 2.8 | 500 | - | 63 |
| 1.316 | 250 | 2.1 | 500 | 65 | - |
| 1.316 | 500 | 2.1 | 500 | 65 | - |
| 1.316 | 250 | 2.1 | 1000 | 35 | - |
| 1.316 | 500 | 2.1 | 1000 | 50 | - |
| 1.316 | 62 | 2.8 | 250 | - | 50 |
| 1.316 | 125 | 2.8 | 250 | - | 50 |
| 1.316 | 125 | 2.8 | 500 | - | 55 |
| 1.316 | 250 | 2.8 | 500 | - | 60 |
| 1.321 | 62 | 2.8 | 250 | - | 65 |
| 1.321 | 125 | 2.8 | 250 | - | 80 |
| 1.321 | 125 | 2.8 | 500 | - | 60 |
| 1.321 | 250 | 2.8 | 500 | - | 70 |
| 1.325 | 250 | 2.1 | 500 | 60 | - |
| 1.325 | 500 | 2.1 | 500 | 50 | - |
| 1.325 | 250 | 2.1 | 1000 | 50 | - |
| 1.325 | 500 | 2.1 | 1000 | 70 | - |

Tabelle 3 (Fortsetzung)

| Safener Verb.Nr | Aufwand-menge g AS/ha | Herbizid Nr. | Aufwand-menge g AS/ha | Relative Schutzwirkung in Weizen in % | Relative Schutzwirkung in Gerste in % |
|---|---|---|---|---|---|
| 1.327 | 62 | 2.8 | 250 | - | 70 |
| 1.327 | 125 | 2.8 | 250 | - | 80 |
| 1.327 | 125 | 2.8 | 500 | - | 50 |
| 1.327 | 250 | 2.8 | 500 | - | 50 |
| 1.333 | 250 | 2.1 | 500 | 63 | - |
| 1.333 | 500 | 2.1 | 500 | 73 | - |
| 1.333 | 250 | 2.1 | 1000 | 35 | - |
| 1.333 | 500 | 2.1 | 1000 | 55 | - |
| 1.334 | 62 | 2.8 | 250 | - | 75 |
| 1.334 | 125 | 2.8 | 250 | - | 85 |
| 1.334 | 125 | 2.8 | 500 | - | 63 |
| 1.334 | 250 | 2.8 | 500 | - | 63 |
| 1.336 | 250 | 2.1 | 500 | 70 | - |
| 1.336 | 500 | 2.1 | 500 | 75 | - |
| 1.336 | 250 | 2.1 | 1000 | 45 | - |
| 1.336 | 500 | 2.1 | 1000 | 45 | - |
| 1.336 | 62 | 2.8 | 250 | 65 | 60 |
| 1.336 | 125 | 2.8 | 250 | 65 | 60 |
| 1.336 | 125 | 2.8 | 500 | 85 | 53 |
| 1.336 | 250 | 2.8 | 500 | 85 | 23 |
| 1.337 | 250 | 2.1 | 500 | 60 | - |
| 1.337 | 500 | 2.1 | 500 | 55 | - |
| 1.337 | 250 | 2.1 | 1000 | 45 | - |
| 1.337 | 500 | 2.1 | 1000 | 50 | - |
| 1.337 | 62 | 2.8 | 250 | 65 | 65 |
| 1.337 | 125 | 2.8 | 250 | 55 | 50 |
| 1.337 | 125 | 2.8 | 500 | 70 | 63 |
| 1.337 | 250 | 2.8 | 500 | 60 | 78 |
| 1.341 | 250 | 2.1 | 500 | 58 | - |
| 1.341 | 500 | 2.1 | 500 | 73 | - |
| 1.341 | 250 | 2.1 | 1000 | 25 | - |
| 1.341 | 500 | 2.1 | 1000 | 60 | - |
| 1.341 | 62 | 2.8 | 250 | - | 90 |
| 1.341 | 125 | 2.8 | 250 | - | 90 |
| 1.341 | 125 | 2.8 | 500 | - | 63 |
| 1.341 | 250 | 2.8 | 500 | - | 68 |

Tabelle 3 (Fortsetzung)

| Safener Verb.Nr | Aufwand-menge g AS/ha | Herbizid Nr. | Aufwand-menge g AS/ha | Relative Schutzwirkung in Weizen in % | Relative Schutzwirkung in Gerste in % |
|---|---|---|---|---|---|
| 1.353 | 62 | 2.8 | 250 | - | 65 |
| 1.353 | 125 | 2.8 | 250 | - | 75 |
| 1.353 | 125 | 2.8 | 500 | - | 65 |
| 1.353 | 250 | 2.8 | 500 | - | 60 |
| 1.355 | 250 | 2.1 | 500 | 78 | - |
| 1.355 | 500 | 2.1 | 500 | 78 | - |
| 1.355 | 250 | 2.1 | 1000 | 45 | - |
| 1.355 | 500 | 2.1 | 1000 | 55 | - |
| 1.355 | 62 | 2.8 | 250 | 50 | - |
| 1.355 | 125 | 2.8 | 250 | 55 | - |
| 1.355 | 125 | 2.8 | 500 | 45 | - |
| 1.355 | 250 | 2.8 | 500 | 55 | - |
| 1.362 | 62 | 2.8 | 250 | - | 90 |
| 1.362 | 125 | 2.8 | 250 | - | 90 |
| 1.362 | 125 | 2.8 | 500 | - | 63 |
| 1.362 | 250 | 2.8 | 500 | - | 73 |
| 1.363 | 62 | 2.8 | 250 | - | 80 |
| 1.363 | 125 | 2.8 | 250 | - | 80 |
| 1.363 | 125 | 2.8 | 500 | - | 63 |
| 1.363 | 250 | 2.8 | 500 | - | 63 |

- : nicht geprüft.

## Patentansprüche

**1.** Verfahren zum Schützen von Kulturpflanzen gegen schädigende Wirkungen herbizid wirksamer 2-[4-(5-Chlor-3-fluorpyridin-2-yloxy)-phenoxy]-propionsäure Derivate der Formel II

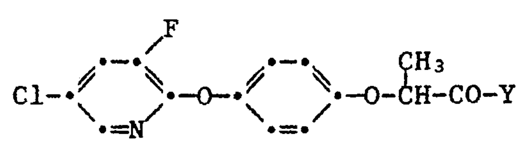

(II)

worin Y für eine Gruppe $-NR^{16}R^{17}$, $-O-R^{18}$, $-S-R^{18}$ oder $-O-N=CR^{19}R^{20}$ steht,
$R^{16}$ und $R^{17}$ unabhängig voneinander Wasserstoff, $C_1$-$C_8$-Alkoxy, $C_1$-$C_8$-Alkyl, Phenyl oder Benzyl,
$R^{16}$ uns $R^{17}$ zusammen mit dem sie tragenden Stickstoffatom einen 5-bis 6-gliedrigen gesättigten Stickstoffheterocyclus, der durch ein Sauerstoff oder Schwefelatom unterbrochen sein kann,
$R^{18}$ Wasserstoff oder das Aequivalent eines Alkalimetall-, Erdalkalimetall-, Kupfer- oder Eisen-Ions; einen quaternären $C_1$-$C_4$-Alkylammonium- oder $C_1$-$C_4$-Hydroxyalkylammonium-Rest; einen gegebenenfalls ein- oder mehrfach durch Amino, Halogen, Hydroxyl, Cyan, Nitro, Phenyl, $C_1$-$C_4$-Alkoxy, Polyäthoxy mit 2 bis 6 Aethylenoxideinheiten, $-COOR^{21}$, $-COSR^{21}$, $-CONH_2$-, $-CON(C_1$-$C_4$-alkoxy$)$-$C_1$-$C_4$-alkyl,

-CO-N-di-$C_1$-$C_4$-alkyl, -CONH-$C_1$-$C_4$-alkyl, -N($C_1$-$C_4$-alkoxy)-$C_1$-$C_4$-alkyl oder Di-$C_1$-$C_4$-alkylamino substituierten $C_1$-$C_9$-Alkylrest;
einen gegebenenfalls durch Halogen oder $C_1$-$C_4$-Alkoxy substituierten $C_3$-$C_9$-Alkenylrest;
einen gegebenenfalls durch Halogen oder $C_1$-$C_4$-Alkoxy substituierten $C_3$-$C_9$-Alkinylrest;
$C_3$-$C_9$-Cycloalkyl; oder gegebenenfalls durch Cyan, $C_1$-$C_4$-Alkyl, $C_1$-$C_4$-Alkoxy, Acetyl, -$COOR^{21}$, -$COSR^{21}$, -$CONH_2$, -CON($C_1$-$C_4$-alkoxy)-$C_1$-$C_4$-alkyl, -CO-N-di-$C_1$-$C_4$-alkyl oder -CONH-$C_1$-$C_4$-alkyl substituiertes Phenyl,
$R^{19}$ und $R^{20}$ unabhängig voneinander $C_1$-$C_4$-Alkyl oder zusammen eine 3-bis 6-gliedrige Alkylenkette und
$R^{21}$ Wasserstoff, $C_1$-$C_6$-Alkyl, $C_1$-$C_6$-Halogenalkyl, $C_2$-$C_6$-Alkoxyalkyl, $C_3$-$C_6$-Alkenyl, $C_3$-$C_6$-Halogenalkenyl, $C_3$-$C_6$-Alkinyl oder $C_3$-$C_6$-Halogenalkinyl bedeuten, dadurch gekennzeichnet, dass man die Kulturpflanzen, Teile dieser Pflanzen oder für den Anbau der Kulturpflanzen bestimmte Böden mit einer Verbindung der Formel I

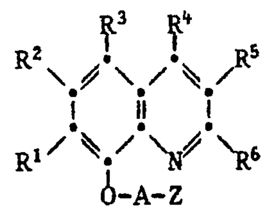

(I),

worin $R^1$, $R^2$ und $R^3$ unabhängig voneinander Wasserstoff, Halogen, Nitro, Cyano, $C_1$-$C_3$-Alkyl oder $C_1$-$C_3$-Alkoxy,
$R^4$, $R^5$ und $R^6$ unabhängig voneinander Wasserstoff, Halogen oder $C_1$-$C_3$-Alkyl,
A eine der Gruppen -$CH_2$-, -$CH_2$-$CH_2$- oder -$CH(CH_3)$- und
Z Cyan oder Amidoxim, welches am Sauerstoffatom acyliert sein kann, eine Carboxylgruppe oder ein Salz davon, eine Mercaptocarbonylgruppe oder ein Salz davon, eine Carbonsäureestergruppe, eine Carbonsäurethiolestergruppe,eine unsubstituierte oder substituierte Carbonsäureamidgruppe, ein cyclisiertes, unsubstituiertes oder substituiertes Derivat einer Carbonsäureamidgruppe oder eine Carbonsäurehydrazidgruppe oder
A und Z zusammen einen unsubstituierten oder substituierten Tetrahydrofuran-2-on-Ring bedeuten, unter Einschluss ihrer Säureadditionssalze und Metallkomplexe, oder einem Mittel, welches eine dieser Verbindungen enthält, behandelt.

2. Verfahren nach Anspruch 1, dadurch gekennzeichnet, dass man eine Verbindungen der Formel I, in welcher $R^1$, $R^2$ und $R^3$ unabhängig voneinander Wasserstoff, Halogen, Nitro, Cyan, $C_1$-$C_3$-Alkyl oder $C_1$-$C_3$-Alkoxy,
$R^4$, $R^3$ und $R^6$ unabhängig voneinander Wasserstoff, Halogen oder $C_1$-$C_3$-Alkyl,
A eine der Gruppen -$CH_2$-, -$CH_2$-$CH_2$- oder -$CH(CH_3)$- und
Z Cyan, eine der Gruppen -$C(NH_2)$=N-OH oder -$C(NH_2)$=N-O-CO-E einen gegebenenfalls substituierten Oxazolin-2-yl-Rest, -$COOR^{12}$, -$COSR^{13}$ oder -$CONR^{14}R^{15}$ bedeuten, worin
E für -$R^7$, -$OR^8$, -$SR^9$ oder -$NR^{10}$ $R^{11}$ steht, worin
$R^7$ $C_1$-$C_7$-Alkyl, welches unsubstituiert oder durch Halogen oder $C_1$-$C_4$-Alkoxy substituiert ist, $C_3$-$C_6$-Cycloalkyl, $C_2$-$C_4$-Alkenyl, Phenyl, welches unsubstituiert oder durch Halogen, Nitro oder $C_1$-$C_3$-Alkyl substituiert ist, Benzyl, welches unsubstituiert oder durch Halogen, Nitro oder $C_1$-$C_3$-Alkyl substituiert ist, oder einen 5-bis 6-gliedrigen heterocyclischen Ring, welcher ein oder zwei Heteroatome aus der Gruppe N, O und S enthält und unsubstituiert oder durch Halogen substituiert ist,
$R^8$, $R^9$ und $R^{10}$ unabhängig voneinander $C_1$-$C_8$-Alkyl, welches unsubstituiert oder durch Halogen substituiert ist, $C_2$-$C_4$-Alkenyl, $C_3$-$C_6$-Alkinyl, Phenyl, welches unsubstituiert oder durch Halogen, $C_1$-$C_3$-Alkyl, $C_1$-$C_3$-Alkoxy, Trifluormethyl oder Nitro substituiert ist, oder Benzyl, welches unsubstituiert oder durch Halogen oder Nitro substituiert ist,
$R^{11}$ Wasserstoff, $C_1$-$C_8$-Alkyl oder $C_1$-$C_3$-Alkoxy oder
$R^{10}$ und $R^{11}$ gemeinsam mit dem Stickstoffatom, an das sie gebunden sind, einen 5- bis 6-gliedrigen Heterocyclus, welcher noch ein weiteres Heteroatom aus der Gruppe N, O und S enthalten kann, $R^{12}$, $R^{13}$ und $R^{14}$ Wasserstoff oder einen gegebenenfalls substituierten Alkyl-, Alkenyl-, Alkinyl-, Cycloalkyl-, Phenyl- oder Naphthylrest oder einen gegebenenfalls substituierten heterocyclischen Rest oder $R^{12}$ und

$R^{13}$ auch ein Kation oder $R^{14}$ auch einen Alkoxyrest und $R^{15}$ Wasserstoff, Amino, mono- oder disubstituiertes Amino oder einen gegebenenfalls substituierten Alkyl-, Alkenyl-, Cycloalkyl- oder Phenylrest oder $R^{14}$ und $R^{15}$ gemeinsam mit dem Stickstoffatom, an das sie gebunden sind, einen gegebenenfalls substituierten heterocyclischen Rest bedeuten, oder A und Z zusammen einen gegebenenfalls substituierten Tetrahydrofuran-2-on-Ring bilden, unter Einschluss ihrer Säureadditionssalze und Metallkomplexe, oder ein Mittel, welches eine dieser Verbindungen enthält, verwendet.

3. Verfahren nach Anspruch 1, dadurch gekennzeichnet, dass man eine Verbindung der Formel I, in welcher $R^1$, $R^2$, $R^4$, $R^5$ und $R^6$ Wasserstoff bedeuten, $R^3$ für Wasserstoff oder Chlor und der Rest -A-Z für eine Gruppe $-CH_2-COOR^{16}$ oder $-CH(CH_3)-COOR^{16}$ steht, worin $R^{16}$ $C_1$-$C_{12}$-Alkyl, $C_3$-$C_6$-Alkenyl, Phenyl-$C_1$-$C_4$-alkyl oder Phenoxy-$C_1$-$C_4$-alkyl steht.

4. Verfahren nach Anspruch 1, dadurch gekennzeichnet, dass man
2-Chinolin-8-yloxy-essigsäureisopropylester,
2-(5-Chlorchinolin-8-yloxy)-essigsäure-n-dodecylester,
2-(5-Chlorchinolin-8-yloxy)-essigsäure-n-butylester,
2-(5-Chlorchinolin-8-yloxy)-essigsäure-n-oktylester,
2-Chinolin-8-yloxy-essigsäure-s-butylester,
2-(5-Chlorchinolin-8-yloxy)-thioessigsäure-n-oktylester,
2-(5-Chlorchinolin-8-yloxy)-essigsäure-(2-butenyl)-ester,
2-(5-chlorchinolin-8-yloxy)-essigsäuremethallylester,
2-(5-Chlorchinolin-8-yloxy)-essigsäure-(2-isopropyloxyäthyl)-ester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäurecyclohexylester,
2-(5-Chlorchinolin-8-yloxy)-essigsäure-s-butylester,
2-(5-Chlorchinolin-8-yloxy)-essigsäure-(2-methylpentyl)-ester,
2-(5-Chlorchinolin-8-yloxy)-thioessigsäure-n-butylester,
2-(5-Chlorchinolin-8-yloxy)-essigsäure-(3,6-dioxadecyl)-ester,
2-(5-Chlorchinolin-8-yloxy)-essigsäure-(3-methoxybutyl)-ester,
2-(5-Chlorchinolin-8-yloxy)-essigsäure-(1-äthylbutyl)-ester,
2-(5-Chlorchinolin-8-yloxy)-essigsäure-(2-äthylbutyl)-ester,
2-(5-Chlorchinolin-8-yloxy)-essigsäure-(1-methylisopentyl)-ester,
2-(5-Chlorchinolin-8-yloxy)-essigsäure-n-undecylester,
2-(5-Chlorchinolin-8-yloxy)-essigsäure-(2-methylbutyl)-ester,
2-(5-Chlorchinolin-8-yloxy)-thioessigsäure-s-butylester,
2-(5-Chlorchinolin-8-yloxy)-essigsäure-(3,6-dioxaheptyl)-ester,
2-(5-Chlorchinolin-8-yloxy)-essigsäure-n-heptylester,
2-(5-Chlorchinolin-8-yloxy)-thioessigsäure-n-dodecylester,
2-(5-Chlorchinolin-8-yloxy)-thioessigsäure-n-decylester,
2-(5-Chlorchinolin-8-yloxy)-essigsäure-(1-propylpropargyl)-ester,
2-(5-Chlorchinolin-8-yloxy)-essigsäure-(1-methylisobutyl)-ester,
2-(5-Chlorchinolin-8-yloxy)-thioessigsäure-tert.butylester,
2-(5-Chlorchinolin-8-yloxy)-essigsäure-neopentylester,
2-(5-Chlorchinolin-8-yloxy)-thioessigsäure-n-propylester,
2-(5-Chlorchinolin-8-yloxy)-essigsäure-(1-methylhexyl)-ester,
2-(5-Chlorchinolin-8-yloxy)-thioessigsäureäthylester,
2-(5-Chlorchinolin-8-yloxy)-essigsäure-(2-äthylhexyl)-ester,
2-(5-Chlorchinolin-8-yloxy)-thioessigsäure-i-butylester,
2-Chinolin-8-yloxy-thioessigsäure-n-decylester,
2-(5-Chlorchinolin-8-yloxy)-essigsäure-i-pentylester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-thioessigsäure-n-hexylester,
2-(5-Chlorchinolin-8-yloxy)-essigsäure-n-hexylester,
2-(5-Chlorchinolin-8-yloxy)-thioessigsäure-i-propylester,
2-(5-Chlorchinolin-8-yloxy)-essigsäure-(1-pentylallyl)-ester,
2-(5-Chlorchinolin-8-yloxy)-essigsäure-(1-methylpentyl)-ester,

2-(5-Chlorchinolin-8-yloxy)-essigsäure-(1,1-dimethylpropargyl)-ester,
2-(5-Chlorchinolin-8-yloxy)-essigsäure-(1-äthyl-1-methylpropargyl)-ester,
2-(5-Chlorchinolin-8-yloxy)-essigsäure-n-butyloxycarbonylmethyl-ester,
2-(5-Chlorchinolin-8-yloxy)-essigsäure-(1-n-butyloxycarbonyläthyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-[1-methyl-2-(2-methylphenoxy)-äthyl]-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-[1-methyl-2-(2-isopropylphenoxy)-äthyl]-ester,
2-(5-Chlorchinolin-8-yloxy)-essigsäure-(1-phenylpropyl)-ester,
2-(5-Chlorchinolin-8-yloxy)-essigsäure-[1-methyl-2-(2-äthylphenoxy)-äthyl]-ester,
2-(5-Chlorchinolin-8-yloxy)-essigsäure-[1-methyl-2-(3-äthylphenoxy)-äthyl]-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenylpropyl)-ester,
2-(5-Chlorchinolin-8-yloxy)-essigsäure-[1-methyl-2-(3-methylphenoxy)-äthyl]-ester,
2-(5-Chlorchinolin-8-yloxy)-essigsäure-[1-methyl-2-(4-isopropylphenoxy)-äthyl]-ester oder
2-(5-Chlorchinolin-8-yloxy)-essigsäure-[1-methyl-2-(4-methylphenoxy)-äthyl]-ester
oder ein Mittel, welches eine dieser Verbindungen enthält, verwendet.

5. Verfahren nach Anspruch 1 dadurch gekennzeichnet, dass man
2-(5-Chlorchinolin-8-yloxy)-essigsäuremethallylester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäure-(1-äthylbutyl)-ester,
2-(5-Chlorchinolin-8-yloxy)-essigsäure-(1-methylisopentyl)-ester,
2-(5-Chlorchinolin-8-yloxy)-essigsäure-(1-methylhexyl)-ester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-essigsäure-(1-pentylallyl)-ester,
2-(5-Chlorchinolin-8-yloxy)-essigsäure-(1-methylpentyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-(1-phenylpropyl)-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenylpropyl)-ester oder
2-(5-Chlorchinolin-8-yloxy)-essigsäure-[1-methyl-2-(4-methylphenoxy)äthyl]-ester oder
ein Mittel, welches eine dieser Verbindungen enthält, verwendet.

6. Verfahren gemäss Anspruch 1, dadurch gekennzeichnet, dass man
2-(5-Chlorchinolin-8-yloxy)-essigsäuremethallylester,
2-(5-Chlorchinolin-8-yloxy)-essigsäure-(2-phenoxyäthyl)-ester,
2-(5-Chlorchinolin-8-yloxy)-essigsäure-(1-methylisopentyl)-ester oder
2-(5-Chlorchinolin-8-yloxy)-essigsäure-(1-methylhexyl)-ester oder ein Mittel, welches eine dieser Verbindungen enthält, verwendet.

7. Verfahren gemäss Anspruch 1 dadurch gekennzeichnet, dass man 2-(5-Chlorchinolin-8-yloxy)-essigsäure-(1-methylisopentyl)-ester oder ein Mittel, welches diese Verbindung enthält, verwendet.

8. Verfahren nach Anspruch 1, dadurch gekennzeichnet, dass man 2-(5-Chlorchinolin-8-yloxy)-essigsäuremethallylester oder ein Mittel, welches diese Verbindung enthält, verwendet.

9. Verfahren gemäss Anspruch 1, dadurch gekennzeichnet, dass von 2-(5-Chlorchinolin-8-yloxy)-essigsäure-(2-phenoxyäthyl)-ester oder ein Mittel, welches diese Verbindung enthält, verwendet.

**10.** Verfahren gemäss Anspruch 1, dadurch gekennzeichnet, dass man 2-(5-Chlorchinolin-8-yloxy)-essigsäure-(1-methylhexyl)-ester oder ein Mittel, welches diese Verbindung enthält, verwendet.

**11.** Verfahren nach Anspruch 1 zum Schützen von Kulturpflanzen gegen schädigende Wirkungen von Herbiziden der Formel II, worin Y für die Gruppen -O-$R^{18}$ , -S-$R^{18}$ , oder -O-N = $CR^{19}$ $R^{20}$ steht, wobei $R^{18}$ Wasserstoff, $C_1$-$C_4$-Alkyl, $C_3$-$C_4$-Alkenyl, $C_3$-$C_4$-Alkinyl oder durch $C_1$-$C_4$-Alkoxyarbonyl oder Di-$C_1$-$C_4$-alkylamino substituiertes $C_1$-$C_4$-Alkyl und
$R^{19}$ und $R^{20}$ unabhängig voneinander $C_1$-$C_4$-Alkyl oder
$R^{19}$ und $R^{20}$ zusammen eine $C_4$-$C_7$-Alkylenkette bedeuten.

**12.** Verfahren gemäss Anspruch 11, dadurch gekennzeichnet, dass Y für Methoxy, Aethoxy, Propyloxy, Isopropyloxy, Butyloxy, Dimethylaminoäthoxy, Propargyloxy, 1-Cyano-1-methyläthoxy, Methoxycarbonylmethylthio, 1-Aethoxycarbonyläthoxy, Butyloxycarbonyl, -O-N = $C(CH_3)_2$,-O-N = $C(CH_3)C_2H_5$ oder -O-N = $C(CH_2)_5$ steht.

**13.** Verfahren gemäss Anspuch 1 zum Schützen von Kulturpflanzen gegen schädigende Wirkungen von 2-[4-(5-Chlor-3-fluorpyridin-2-yloxy)-phenoxy]-propionsäuremethylester.

**14.** Verfahren gemäss Anspruch 1 zum Schützen von Kulturpflanzen gegen schädigende Wirkungen von 2-[4-(5-Chlor-3-fluorpyridin-2-yloxy)-phenoxy]-propionsäurepropargylester.

**15.** Verfahren nach Anspruch 1 zum Schützen von Kulturpflanzen gegen schädigende Wirkungen von 2-[4-(5-Chlor-3-fluorpyridin-2-yloxy)-phenoxy]-thiopropionsäure-S-methoxycarbonylmethylester.

**16.** Verfahren nach Anspruch 1 zum Schützen von Kulturpflanzen gegen schädigende Wirkungen von 2-[4-(5-Chlor-3-fluorpyridin-2-yloxy)-phenoxy]-propionsäure-(1-äthoxycarbonyläthyl)-ester.

**17.** Verfahren nach Anspruch 1 zum Schützen von Kulturpflanzen gegen schädigende Wirkungen von 2-[4-(5-Chlor-3-fluorpyridin-2-yloxy)-phenoxy]-propionsäuremethylester, 2-[4-(5-Chlor-3-fluorpyridin-2-yloxy)-phenoxy]-propionsäurepropargylester, 2-[4-(5-Chlor-3-fluorpyridin-2-yloxy)-phenoxy]-thiopropionsäure-S -methoxycarbonylmethylester oder 2-[4-(-5Chlor3-fluorpyridin-2-yloxy)-phenoxy]-propionsäure-(1-äthoxycarbonyläthyl)-ester, dadurch gekennzeichnet, dass man die Kulturpflanzen, Teile dieser Pflanzen oder für den Anbau der Kulturpflanzen bestimmte Böden mit
2-Chinolin-8-yloxy-essigsäureisopropylester,
2-(5-Chlorchinolin-8-yloxy)-essigsäure-n-dodecylester,
2-(5-Chlorchinolin-8-yloxy)-essigsäure-n-butylester,
2-(5-Chlorchinolin-8-yloxy)-essigsäure-n-oktylester,
2-Chinolin-8-yloxy-essigsäure-s-butylester,
2-(5-Chlorchinolin-8-yloxy)-thioessigsäure-n-oktylester`
2-(5-Chlorchinolin-8-yloxy)-essigsäure-(2-butenyl)-ester,
2-(5-Chlorchinolin-8-yloxy)-essigsäuremethallylester,
2-(5-Chlorchinolin-8-yloxy)-essigsäure-(2-isopropyloxyäthyl)-ester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäurecyclohexylester,
2-(5-Chlorchinolin-8-yloxy)-essigsäure-s-butylester,
2-(5-Chlorchinolin-8-yloxy)-essigsäure-(2-methylpentyl)-ester,
2-(5-Chlorchinolin-8-yloxy)-thioessigsäure-n-butylester,
2-(5-Chlorchinolin-8-yloxy)-essigsäure-(3,6-dioxadecyl)-ester,
2-(5-Chlorchinolin-8-yloxy)-essigsäure-(3-methoxybutyl)-ester,
2-(5-Chlorchinolin-8-yloxy)-essigsäure-(1-äthylbutyl)-ester,
2-(5-Chlorchinolin-8-yloxy)-essigsäure-(2-äthylbutyl)-ester,
2-(5-Chlorchinolin-8-yloxy)-essigsäure-(1-methylisopentyl)-ester,
2-(5-Chlorchinolin-8-yloxy)-essigsäure-n-undecylester,
2-(5-Chlorchinolin-8-yloxy)-essigsäure-(2-methylbutyl)-ester,
2-(5-Chlorchinolin-8-yloxy)-thioessigsäure-s-butylester,
2-(5-Chlorchinolin-8-yloxy)-essigsäure-(3,6-dioxaheptyl)-ester,
2-(5-Chlorchinolin-8-yloxy)-essigsäure-n-heptylester,

2-(5-Chlorchinolin-8-yloxy)-thioessigsäure-n-dodecylester,
2-(5-Chlorchinolin-8-yloxy)-thioessigsäure-n-decylester,
2-(5-Chlorchinolin-8-yloxy)-essigsäure-(1-propylpropargyl)-ester,
2-(5-Chlorchinolin-8-yloxy)-essigsäure-(1-methylisobutyl)-ester,
2-(5-Chlorchinolin-8-yloxy)-thioessigsäure-tert.butylester,
2-(5-Chlorchinolin-8-yloxy)-essigsäure-neopentylester,
2-(5-Chlorchinolin-8-yloxy)-thioessigsäure-n-propylester,
2-(5-Chlorchinolin-8-yloxy)-essigsäure-(1-methylhexyl)-ester,
2-(5-Chlorchinolin-8-yloxy)-thioessigsäureäthylester,
2-(5-Chlorchinolin-8-yloxy)-essigsäure-(2-äthylhexyl)-ester,
2-(5-Chlorchinolin-8-yloxy)-thioessigsäure-i-butylester,
2-Chinolin-8-yloxy-thioessigsäure-n-decylester,
2-(5-Chlorchinolin-8-yloxy)-essigsäure-i-pentylester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-thioessigsäure-n-hexylester,
2-(5-Chlorchinolin-8-yloxy)-essigsäure-n-hexylester,
2-(5-Chlorchinolin-8-yloxy)-thioessigsäure-i-propylester,
2-(5-Chlorchinolin-8-yloxy)-essigsäure-(1-pentylallyl)-ester,
2-(5-Chlorchinolin-8-yloxy)-essigsäure-(1-methylpentyl)-ester,
2-(5-Chlorchinolin-8-yloxy)-essigsäure-(1,1-dimethylpropargyl)-ester,
2-(5-Chlorchinolin-8-yloxy)-essigsäure-(1-äthyl-1-methylpropargyl)-ester,
2-(5-Chlorchinolin-8-yloxy)-essigsäure-n-butyloxycarbonylmethylester,
2-(5-Chlorchinolin-8-yloxy)-essigsäure-(1-n-butyloxcarbonyläthyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-[1-methyl-2-(2-methylphenoxy)-äthyl]-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-[1-methyl-2-(2-isopropylphenoxy)-äthyl]-ester,
2-(5-Chlorchinolin-8-yloxy)-essigsäure-(1-phenylpropyl)-ester,
2-(5-Chlorchinolin-8-yloxy)-essigsäure-[1-methyl-2-(2-äthylphenoxy)-äthyl]-ester,
2-(5-Chlorchinolin-8-yloxy)-essigsäure-[1-methyl-2-(3-äthylphenoxy)-äthyl]-ester,
2-(5-Chlorchinolin-8-yloxy)-essigsäure-(1-methyl-2-phenoxäthyl)-ester,
2-(5-Chlorchinolin-8-yloxy)-essigsäure-(1-methyl-3-phenylpropyl)-ester,
2-(5-Chlorchinolin-8-yloxy)-essigsäure-[1-methyl-2-(3-methylphenoxy)-äthyl]-ester,
2-(5-Chlorchinolin-8-yloxy)-essigsäure-[1-methyl-2-(4-isopropylphenoxy)-äthyl]-ester oder
2-(5-Chlorchinolin-8-yloxy)-essigsäure-[1-methyl-2-(4-methylphenoxy)-äthyl]-ester
oder einem Mittel, welches eine dieser Verbindungen enthält, behandelt.

**18.** Verfahren gemäss Anspruch 17, dadurch gekennzeichnet, dass man die Kulturpflanzen, Teile dieser Pflanzen oder für den Anbau der Kulturpflanzen bestimmte Böden mit
2-(5-Chlcrchinolin-8-yloxy)-essigsäuremethallylester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäure-(1-äthylbutyl)-ester,
2-(5-Chlorchinolin-8-yloxy)-essigsäure-(1-methylisopentyl)-ester,
2-(5-Chlorchinolin-8-yloxy)-essigsäure-(1-methylhexyl)-ester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-essigsäure-(1-pentylallyl)-ester,
2-(5-Chlorchinolin-8-yloxy)-essigsäure-(1-methylpentyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,

2-(5-Chlorchinolin-8-yloxy)-essigsäure-(1-phenylpropyl)-ester,
2-(5-Chlorchinolin-8-yloxy)-essigsäure-(1-methyl-2-phenoxyäthyl)-ester
2-(5-Chlorchinolin-8-yloxy)-essigsäure-(1-methyl-3-phenylpropyl)-ester oder
2-(5-Chlorchinolin-8-yloxy)-essigsäure-[1-methyl-2-(4-methylphenoxy)-äthyl]-ester oder
einem Mittel, welches eine dieser Verbindungen enthält, behandelt.

**19.** Verfahren nach Anspruch 17, dadurch gekennzeichnet, dass man die Kulturpflanzen, Teile dieser Pflanzen oder für den Anbau der Kulturpflanzen bestimmte Böden mit 2-(5-Chlorchinolin-8-yloxy)-essigsäure-(1-methylisopentyl)-ester, 2-(5-Chlorchinolin-8-yloxy)-essigsäure-(2-phenoxyäthyl)-ester, 2-(5-Chlorchinolin-8-yloxy)-essigsäure-(1-methylhexyl)-ester oder 2-(5-Chlorchinolin-8-yloxy)-essigsäure-methallylester oder einem Mittel, welches eine dieser Verbindungen enthält, behandelt.

**20.** Verfahren nach Anspruch 1 zum Schützen von Getreide.

**21.** Verfahren nach Anspruch 20 zum Schützen von Weizen, Gerste, Roggen und Reis.

**22.** Verfahren nach Anspruch 1, dadurch gekennzeichnet, dass man Kulturpflanzenbestände oder Anbauflächen für Kulturpflanzen mit 0,01 bis 10 kg/ha einer Verbindung der Formel I gemäss Anspruch 1 behandelt.

**23.** Verfahren nach Anspruch 22, dadurch gekennzeichnet, dass man Kulturpflanzenbestände oder Anbauflächen für Kulturpflanzen mit 0,05 bis 0.5 kg/ha einer Verbindung der Formel I gemäss Anspruch 1 behandelt.

**24.** Verfahren nach Anspruch 1, dadurch gekennzeichnet, dass man Samen der Kulturpflanzen mit einer Verbindung der Formel I gemäss Anspruch 1 behandelt.

**25.** Verfahren nach Anspruch 24, dadurch gekennzeichnet, dass man Samen der Kulturpflanze mit 0,01 bis 10 g/kg Samen einer Verbindung der Formel I gemäss Anspruch 1 behandelt.

**26.** Verfahren nach Anspruch 25, dadurch gekennzeichnet, dass man Samen der Kulturpflanzen mit 0,05 bis 2 g/kg Samen einer Verbindung der Formel I gemäss Anspruch 1 behandelt.

**27.** Verfahren nach Anspruch 24 zum Schützen von Kulturpflanzen gegen schädigende Wirkungen von 2-[4-(5-Chlor-3-fluorpyridin-2-yloxy)-phenoxy]-propionsäuremethylester, 2-[4-(5-Chlor-3-fluorpyridin-2-yloxy)-phenoxy]-propionsäurepropargylester, 2-[4-(5-Chlor-3-fluorpyridin-2-yloxy)-phenoxy]-thiopropionsäure-S-methoxycarbonylmethylester oder 2-[4-(5-Chlor-3-fluorpyridin-2-yloxy)-phenoxy]-propionsäure-(1-äthoxycarbonyläthyl)-ester, dadurch gekennzeichnet, dass man die Samen der Kulturpflanzen, mit
2-Chinolin-8-yloxy-essigsäureisopropylester,
2-(5-Chlorchinolin-8-yloxy)-essigsäure-n-dodecylester,
2-(5-Chlorchinolin-8-yloxy)-essigsäure-n-butylester,
2-(5-Chlorchinolin-8-yloxy)-essigsäure-n-oktylester,
2-Chinolin-8-yloxy-essigsäure-s-butylester,
2-(5-Chlorchinolin-8-yloxy)-thioessigsäure-n-oktylester,
2-(5-Chlorchinolin-8-yloxy)-essigsäure-(2-butenyl)-ester,
2-(5-Chlorchinolin-8-yloxy)-essigsäuremethallylester,
2-(5-Chlorchinolin-8-yloxy)-essigsäure-(2-isopropyloxyäthyl)-ester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäurecyclohexylester,
2-(5-Chlorchinolin-8-yloxy)-essigsäure-s-butylester,
2-(5-Chlorchinolin-8-yloxy)-essigsäure-(2-methylpentyl)-ester,
2-(5-Chlorchinolin-8-yloxy)-thioessigsäure-n-butylester,
2-(5-Chlorchinolin-8-yloxy)-essigsäure-(3,6-dioxadecyl)-ester,
2-(5-Chlorchinolin-8-yloxy)-essigsäure-(3-methoxybutyl)-ester,
2-(5-Chlorchinolin-8-yloxy)-essigsäure-(1-äthylbutyl)-ester,
2-(5-Chlorchinolin-8-yloxy)-essigsäure-(2-äthylbutyl)-ester,

2-(5-Chlorchinolin-8-yloxy)-essigsäure-(1-methylisopentyl)-ester,
2-(5-Chlorchinolin-8-yloxy)-essigsäure-n-undecylester,
2-(5-Chlorchinolin-8-yloxy)-essigsäure-(2-methylbutyl)-ester,
2-(5-Chlorchinolin-8-yloxy)-thioessigsäure-s-butylester,
2-(5-Chlorchinolin-8-yloxy)-essigsäure-(3,6-dioxaheptyl)-ester,
2-(5-Chlorchinolin-8-yloxy)-essigsäure-n-heptylester,
2-(5-Chlorchinolin-8-yloxy)-thioessigsäure-n-dodecylester,
2-(5-Chlorchinolin-8-yloxy)-thioessigsäure-n-decylester,
2-(5-Chlorchinolin-8-yloxy)-essigsäure-(1-propylpropargyl)-ester,
2-(5-Chlorchinolin-8-yloxy)-essigsäure-(1-methylisobutyl)-ester,
2-(5-Chlorchinolin-8-yloxy)-thioessigsäure-tert.butylester,
2-(5-Chlorchinolin-8-yloxy)-essigsäure-neopentylester,
2-(5-Chlorchinolin-8-yloxy)-thioessigsäure-n-propylester,
2-(5-Chlorchinolin-8-yloxy)-essigsäure-(1-methylhexyl)-ester,
2-(5-Chlorchinolin-8-yloxy)-thioessigsäureäthylester,
2-(5-Chlorchinolin-8-yloxy)-essigsäure-(2-äthylhexyl)-ester,
2-(5-Chlorchinolin-8-yloxy)-thioessigsäure-i-butylester,
2-Chinolin-8-yloxy-thioessigsäure-n-decylester,
2-(5-Chlorchinolin-8-yloxy)-essigsäure-i-pentylester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-thioessigsäure-n-hexylester,
2-(5-Chlorchinolin-8-yloxy)-essigsäure-n-hexylester,
2-(5-Chlorchinolin-8-yloxy)-thioessigsäure-i-propylester,
2-(5-Chlorchinolin-8-yloxy)-essigsäure-(1-pentylallyl)-ester,
2-(5-Chlorchinolin-8-yloxy)-essigsäure-(1-methylpentyl)-ester,
2-(5-Chlorchinolin-8-yloxy)-essigsäure-(1,1-dimethylpropargyl)-ester,
2-(5-Chlorchinolin-8-yloxy)-essigsäure-(1-äthyl-1-methylpropargyl)-ester,
2-(5-Chlorchinolin-8-yloxy)-essigsäure-n-butyloxycarbonylmethylester,
2-(5-Chlorchinolin-8-yloxy)-essigsäure-(1-n-butyloxycarbonyläthyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-[1-methyl-2-(2-methylphenoxy)-äthyl]-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-[1-methyl-2-(2-isopropylphenoxy)-äthyl]-ester,
2-(5-Chlorchinolin-8-yloxy)-essigsäure-(1-phenylpropyl)-ester,
2-(5-Chlorchinolin-8-yloxy)-essigsäure-[1-methyl-2-(2-äthylphenoxy)-äthyl]-ester,
2-(5-Chlorchinolin-8-yloxy)-essigsäure-[1-methyl-2-(3-äthylphenoxy)-äthyl]-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenylpropyl)-ester,
2-(5-Chlorchinolin-8-yloxy)-essigsäure-[1-methyl-2-(3-methylphenoxy)-äthyl]-ester,
2-(5-Chlorchinolin-8-yloxy)-essigsäure-[1-methyl-2-(4-isopropylphenoxy)-äthyl]-ester oder
2-(5-Chlorchinolin-8-yloxy)-essigsäure-[1-methyl-2-(4-methylphenoxy)-äthyl]-ester
oder einem Mittel, welches eine dieser Verbindungen enthält, behandelt.

**28.** Verfahren gemäss Anspruch 27, dadurch gekennzeichnet, dass man die Samen der Kulturpflanzen mit
2-(5-Chlorchinolin-8-yloxy)-essigsäuremethallylester,
2-(5-Chlorchinolin-8-yloxy)>-essigsäure-(2-phenoxyäthyl)-ester,
2(5-Chlorchinolin-8-yloxy)-essigsäure-(1-methylbutyl)-ester,
2-(5-Chlorchinolin-8-yloxy)-essigsäure-(1-äthylbutyl)-ester,
2-(5-Chlorchinolin-8-yloxy)-essigsäure-(1-methylisopentyl)-ester,
2-(5-Chlorchinolin-8-yloxy)-essigsäure-(1-methylhexyl)-ester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-essigsäure-(1-pentylallyl)-ester oder
2-(5-Chlorchinolin-8-yloxy)-essigsäure-(1-methylpentyl)-ester oder

2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-(1-phenylpropyl)-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenylpropyl)-ester oder
2-(5-Chlorchinolin-8-yloxy)-essigsäure-[1-methyl-2-(4-methylphenoxy)-äthyl]-ester oder
einem Mittel, welches eine dieser Verbindungen enthält, behandelt.

**29.** Verfahren gemäss Anspruch 27, dadurch gekennzeichnet, dass man die Samen der Kulturpflanzen mit 2-(5-Chlorchinolin-8-yloxy)-essigsäure-(1-methylisopentyl)-ester, 2-(5-Chlorchinolin-8-yloxy)-essigsäure-(2-phenoxyäthyl)-ester, 2-(5-Chlorchinolin-8-yloxy)-essigsäure-(1-methylhexyl)-ester oder 2-(5-Chlorchinolin-8-yloxy)-essigsäuremethallylester oder einem Mittel, welches eine dieser Verbindungen enthält, behandelt.

**30.** Verfahren nach Anspruch 27 zum Schützen von Getreide.

**31.** Verwendung von Verbindungen der Formel I gemäss Anspruch 1 zum Schützen von Kulturpflanzen gegen schädigende Wirkungen von Verbindungen der Formel II gemäss Anspruch 1.

**32.** Verfahren zur selektiven Bekämpfung von Unkräutern in Kulturpflanzenbeständen, dadurch gekennzeichnet, dass man die Kulturpflanzenbestände, Teile der Kulturpflanzen oder Anbauflächen der Kulturpflanzen mit einem Antidot der Formel I gemäss Anspruch 1 und einem Herbizid der Formel II gemäss Anspruch 1 behandelt.

**33.** Herbizides Mittel, dadurch gekennzeichnet, dass es neben einem herbiziden Wirkstoff der Formel II gemäss Anspruch 1 ein Antidot der Formel I gemäss Anspruch 1 enthält.

**34.** Mittel gemäss Anspruch 33, dadurch gekennzeichnet, dass es neben einem Herbizid aus der Reihe 2-[4-(5-Chlor-3-fluorpyridin-2-yloxy)-phenoxy]-propionsäuremethylester, 2-[4-(5-Chlor-3-fluorpyridin-2-yloxy)-phenoxy]-propionsäurepropargylester, 2-[4-(5-Chlor-3-fluorpyridin-2-yloxy)-phenoxy]-thiopropionsäure-S-methoxycarbonylmethylester oder 2-[4-(5-Chlor-3-fluorpyridin-2-yloxy)-phenoxy]-propionsäure-(1-äthoxycarbonyläthyl)-ester ein Antidot aus der Reihe
2-Chinolin-8-yloxy-essigsäureisopropylester,
2-(5-Chlorchinolin-8-yloxy)-essigsäure-n-dodecylester,
2-(5-Chlorchinolin-8-yloxy)-essigsäure-n-butylester,
2-(5-Chlorchinolin-8-yloxy)-essigsäure-n-oktylester,
2-Chinolin-8-yloxy-essigsäure-s-butylester,
2-(5-Chlorchinolin-8-yloxy)-thioessigsäure-n-oktylester,
2-(5-Chlorchinolin-8-yloxy)-essigsäure-(2-butenyl)-ester,
2-(5-Chlorchinolin-8-yloxy)-essigsäuremethallylester,
2-(5-Chlorchinolin-8-yloxy)-essigsäure-(2-isopropyloxyäthyl)-ester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäurecyclohexylester,
2-(5-Chlorchinolin-8-yloxy)-essigsäure-s-butylester,
2-(5-Chlorchinolin-8-yloxy)-essigsäure-(2-methylpentyl)-ester,
2-(5-Chlorchinolin-8-yloxy)-thioessigsäure-n-butylester,
2-(5-Chlorchinolin-8-yloxy)-essigsäure-(3,6-dioxadecyl)-ester,
2-(5-Chlorchinolin-8-yloxy)-essigsäure-(3-methoxybutyl)-ester,
2-(5-Chlorchinolin-8-yloxy)-essigsäure-(1-äthylbutyl)-ester,
2-(5-Chlorchinolin-8-yloxy)-essigsäure-(2-äthylbutyl)-ester,
2-(5-Chlorchinolin-8-yloxy)-essigsäure-(1-methylisopentyl)-ester,
2-(5-Chlorchinolin-8-yloxy)-essigsäure-n-undecylester,
2-(5-Chlorchinolin-8-yloxy)-essigsäure-(2-methylbutyl)-ester,
2-(5-Chlorchinolin-8-yloxy)-thioessigsäure-s-butylester,

2-(5-Chlorchinolin-8-yloxy)-essigsäure-(3,6-dioxaheptyl)-ester,
2-(5-Chlorchinolin-8-yloxy)-essigsäure-n-heptylester,
2-(5-Chlorchinolin-8-yloxy)-thioessigsäure-n-dodecylester,
2-(5-Chlorchinolin-8-yloxy)-thioessigsäure-n-decylester,
2-(5-Chlorchinolin-8-yloxy)-essigsäure-(1-propylpropargyl)-ester,
2-(5-Chlorchinolin-8-yloxy)-essigsäure-(1-methylisobutyl)-ester,
2-(5-Chlorchinolin-8-yloxy)-thioessigsäure-tert.butylester,
2-(5-Chlorchinolin-8-yloxy)-essigsäure-neopentylester,
2-(5-Chlorchinolin-8-yloxy)-thioessigsäure-n-propylester,
2-(5-Chlorchinolin-8-yloxy)-essigsäure-(1-methylhexyl)-ester,
2-(5-Chlorchinolin-8-yloxy)-thioessigsäureäthylester,
2-(5-Chlorchinolin-8-yloxy)-essigsäure-(2-äthylhexyl)-ester,
2-(5-Chlorchinolin-8-yloxy)-thioessigsäure-i-butylester,
2-Chinolin-8-yloxy-thioessigsäure-n-decylester,
2-(5-Chlorchinolin-8-yloxy)-essigsäure-i-pentylester,
2-(5-Chlorchinolin-8-yloxy)-essigsäure-(1-äthylpentyl)-ester,
2-(5-Chlorchinolin-8-yloxy)-essigsäure(1-propylbutyl)-ester,
2-(5-Chlorchinolin-8-yloxy)-thioessigsäure-n-hexylester,
2-(5-Chlorchinolin-8-yloxy)-essigsäure-n-hexylester,
2-(5-Chlorchinolin-8-yloxy)-thioessigsäure-i-propylester,
2-(5-Chlorchinolin-8-yloxy)-essigsäure-(1-pentylallyl)-ester,
2-(5-Chlorchinolin-8-yloxy)-essigsäure-(1-methylpentyl)-ester,
2-(5-Chlorchinolin-8-yloxy)-essigsäure-(1,1-dimethylpropargyl)-ester,
2-(5-Chlorchinolin-8-yloxy)-essigsäure-(1-äthyl-1-methylpropargyl)-ester,
2-(5-Chlorchinolin-8-yloxy)-essigsäure-n-butyloxycarbonylmethylester,
2-(5-Chlorchinolin-8-yloxy)-essigsäure-(1-n-butyloxycarbonyläthyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-[1-methyl-2-(2-methylphenoxy)-äthyl]-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-[1-methyl-2-(2-isopropylphenoxy)-äthyl]-ester,
2-(5-Chlorchinolin-8-yloxy)-essigsäure-(1-phenylpropyl)-ester,
2-(5-Chlorchinolin-8-yloxy)-essigsäure-[1-methyl-2-(2-äthylphenoxy)-äthyl]-ester,
2-(5-Chlorchinolin-8-yloxy)-essigsäure-[1-methyl-2-(3-äthylphenoxy)-äthyl]-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenylpropyl)-ester,
2-(5-Chlorchinolin-8-yloxy)-essigsäure-[1-methyl-2-(3-methylphenoxy)-äthyl]-ester,
2-(5-Chlorchinolin-8-yloxy)-essigsäure-[1-methyl-2-(4-isopropylphenoxy)-äthyl]-ester oder
2-(5-Chlorchinolin-8-yloxy)-essigsäure-[1-methyl-2-(4-methylphenoxy)-äthyl]-ester enthält.

**35.** Mittel gemäss Anspruch 34, dadurch gekennzeichnet, dass es ein Antidot aus der Reihe
2-(5-Chlorchinolin-8-yloxy)-essigsäuremethallylester,
2-(5-Chlorchinolin-8-yloxy)-essigsäure-(2-phenoxyäthyl)-ester,
2-(5-Chlorchinolin-8-yloxy)-essigsäure-(1-methylbutyl)-ester,
2-(5-Chlorchinolin-8-yloxy)-essigsäure-(1-äthylbutyl)-ester,
2-(5-Chlorchinolin-8-yloxy)-essigsäure-(1-methylisopentyl)-ester,
2-(5-Chlorchinolin-8-yloxy)-essigsäure-(1-methylhexyl)-ester,
2-(5-Chlorchinolin-8-yloxy)-essigsäure-(1-äthylpentyl)-ester,
2-(5-Chlorchinolin-8-yloxy)-essigsäure-(1-propylbutyl)-ester,
2-(5-Chlorchinolin-8-yloxy)-essigsäure-(1-pentylallyl)-ester,
2-(5-Chlorchinolin-8-yloxy)-essigsäure-(1-methylpentyl)-ester,
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,

2-(5-Chlorchinolin-8-yloxy)-essigsäure-(1-phenylpropyl)-ester,
2-(5-Chlorchinolin-8-yloxy)-essigsäure-(1-methyl-2-phenoxyäthyl)-ester
2-(5-Chlorchinolin-8-yloxy)-essigsäure-(1-methyl-3-phenylpropyl)-ester oder
2-(5-Chlorchinolin-8-yloxy)-essigsäure-[1-methyl-2-(4-methylphenoxy)-äthyl]-ester enthält.

**36.** Mittel gemäss Anspruch 34, dadurch gekennzeichnet, dass es als Antidot
2-(5-Chlorchinolin-8-yloxy)-essigsäuremethallylester,
2-(5-Chlorchinolin-8-yloxy)-essigsäure-(2-phenoxyäthyl)-ester,
2-(5-Chlorchinolin-8-yloxy)-essigsäure-(1-methylisopentyl)-ester oder
2-(5-Chlorchinolin-8-yloxy)-essigsäure-(1-methylhexyl)-ester enthält.

**37.** Eine Verbindung aus der Gruppe
2-(5-Chlorchinolin-8-yloxy)-essigsäure-(R-1-methylisopentyl)-ester,
2-(5-Chlorchinolin-8-yloxy)-essigsäure-(S-1-methylisopentyl)-ester,
2-(5-Chlorchinolin-8-yloxy)-essigsäure(R-1-methylhexyl)-ester,
2-(5-Chlorchinolin-8-yloxy)-essigsäure-(S-1-methylhexyl-ester.
2-(5-Chlorchinolin-8-yloxy)-essigsäure-(1-methylisohexyl)-ester,
2-(5-Chlorchinolin-8-yloxy)-essigsäure-(1-phenylisobutyl)-ester,
2-(5-Chlorchinolin-8-yloxy)-essigsäure-(1-phenyläthyl)-ester,
2-(5-Chlorchinolin-8-yloxy)-essigsäure-[1-methyl-2-(4-äthylphenoxy)-äthyl]-ester,
2-(5-Chlorchinolin-8-yloxy)-essigsäure-(1-methyl-2-phenyläthyl)-ester,
2-(5-Chlorchinolin-8-yloxy)-essigsäure-(1-phenylpropyl)-ester,
2-(5-Chlorchinolin-8-yloxy)-essigsäure-(1-methyl-2-phenoxyäthyl)-ester,
2-(5-Chlorchinolin-8-yloxy)-essigsäure-(1-methyl-3-phenylpropyl)-ester und
2-(5-Chlorchinolin-8-yloxy)-essigsäure-[1-methyl-2-(4-methylphenoxy)-äthyl]-ester.

**38.** Verfahren zur Herstellung der Mittel gemäss Anspruch 33, dadurch gekennzeichnet, dass man die Wirkstoffe der Formel I und II intensiv miteinander und gegebenenfalls einem Trägermaterial und/oder oberflächenaktivem Mittel vermischt.

**39.** Verfahren zur Herstellung der Verbindungen gemäss Anspruch 37, dadurch gekennzeichnet, dass man 5-Chlor-8-hydroxychinolin in Gegenwart eines säurebindenden Mittels mit einer Verbindung aus der Reihe
Bromessigsäure-(R-1-methylisopentyl)-ester,
Bromessigsäure-(S-1-methylisopentyl)-ester,
Bromessigsäure-(R-1-methylhexyl)-ester,
Bromessigsäure-(S-1-methylhexyl)-ester,
Bromessigsäure-(1-methylisohexyl)-ester,
Bromessigsäure-(1-phenylisobutyl)-ester,
Bromessigsäure-(1-phenyläthyl)-ester,
Bromessigsäure-[1-methyl-2-(4-äthylphenoxy)-äthyl]-ester,
Bromessigsäure-(1-methyl-2-phenyläthyl)-ester,
Bromessigsäure-(1-phenylpropyl)-ester,
Bromessigsäure-(1-methyl-2-phenoxyäthyl)-ester,
Bromessigsäure-(1-methyl-3-phenylpropyl)-ester und
Bromessigsäure-[1-methyl-2-(4-methylphenoxy)-äthyl]-ester umsetzt.

## Claims

**1.** A process for the protection of cultivated plants against the harmful effects of herbicidally active 2-[4-(5-chloro-3-fluoropyridin-2-yloxy)-phenoxy]-propionic acid derivatives of the formula II

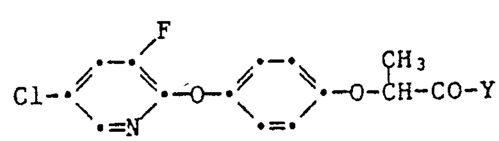

(II)

in which Y is a group $-NR^{16}R^{17}$, $-O-R^{18}$, $-S-R^{18}$ or $-O-N=CR^{19}R^{20}$, $R^{16}$ and $R^{17}$ independently of one another are each hydrogen, $C_1$-$C_8$alkoxy, $C_1$-$C_8$alkyl, phenyl or benzyl, $R^{16}$ and $R^{17}$ together with the nitrogen atom carrying them form a 5- or 6-membered saturated nitrogen heterocycle which can be interrupted by an oxygen or sulfur atom, $R^{18}$ is hydrogen or the equivalent of an alkali metal, alkaline-earth metal, copper or iron ion; or is a quaternary $C_1$-$C_4$alkylammonium or $C_1$-$C_4$hydroxyalkyl-ammonium radical; a $C_1$-$C_9$alkyl radical which is unsubstituted or mono- or polysubstituted by amino, halogen, hydroxyl, cyano, nitro, phenyl, $C_1$-$C_4$-alkoxy, polyethoxy having 2 to 6 ethylene oxide units, $-COOR^{21}$, $-COSR^{21}$, $-CONH_2$-, $-CON(C_1$-$C_4$alkoxy)-$C_1$-$C_4$alkyl, -CO-N-di-$C_1$-$C_4$alkyl, -CONH-$C_1$-$C_4$alkyl, -N($C_1$-$C_4$alkoxy)-$C_1$-$C_4$alkyl or di-$C_1$-$C_4$alkylamino; or is a $C_3$-$C_9$alkenyl radical which is unsubstituted or substituted by halogen or $C_1$-$C_4$alkoxy; or is a $C_3$-$C_9$alkynyl radical which is unsubstituted or substituted by halogen or $C_1$-$C_4$alkoxy; or is $C_3$-$C_9$cycloalkyl; or phenyl which is unsubstituted or substituted by cyano, $C_1$-$C_4$alkyl, $C_1$-$C_4$alkoxy, acetyl, $-COOR^{21}$, $-COSR^{21}$, $-CONH_2$, -CON($C_1$-$C_4$-alkoxy)-$C_1$-$C_4$alkyl, -CO-N-di-$C_1$-$C_4$alkyl or -CONH-$C_1$-$C_4$alkyl, $R^{19}$ and $R^{20}$ independently of one another are each $C_1$-$C_4$alkyl, or together form a 3- to 6-membered alkylene chain, and $R^{21}$ is hydrogen, $C_1$-$C_6$alkyl, $C_1$-$C_6$haloalkyl, $C_2$-$C_6$alkoxyalkyl, $C_3$-$C_6$alkenyl, $C_3$-$C_6$haloalkenyl, $C_3$-$C_6$alkynyl or $C_3$-$C_6$haloalkynyl, which comprises treating the cultivated plants or parts of these plants, or soils intended for the growing of the cultivated plants, with a compound of the formula I

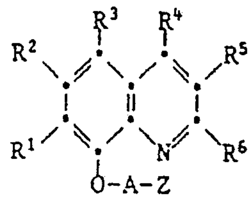

(I)

in which $R^1$, $R^2$ and $R^3$ independently of one another are each hydrogen, halogen, nitro, cyano, $C_1$-$C_3$alkyl or $C_1$-$C_3$alkoxy, $R^4$, $R^5$ and $R^6$ independently of one another are each hydrogen, halogen, or $C_1$-$C_3$alkyl, A is one of the groups $-CH_2$-, $-CH_2$-$CH_2$- or $-CH(CH_3)$-, and Z is cyano or amidoxime, which can be acylated on the oxygen atom, or is a carboxyl group or a salt thereof, a mercaptocarbonyl group or a salt thereof, a carboxylic acid ester group, a carboxylic acid thiol ester group, an unsubstituted or substituted carboxylic acid amide group, a cyclised, unsubstituted or substituted derivative of a carboxylic acid amide group or a carboxylic acid hydrazide group, or A and Z together form an unsubstituted or substituted tetrahydrofuran-2-one ring; with the inclusion of the acid addition salts and metal complexes thereof; or with a composition comprising one of these compounds.

2. A process according to claim 1, which comprises using a compound of the formula I in which $R^1$, $R^2$ and $R^3$ independently of one another are each hydrogen, halogen, nitro, cyano, $C_1$-$C_3$alkyl or $C_1$-$C_3$alkoxy, $R^4$, $R^5$ and $R^6$ independently of one another are each hydrogen, halogen or $C_1$-$C_3$alkyl, A is one of the groups $-CH_2$-, $-CH_2$-$CH_2$- or $-CH(CH_3)$-, and Z is cyano, one of the groups $-C(NH_2)$-N-OH or $-C(NH_2)=N$-O-CO-E, an unsubstituted or substituted oxazolin-2-yl radical, $-COOR^{12}$, $-COSR^{13}$ or $-CONR^{14}R^{15}$, in which E is $R^7$ , $-OR^8$, $-SR^9$ or $-NR^{10}-R^{11}$, in which $R^7$ is $C_1$-$C_7$alkyl which is unsubstituted or substituted by halogen or $C_{1-4}$alkoxy, or is $C_3$-$C_6$cycloalkyl, $C_2$-$C_4$alkenyl, phenyl which is unsubstituted or substituted by halogen, nitro, or $C_1$-$C_3$alkyl, benzyl which is unsubstituted or substituted by halogen, nitro or $C_1$-$C_3$alkyl, or is a 5- or 6-membered heterocyclic ring which contains one or two hetero atoms from the group N, O and S, and which is unsubstituted or substituted by halogen, $R^8$, $R^8$ and $R^{10}$ independently of one another are each $C_1$-$C_8$alkyl which is unsubstituted or substituted by halogen, or are $C_2$-$C_4$alkenyl, $C_3$-$C_6$alkynyl, phenyl which is unsubstituted or substituted by halogen, $C_1$-$C_3$alkyl, $C_1$-$C_3$alkoxy, trifluoromethyl or nitro, or benzyl which is unsubstituted or substituted by halogen or nitro, $R^{11}$ is hydrogen, $C_1$-$C_8$alkyl or $C_1$-$C_3$alkoxy, or $R^{10}$ and $R^{11}$ together with the nitrogen atom to which they are bound form a 5- or 6-membered heterocycle which can contain a further hetero atom from the group N, O and S, $R^{12}$, $R^{13}$ and $R^{14}$ are hydrogen, or an unsubstituted or substituted alkyl, alkenyl, alkynyl, cycloalkyl, phenyl or naphthyl radical, or an unsubstituted or substituted heterocyclic radical, or $R^{12}$ and $R^{13}$ are also a cation, or $R^{14}$ is also an alkoxy radical, and $R^{15}$ is hydrogen, amino, mono- or disubstituted amino or an unsubstituted or substituted alkyl, alkenyl, cycloalkyl or phenyl radical, or $R^{14}$ and $R^{15}$ together with the nitrogen atom to which they are bound form an unsubstituted or substituted heterocyclic radical, or A and Z together

form an unsubstituted or substituted tetrahydrofuran-2-one ring, with the inclusion of the acid addition salts and metal complexes thereof; or a composition which contains one of these compounds.

**3.** A process according to claim 1, which comprises using a compound of the formula I in which $R^1$, $R^2$, $R^4$, $R^5$ and $R^6$ are hydrogen, $R^3$ is hydrogen or chlorine, and the radical -A-Z denotes a group $-CH_2-COOR^{16}$ or - $CH(CH_3)-COOR^{16}$, in which $R^{16}$ is $C_1$-$C_{12}$alkyl, $C_3$-$C_6$alkenyl, phenyl-$C_1$-$C_4$alkyl or phenoxy-$C_1$-$C_4$alkyl.

**4.** A process according to claim 1, which comprises using
isopropyl 2-quinolin-8-yloxyacetate,
n-dodecyl 2-(5-chloroquinolin-8-yloxy)acetate,
n-butyl 2-(5-chloroquinolin-8-yloxy)acetate,
n-octyl 2-(5-chloroquinolin-8-yloxy)acetate,
s-butyl 2-quinolin-8-yloxy-acetate,
n-octyl 2-(5-chloroquinolin-8-yloxy)thioacetate,
2-butenyl 2-(5-chloroquinolin-8-yloxy)acetate,
methallyl 2-(5-chloroquinolin-8-yloxy)acetate,
2-isopropyloxyethyl 2-(5-chloroquinolin-8-yloxy)acetate,
2-phenoxyethyl 2-(5-chloroquinolin-8-yloxy)acetate,
1-methylbutyl 2-(5-chloroquinolin-8-yloxy)acetate,
cyclohexyl 2-(5-chloroquinolin-8-yloxy)acetate,
s-butyl 2-(5-chloroquinolin-8-yloxy)acetate,
2-methylpentyl 2-(5-chloroquinolin-8-yloxy)acetate,
n-butyl 2-(5-chloroquinolin-8-yloxy)thioacetate,
3,6-dioxadecyl 2-(5-chloroquinolin-8-yloxy)acetate,
3-methoxybutyl 2-(5-chloroquinolin-8-yloxy)acetate,
1-ethylbutyl 2-(5-chloroquinolin-8-yloxy)acetate,
2-ethylbutyl 2-(5-chloroquinolin-8-yloxy)acetate,
1-methylisopentyl 2-(5-chloroquinolin-8-yloxy)acetate,
n-undecyl 2-(5-chloroquinolin-8-yloxy)acetate,
2-methylbutyl 2-(5-chloroquinolin-8-yloxy)acetate,
s-butyl 2-(5-chloroquinolin-8-yloxy)thioacetate,
3,6-dioxaheptyl 2-(5-chloroquinolin-8-yloxy)acetate,
n-heptyl 2-(5-chloroquinolin-8-yloxy)acetate,
n-dodecyl 2-(5-chloroquinolin-8-yloxy)thioacetate,
n-decyl 2-(5-chloroquinolin-8-yloxy)thioacetate,
1-propylpropargyl 2-(5-chloroquinolin-8-yloxy)acetate,
1-methylisobutyl 2-(5-chloroquinolin-8-yloxy)acetate,
tert-butyl 2-(5-chloroquinolin-8-yloxy)thioacetate,
neopentyl 2-(5-chloroquinolin-8-yloxy)acetate,
n-propyl 2-(5-chloroquinolin-8-yloxy)thioacetate,
1-methylhexyl 2-(5-chloroquinolin-8-yloxy)acetate,
ethyl 2-(5-chloroquinolin-8-yloxy)thioacetate,
2-ethylhexyl 2-(5-chloroquinolin-8-yloxy)acetate,
i-butyl 2-(5-chloroquinolin-8-yloxy)thioacetate,
n-decyl 2-quinolin-8-yloxy-thioacetate,
i-pentyl 2-(5-chloroquinolin-8-yloxy)acetate,
1-ethylpentyl 2-(5-chloroquinolin-8-yloxy)acetate,
1-propylbutyl 2-(5-chloroquinolin-8-yloxy)acetate,
n-hexyl 2-(5-chloroquinolin-8-yloxy)thioacetate,
n-hexyl 2-(5-chloroquinolin-8-yloxy)acetate,
i-propyl 2-(5-chloroquinolin-8-yloxy)thioacetate,
1-pentylallyl 2-(5-chloroquinolin-8-yloxy)acetate,
1-methylpentyl 2-(5-chloroquinolin-8-yloxy)acetate,
1,1-dimethylpropargyl 2-(5-chloroquinolin-8-yloxy)acetate,
1-ethyl-1-methyl-propargyl 2-(5-chloroquinolin-8-yloxy)acetate,
n-butyloxycarbonylmethyl 2-(5-chloroquinolin-8-yloxy)acetate,
1-n-butyloxycarbonylethyl 2-(5-chloroquinolin-8-yloxy)acetate,

1-methylisohexyl 2-(5-chloroquinolin-8-yloxy)acetate,
1-phenylisobutyl 2-(5-chloroquinolin-8-yloxy)acetate,
1-methyl-2-(2-methylphenoxy)ethyl 2-(5-chloroquinolin-8-yloxy)acetate,
1-phenylethyl 2-(5-chloroquinolin-8-yloxy)acetate,
1-methyl-2-(4-ethylphenoxy)ethyl 2-(5-chloroquinolin-8-yloxy)acetate,
1-methyl-2-phenylethyl 2-(5-chloroquinolin-8-yloxy)acetate,
1-methyl-2-(2-isopropylphenoxy)ethyl 2-(5-chloroquinolin-8-yloxy)acetate,
1-phenylpropyl 2-(5-chloroquinolin-8-yloxy)acetate,
1-methyl-2-(2-ethylphenoxy)ethyl 2-(5-chloroquinolin-8-yloxy)acetate,
1-methyl-2-(3-ethylphenoxy)ethyl 2-(5-chloroquinolin-8-yloxy)acetate,
1-methyl-2-phenoxyethyl 2-(5-chloroquinolin-8-yloxy)acetate,
1-methyl-3-phenylpropyl 2-(5-chloroquinolin-8-yloxy)acetate,
1-methyl-2-(3-methylphenoxy)ethyl 2-(5-chloroquinolin-8-yloxy)acetate,
1-methyl-2-(4-isopropylphenoxy)ethyl 2-(5-chloroquinolin-8-yloxy)acetate,
1-methyl-2-(4-methylphenoxy)ethyl 2-(5-chloroquinolin-8-yloxy)acetate,
or a composition which contains one of these compounds.

5. A process according to claim 1, which comprises using methallyl 2-(5-chloroquinolin-8-yloxy)acetate,
2-phenoxyethyl 2-(5-chloroquinolin-8-yloxy)acetate,
1-methylbutyl 2-(5-chloroquinolin-8-yloxy)acetate,
1-ethylbutyl 2-(5-chloroquinolin-8-yloxy)acetate,
1-methylisopentyl 2-(5-chloroquinolin-8-yloxy)acetate,
1-methylhexyl 2-(5-chloroquinolin-8-yloxy)acetate,
1-ethylpentyl 2-(5-chloroquinolin-8-yloxy)acetate,
1-propylbutyl 2-(5-chloroquinolin-8-yloxy)acetate,
1-pentylallyl 2-(5-chloroquinolin-8-yloxy)acetate,
1-methylpentyl 2-(5-chloroquinolin-8-yloxy)acetate,
1-methylisohexyl 2-(5-chloroquinolin-8-yloxy)acetate,
1-phenylisobutyl 2-(5-chloroquinolin-8-yloxy)acetate,
1-phenylethyl 2-(5-chloroquinolin-8-yloxy)acetate,
1-methyl-2-(4-ethylphenoxy)ethyl 2-(5-chloroquinolin-8-yloxy)acetate,
1-methyl-2-phenylethyl 2-(5-chloroquinolin-8-yloxy)acetate,
1-phenylpropyl 2-(5-chloroquinolin-8-yloxy)acetate,
1-methyl-2-phenoxyethyl 2-(5-chloroquinolin-8-yloxy)acetate,
1-methyl-3-phenylpropyl 2-(5-chloroquinolin-8-yloxy)acetate.
1-methyl-2-(4-methylphenoxy)ethyl 2-(5-chloroquinolin-8-yloxy)acetate,
or a composition which contains one of these compounds.

6. A process according to claim 1, which comprises using methyllyl 2-(5-chloroquinolin-8-yloxy)acetate,
2-phenoxyethyl 2-(5-chloroquinolin-8-yloxy)acetate,
1-methylisopentyl 2-(5-chloroquinolin-8-yloxy)acetate or
1-methylhexyl 2-(5-chloroquinolin-8-yloxy)acetate,
or a composition which contains one of these compounds.

7. A process according to claim 1, which comprises using 1-ethylisopentyl 2-(5-chloroquinolin-8-yloxy)-acetate or a composition containing this compound.

8. A process according to claim 1, which comprises using methallyl 2-(5-chloroquinolin-8-yloxy)acetate or a composition containing this compound.

9. A process according to claim 1, which comprises using 2-phenoxyethyl 2-(5-chloroquinolin-8-yloxy)-acetate or a composition containing this compound.

10. A process according to claim 1, which comprises using 1-methylhexyl 2-(5-chloroquinolin-8-yloxy)-acetate or a composition containing this compound.

11. A process according to claim 1 for the protection of cultivated plants against the harmful effects of herbicides of the formula II in which Y is the group $-O-R^{18}$, $-S-R^{18}$ or $-O-N=CR^{19}R^{20}$, in which $R^{18}$ is

hydrogen, $C_1$-$C_4$alkyl, $C_3$-$C_4$alkenyl or $C_3$-$C_4$alkynyl, or $C_1$-$C_4$alkyl substituted by $C_1$-$C_4$alkoxycarbonyl or di-$C_1$-$C_4$alkylamino, $R^{19}$ and $R^{20}$ independently of one another are $C_1$-$C_4$alkyl, or $R^{19}$ and $R^{20}$ together are a $C_4$-$C_7$alkylene chain.

**12.** A process according to claim 11, wherein Y is methoxy, ethoxy, propyloxy, isopropyloxy, butyloxy, dimethylaminoethoxy, propargyloxy, 1-cyano-1-methylethoxy, methoxycarbonylmethylthio, 1-ethoxycarbonylethoxy, butyloxycarbonyl, $-O-N=C(CH_3)_2$, $-O-N=C(CH_3)C_2H_5$ or $-O-N=C(CH_2)_5$.

**13.** A process according to claim 1 for the protection of cultivated plants against the harmful effects of methyl 2-[4-(5-chloro-3-fluoropyridin-2-yloxy)phenoxy]propionate.

**14.** A process according to claim 1 for the protection of cultivated plants against the harmful effects of propargyl 2-[4-(5-chloro-3-fluoropyridin-2-yloxy)phenoxy]propionate.

**15.** A process according to claim 1 for the protection of cultivated plants against the harmful effects of S-methoxycarbonylmethyl 2-[4-(5-chloro-3-fluoropyridin-2-yloxy)phenoxy]thiopropionate.

**16.** A process according to claim 1 for the protection of cultivated plants against the harmful effects of 1-ethoxycarbonylethyl 2-[4-(5-chloro-3-fluoropyridin-2-yloxy)phenoxy]propionate.

**17.** A process according to claim 1 for the protection of cultivated plants against the harmful effects of methyl 2-[4-(5-chloro-3-fluoropyridin-2-yloxy)phenoxy]propionate, propargyl 2-[4-(5-chloro-3-fluoropyridin-2-yloxy)phenoxy]propionate, S-methoxycarbonylmethyl 2-[4-(5-chloro-3-fluoropyridin-2-yloxy)phenoxy]thiopropionate or 1-ethoxycarbonylethyl 2-[4-(5-chloro-3-fluoropyridin-2-yloxy)phenoxy]-propionate, which comprises treating the cultivated plants or parts of these plants or soils intended for the growing of the cultivated plants with
isopropyl 2-quinolin-8-yloxyacetate,
n-dodecyl 2-(5-chloroquinolin-8-yloxy)acetate,
n-butyl 2-(5-chloroquinolin-8-yloxy)acetate,
n-octyl 2-(5-chloroquinolin-8-yloxy)acetate,
s-butyl 2-quinolin-8-yloxy-acetate,
n-octyl 2-(5-chloroquinolin-8-yloxy)thioacetate,
2-butenyl 2-(5-chloroquinolin-8-yloxy)acetate,
methallyl 2-(5-chloroquinolin-8-yloxy)acetate,
2-isopropyloxyethyl 2-(5-chloroquinolin-8-yloxy)acetate,
2-phenoxyethyl 2-(5-chloroquinolin-8-yloxy)acetate,
1-methylbutyl 2-(5-chloroquinolin-8-yloxy)acetate,
cyclohexyl 2-(5-chloroquinolin-8-yloxy)acetate,
s-butyl 2-(5-chloroquinolin-8-yloxy)acetate,
2-methylpentyl 2-(5-chloroquinolin-8-yloxy)acetate,
n-butyl 2-(5-chloroquinolin-8-yloxy)thioacetate,
3,6-dioxadecyl 2-(5-chloroquinolin-8-yloxy)acetate,
3-methoxybutyl 2-(5-chloroquinolin-8-yloxy)acetate,
1-ethylbutyl 2-(5-chloroquinolin-8-yloxy)acetate,
2-ethylbutyl 2-(5-chloroquinolin-8-yloxy)acetate,
1-methylisopentyl 2-(5-chloroquinolin-8-yloxy)acetate,
n-undecyl 2-(5-chloroquinolin-8-yloxy)acetate,
2-methylbutyl 2-(5-chloroquinolin-8-yloxy)acetate,
s-butyl 2-(5-chloroquinolin-8-yloxy)thioacetate,
3,6-dioxaheptyl 2-(5-chloroquinolin-8-yloxy)acetate,
n-heptyl 2-(5-chloroquinolin-8-yloxy)acetate,
n-dodecyl 2-(5-chloroquinolin-8-yloxy)thioacetate,
n-decyl 2-(5-chloroquinolin-8-yloxy)thioacetate,
1-propylpropargyl 2-(5-chloroquinolin-8-yloxy)acetate,
1-methylisobutyl 2-(5-chloroquinolin-8-yloxy)acetate,
tert-butyl 2-(5-chloroquinolin-8-yloxy)thioacetate,
neopentyl 2-(5-chloroquinolin-8-yloxy)acetate,
n-propyl 2-(5-chloroquinolin-8-yloxy)thioacetate,

1-methylhexyl 2-(5-chloroquinolin-8-yloxy)acetate,
ethyl 2-(5-chloroquinolin-8-yloxy)thioacetate,
2-ethylhexyl 2-(5-chloroquinolin-8-yloxy)acetate,
i-butyl 2-(5-chloroquinolin-8-yloxy)thioacetate,
n-decyl 2-quinolin-8-yloxy-thioacetate,
i-pentyl 2-(5-chloroquinolin-8-yloxy)acetate,
1-ethylpentyl 2-(5-chloroquinolin-8-yloxy)acetate,
1-propylbutyl 2-(5-chloroquinolin-8-yloxy)acetate,
n-hexyl 2-(5-chloroquinolin-8-yloxy)thioacetate,
n-hexyl 2-(5-chloroquinolin-8-yloxy)acetate,
i-propyl 2-(5-chloroquinolin-8-yloxy)thioacetate,
1-pentylallyl 2-(5-chloroquinolin-8-yloxy)acetate,
1-methylpentyl 2-(5-chloroquinolin-8-yloxy)acetate,
1,1-dimethylpropargyl 2-(5-chloroquinolin-8-yloxy)acetate,
1-ethyl-1-methyl-propargyl 2-(5-chloroquinolin-8-yloxy)acetate,
n-butyloxycarbonylmethyl 2-(5-chloroquinolin-8-yloxy)acetate,
1-n-butyloxycarbonylethyl 2-(5-chloroquinolin-8-yloxy)acetate,
1-methylisohexyl 2-(5-chloroquinolin-8-yloxy)acetate,
1-phenylisobutyl 2-(5-chloroquinolin-8-yloxy)acetate,
1-methyl-2-(2-methylphenoxy)ethyl 2-(5-chloroquinolin-8-yloxy)acetate,
1-phenylethyl 2-(5-chloroquinolin-8-yloxy)acetate,
1-methyl-2-(4-ethylphenoxy)ethyl 2-(5-chloroquinolin-8-yloxy)acetate,
1-methyl-2-phenylethyl 2-(5-chloroquinolin-8-yloxy)acetate,
1-methyl-2-(2-isopropylphenoxy)ethyl 2-(5-chloroquinolin-8-yloxy)acetate,
1-phenylpropyl 2-(5-chloroquinolin-8-yloxy)acetate,
1-methyl-2-(2-ethylphenoxy)ethyl 2-(5-chloroquinolin-8-yloxy)acetate,
1-methyl-2-(3-ethylphenoxy)ethyl 2-(5-chloroquinolin-8-yloxy)acetate,
1-methyl-2-phenoxyethyl 2-(5-chloroquinolin-8-yloxy)acetate,
1-methyl-3-phenylpropyl 2-(5-chloroquinolin-8-yloxy)acetate,
1-methyl-2-(3-methylphenoxy)ethyl 2-(5-chloroquinolin-8-yloxy)acetate,
1-methyl-2-(4-isopropylphenoxy)ethyl 2-(5-chloroquinolin-8-yloxy)acetate,
1-methyl-2-(4-methylphenoxy)ethyl 2-(5-chloroquinolin-8-yloxy)acetate,
or with a composition which contains one of these compounds.

**18.** A process according to claim 17, which comprises treating the cultivated plants or parts of these plants or soils intended for the growing of the cultivated plants with
methallyl 2-(5-chloroquinolin-8-yloxy)acetate,
2-phenoxyethyl 2-(5-chloroquinolin-8-yloxy)acetate,
1-methylbutyl 2-(5-chloroquinolin-8-yloxy)acetate,
1-ethylbutyl 2-(5-chloroquinolin-8-yloxy)acetate,
1-methylisopentyl 2-(5-chloroquinolin-8-yloxy)acetate,
1-methylhexyl 2-(5-chloroquinolin-8-yloxy)acetate,
1-ethylpentyl 2-(5-chloroquinolin-8-yloxy)acetate,
1-propylbutyl 2-(5-chloroquinolin-8-yloxy)acetate,
1-pentylallyl 2-(5-chloroquinolin-8-yloxy)acetate,
1-methylpentyl 2-(5-chloroquinolin-8-yloxy)acetate,
1-methylisohexyl 2-(5-chloroquinolin-8-yloxy)acetate,
1-phenylisobutyl 2-(5-chloroquinolin-8-yloxy)acetate,
1-phenylethyl 2-(5-chloroquinolin-8-yloxy)acetate,
1-methyl-2-(4-ethylphenoxy)ethyl 2-(5-chloroquinolin-8-yloxy)acetate,
1-methyl-2-phenylethyl 2-(5-chloroquinolin-8-yloxy)acetate,
1-phenylpropyl 2-(5-chloroquinolin-8-yloxy)acetate,
1-methyl-2-phenoxyethyl 2-(5-chloroquinolin-8-yloxy)acetate,
1-methyl-3-phenylpropyl 2-(5-chloroquinolin-8-yloxy)acetate.
1-methyl-2-(4-methylphenoxy)ethyl 2-(5-chloroquinolin-8-yloxy)acetate,
or with a composition which contains one of these compounds.

**19.** A process according to claim 17, which comprises treating the cultivated plants or parts of these plants,

or soils intended for the growing of the cultivated plants, with 1-methylisopentyl 2-(5-chloroquinolin-8-yloxy)acetate, 2-phenoxyethyl 2-(5-chloroquinolin-8-yloxy)acetate, 1-methylhexyl 2-(5-chloroquinolin-8-yloxy)acetate or methallyl 2-(5-chloroquinolin-8-yloxy)acetate, or with a composition containing one of these compounds.

**20.** A process according to claim 1 for the protection of cereals.

**21.** A process according to claim 20 for the protection of wheat, barley, rye and rice.

**22.** A process according to claim 1, which comprises treating crops of cultivated plants or cultivation areas for cultivated plants with 0.01 to 10 kg/ha of a compound of the formula I according to claim 1.

**23.** A process according to claim 22, which comprises treating crops of cultivated plants or cultivation areas for cultivated plants with 0.05 to 0.5 kg/ha of a compound of the formula I according to claim 1.

**24.** A process according to claim 1, which comprises treating seed of the cultivated plants with a compound of the formula I according to claim 1.

**25.** A process according to claim 24, which comprises treating seed of the cultivated plants with 0.01 to 10 g of a compound of the formula I according to claim 1 per kg of seed.

**26.** A process according to claim 25, which comprises treating seed of the cultivated plants with 0.05 to 2 g of a compound of the formula I according to claim 1 per kg of seed.

**27.** A process according to claim 24 for the protection of cultivated plants against the harmful effects of methyl 2-[4-(5-chloro-3-fluoropyridin-2-yloxy)phenoxy]propionate, propargyl 2-[4-(5-chloro-3-fluoropyridin-2-yloxy)phenoxy]propionateS-methoxycarbonylmethyl 2-[4-(5-chloro-3-fluoropyridin-2-yloxy)phenoxy]thiopropionate or 1-ethoxycarbonylethyl 2-[4-(5-chloro-3-fluoropyridin-2-yloxy)phenoxy]-propionate, which comprises treating the seed of the cultivated plants with:
n-dodecyl 2-(5-chloroquinolin-8-yloxy)acetate,
n-butyl 2-(5-chloroquinolin-8-yloxy)acetate,
n-octyl 2-(5-chloroquinolin-8-yloxy)acetate,
s-butyl 2-quinolin-8-yloxy-acetate,
n-octyl 2-(5-chloroquinolin-8-yloxy)thioacetate,
2-butenyl 2-(5-chloroquinolin-8-yloxy)acetate,
methallyl 2-(5-chloroquinolin-8-yloxy)acetate,
2-isopropyloxyethyl 2-(5-chloroquinolin-8-yloxy)acetate,
2-phenoxyethyl 2-(5-chloroquinolin-8-yloxy)acetate,
1-methylbutyl 2-(5-chloroquinolin-8-yloxy)acetate,
cyclohexyl 2-(5-chloroquinolin-8-yloxy)acetate,
s-butyl 2-<5-chloroquinolin-8-yloxy)acetate,
2-methylpentyl 2-(5-chloroquinolin-8-yloxy)acetate,
n-butyl 2-(5-chloroquinolin-8-yloxy)thioacetate,
3,6-dioxadecyl 2-(5-chloroquinolin-8-yloxy)acetate,
3-methoxybutyl 2-(5-chloroquinolin-8-yloxy)acetate,
1-ethylbutyl 2-(5-chloroquinolin-8-yloxy)acetate,
2-ethylbutyl 2-(5-ohloroquinolin-8-yloxy)acetate,
1-methylisopentyl 2-(5-chloroquinolin-8-yloxy)acetate,
n-undecyl 2-(5-chloroquinolin-8-yloxy)acetate,
2-methylbutyl 2-(5-chloroquinolin-8-yloxy)acetate,
s-butyl 2-(5-chloroquinolin-8-yloxy)thioacetate,
3,6-dioxaheptyl 2-(5-chloroquinolin-8-yloxy)acetate,
n-heptyl 2-(5-chloroquinolin-8-yloxy)acetate,
n-dodecyl 2-(5-chloroquinolin-8-yloxy)thioacetate,
n-decyl 2-(5-chloroquinolin-8-yloxy)thioacetate,
1-propylpropargyl 2-(5-chloroquinolin-8-yloxy)acetate,
1-methylisobutyl 2-(5-chloroquinolin-8-yloxy)acetate,
tert-butyl 2-(5-chloroquinolin-8-yloxy)thioacetate,

neopentyl 2-(5-chloroquinolin-8-yloxy)acetate,
n-propyl 2-(5-chloroquinolin-8-yloxy)thioacetate,
1-methylhexyl 2-(5-chloroquinolin-8-yloxy)acetate,
ethyl 2-(5-chloroquinolin-8-yloxy)thioacetate,
2-ethylhexyl 2-(5-chloroquinolin-8-yloxy)acetate,
i-butyl 2-(5-chloroquinolin-8-yloxy)-thioacetate,
n-decyl 2-quinolin-8-yloxythioacetate,
i-pentyl 2-(5-chloroquinolin-8-yloxy)acetate,
1-ethylpentyl 2-(5-chloroquinolin-8-yloxy)acetate,
1-propylbutyl 2-(5-chloroquinolin-8-yloxy)acetate,
n-hexyl 2-(5-chloroquinolin-8-yloxy)thioacetate,
n-hexyl 2-(5-chloroquinolin-8-yloxy)acetate,
i-propyl 2-(5-chloroquinolin-8-yloxy)thioacetate,
1-pentylallyl 2-(5-chloroquinolin-8-yloxy)acetate,
1-methylpentyl 2-(5-chloroquinolin-8-yloxy)acetate,
1,1-dimethylpropargyl 2-(5-chloroquinolin-8-yloxy)acetate,
1-ethyl-1-methyl-propargyl 2-(5-chloroquinolin-8-yloxy)acetate,
n-butyloxycarbonylmethyl 2-(5-chloroquinolin-8-yloxy)acetate,
1-n-butyloxycarbonylethyl 2-(5-chloroquinolin-8-yloxy)acetate,
1-methylisohexyl 2-(5-chloroquinolin-8-yloxy)acetate,
1-phenylisobutyl 2-(5-chloroquinolin-8-yloxy)acetate,
1-methyl-2-(2-methylphenoxy)ethyl 2-(5-chloroquinolin-8-yloxy)acetate,
1-phenylethyl 2-(5-chloroquinolin-8-yloxy)acetate,
1-methyl-2-(4-ethylphenoxy)ethyl 2-(5-chloroquinolin-8-yloxy)acetate,
1-methyl-2-phenylethyl 2-(5-chloroquinolin-8-yloxy)acetate,
1-methyl-2-(2-isopropylphenoxy)ethyl 2-(5-chloroquinolin-8-yloxy)acetate,
1-phenylpropyl 2-(5-chloroquinolin-8-yloxy)acetate,
1-methyl-2-(2-ethylphenoxy)ethyl 2-(5-chloroquinolin-8-yloxy)acetate,
1-methyl-2-(3-ethylphenoxy)ethyl 2-(5-chloroquinolin-8-yloxy)acetate,
1-methyl-2-phenoxyethyl 2-(5-chloroquinolin-8-yloxy)acetate,
1-methyl-3-phenylpropyl 2-(5-chloroquinolin-8-yloxy)acetate,
1-methyl-2-(3-methylphenoxy)ethyl 2-(5-chloroquinolin-8-yloxy)acetate,
1-methyl-2-(4-isopropylphenoxy)ethyl 2-(5-chloroquinolin-8-yloxy)acetate,
1-methyl-2-(4-methylphenoxy)ethyl 2-(5-chloroquinolin-8-yloxy)acetate,
or with a composition which contains one of these compounds.

**28.** A process according to claim 27, which comprises treating the seeds of the cultivated plants with:
methallyl 2-(5-chloroquinolin-8-yloxy)acetate,
2-phenoxyethyl 2-(5-chloroquinolin-8-yloxy)acetate,
1-methylbutyl 2-(5-chloroquinolin-8-yloxy)acetate,
1-ethylbutyl 2-(5-chloroquinolin-8-yloxy)acetate,
1-methylisopentyl 2-(5-chloroquinolin-8-yloxy)acetate,
1-methylhexyl 2-(5-chloroquinolin-8-yloxy)acetate,
1-ethylpentyl 2-(5-chloroquinolin-8-yloxy)acetate,
1-propylbutyl 2-(5-chloroquinolin-8-yloxy)acetate,
1-pentylallyl 2-(5-chloroquinolin-8-yloxy)acetate,
1-methylpentyl 2-(5-chloroquinolin-8-yloxy)acetate,
1-methylisohexyl 2-(5-chloroquinolin-8-yloxy)acetate,
1-phenylisobutyl 2-(5-chloroquinolin-8-yloxy)acetate,
1-phenylethyl 2-(5-chloroquinolin-8-yloxy)acetate,
1-methyl-2-(4-ethylphenoxy)ethyl 2-(5-chloroquinolin-8-yloxy)acetate,
1-methyl-2-phenylethyl 2-(5-chloroquinolin-8-yloxy)acetate,
1-phenylpropyl 2-(5-chloroquinolin-8-yloxy)acetate,
1-methyl-2-phenoxyethyl 2-(5-chloroquinolin-8-yloxy)acetate,
1-methyl-3-phenylpropyl 2-(5-chloroquinolin-8-yloxy)acetate.
1-methyl-2-(4-methylphenoxy)ethyl 2-(5-chloroquinolin-8-yloxy)acetate,
or with a composition which contains one of these compounds.

**29.** A process according to claim 27, which comprises treating the seeds of the cultivated plants with 1-methylisopentyl 2-(5-chloroquinolin-8-yloxy)acetate, 2-phenoxyethyl 2-(5-chloroquinolin-8-yloxy)acetate, 1-methylhexyl 2-(5-chloroquinolin-8-yloxy)acetate or methallyl 2-(5-chloroquinolin-8-yloxy)acetate, or with a composition containing one of these compounds.

**30.** A process according to claim 27 for the protection of cereals.

**31.** A method of using compounds of the formula I according to claim 1 for the protection of cultivated plants against the harmful effects of compounds of the formula II according to the claim 1.

**32.** A process for selectively controlling weeds in crops of cultivated plants, which process comprises treating the crops of cultivated plants, parts of the cultivated plants or cultivation areas for the cultivated plants with an antidote of the formula I according to claim 1 and a herbicide of the formula II according to claim 1.

**33.** A herbicidal composition which comprises, besides a herbicidal active ingredient of the formula II according to claim 1, an antidote of the formula I according to claim 1.

**34.** A composition according to claim 33 which comprises, besides a herbicide selected from the series methyl 2-[4-(5-chloro-3-fluoropyridin-2-yloxy)phenoxy]propionate, propargyl 2-[4-(5-chloro-3-fluoropyridin-2-yloxy)phenoxy]propionate, S-methoxycarbonylmethyl 2-[4-(5-chloro-3-fluoro-pyridin-2-yloxy)phenoxy]thiopropionate and 1-ethoxycarbonylethyl 2-[4-(5-chloro-3-fluoropyridin-2-yloxy)-phenoxy]propionate, an antidote selected from the group comprising:
n-dodecyl 2-(5-chloroquinolin-8-yloxy)acetate,
n-butyl 2-(5-chloroquinolin-8-yloxy)acetate,
n-octyl 2-(5-chloroquinolin-8-yloxy)acetate,
s-butyl 2-quinolin-8-yloxy-acetate,
n-octyl 2-(5-chloroquinolin-8-yloxy)thioacetate,
2-butenyl 2-(5-chloroquinolin-8-yloxy)acetate,
methallyl 2-(5-chloroquinolin-8-yloxy)acetate,
2-isopropyloxyethyl 2-(5-chloroquinolin-8-yloxy)acetate,
2-phenoxyethyl 2-(5-chloroquinolin-8-yloxy)acetate,
1-methylbutyl 2-(5-chloroquinolin-8-yloxy)acetate,
cyclohexyl 2-(5-chloroquinolin-8-yloxy)acetate,
s-butyl 2-(5-chloroquinolin-8-yloxy)acetate,
2-methylpentyl 2-(5-chloroquinolin-8-yloxy)acetate,
n-butyl 2-(5-chloroquinolin-8-yloxy)thioacetate,
3,6-dioxadecyl 2-(5-chloroquinolin-8-yloxy)acetate,
3-methoxybutyl 2-(5-chloroquinolin-8-yloxy)acetate,
1-ethylbutyl 2-(5-chloroquinolin-8-yloxy)acetate,
2-ethylbutyl 2-(5-chloroquinolin-8-yloxy)acetate,
1-methylisopentyl 2-(5-chloroquinolin-8-yloxy)acetate,
n-undecyl 2-(5-chloroquinolin-8-yloxy)acetate,
2-methylbutyl 2-(5-chloroquinolin-8-yloxy)acetate,
s-butyl 2-(5-chloroquinolin-8-yloxy)thioacetate,
3,6-dioxaheptyl 2-(5-chloroquinolin-8-yloxy)acetate,
n-heptyl 2-(5-chloroquinolin-8-yloxy)acetate,
n-dodecyl 2-(5-chloroquinolin-8-yloxy)thioacetate,
n-decyl 2-(5-chloroquinolin-8-yloxy)thioacetate,
1-propylpropargyl 2-(5-chloroquinolin-8-yloxy)acetate,
1-methylisobutyl 2-(5-chloroquinolin-8-yloxy)acetate,
tert-butyl 2-(5-chloroquinolin-8-yloxy)thioacetate,
neopentyl 2-(5-chloroquinolin-8-yloxy)acetate,
n-propyl 2-(5-chloroquinolin-8-yloxy)thioacetate,
1-methylhexyl 2-(5-chloroquinolin-8-yloxy)acetate,
ethyl 2-(5-chloroquinolin-8-yloxy)thioacetate,
2-ethylhexyl 2-(5-chloroquinolin-8-yloxy)acetate,
i-butyl 2-(5-chloroquinolin-8-yloxy)thioacetate,

n-decyl 2-quinolin-8-yloxy-thioacetate,
i-pentyl 2-(5-chloroquinolin-8-yloxy)acetate,
1-ethylpentyl 2-(5-chloroquinolin-8-yloxy)acetate,
1-propylbutyl 2-(5-chloroquinolin-8-yloxy)acetate,
n-hexyl 2-(5-chloroquinolin-8-yloxy)thioacetate,
n-hexyl 2-(5-chloroquinolin-8-yloxy)acetate,
i-propyl 2-(5-chloroquinolin-8-yloxy)thioacetate,
1-pentylallyl 2-(5-chloroquinolin-8-yloxy)acetate,
1-methylpentyl 2-(5-chloroquinolin-8-yloxy)acetate,
1,1-dimethylpropargyl 2-(5-chloroquinolin-8-yloxy)acetate,
1-ethyl-1-methyl-propargyl 2-(5-chloroquinolin-8-yloxy)acetate,
n-butyloxycarbonylmethyl 2-(5-chloroquinolin-8-yloxy)acetate,
1-n-butyloxycarbonylethyl 2-(5-chloroquinolin-8-yloxy)acetate,
1-methylisohexyl 2-(5-chloroquinolin-8-yloxy)acetate,
1-phenylisobutyl 2-(5-chloroquinolin-8-yloxy)acetate,
1-methyl-2-(2-methylphenoxy)ethyl 2-(5-chloroquinolin-8-yloxy)acetate,
1-phenylethyl 2-(5-chloroquinolin-8-yloxy)acetate,
1-methyl-2-(4-ethylphenoxy)ethyl 2-(5-chloroquinolin-8-yloxy)acetate,
1-methyl-2-phenylethyl 2-(5-chloroquinolin-8-yloxy)acetate,
1-methyl-2-(2-isopropylphenoxy)ethyl 2-(5-chloroquinolin-8-yloxy)acetate,
1-phenylpropyl 2-(5-chloroquinolin-8-yloxy)acetate,
1-methyl-2-(2-ethylphenoxy)ethyl 2-(5-chloroquinolin-8-yloxy)acetate,
1-methyl-2-(3-ethylphenoxy)ethyl 2-(5-chloroquinolin-8-yloxy)acetate,
1-methyl-2-phenoxyethyl 2-(5-chloroquinolin-8-yloxy)acetate,
1-methyl-3-phenylpropyl 2-(5-chloroquinolin-8-yloxy)acetate,
1-methyl-2-(3-methylphenoxy)ethyl 2-(5-chloroquinolin-8-yloxy)acetate,
1-methyl-2-(4-isopropylphenoxy)ethyl 2-(5-chloroquinolin-8-yloxy)acetate,
1-methyl-2-(4-methylphenoxy) ethyl 2-(5-chloroquinolin-8-yloxy)acetate.

**35.** A composition according to claim 34, which comprises an antidote selected from the series
2-phenoxyethyl 2-(5-chloroquinolin-8-yloxy)acetate,
1-methylbutyl 2-(5-chloroquinolin-8-yloxy)acetate,
1-ethylbutyl 2-(5-chloroquinolin-8-yloxy)acetate,
1-methylisopentyl 2-(5-chloroquinolin-8-yloxy)acetate,
1-methylhexyl 2-(5-chloroquinolin-8-yloxy)acetate,
1-ethylpentyl 2-(5-chloroquinolin-8-yloxy)acetate,
1-propylbutyl 2-(5-chloroquinolin-8-yloxy)acetate,
1-pentylallyl 2-(5-chloroquinolin-8-yloxy)acetate,
1-methylpentyl 2-(5-chloroquinolin-8-yloxy)acetate,
1-methylisohexyl 2-(5-chloroquinolin-8-yloxy)acetate,
1-phenylisobutyl 2-(5-chloroquinolin-8-yloxy)acetate,
1-phenylethyl 2-(5-chloroquinolin-8-yloxy)acetate,
1-methyl-2-(4-ethylphenoxy)ethyl 2-(5-chloroquinolin-8-yloxy)acetate,
1-methyl-2-phenylethyl 2-(5-chloroquinolin-8-yloxy)acetate,
1-phenylpropyl 2-(5-chloroquinolin-8-yloxy)acetate,
1-methyl-2-phenoxyethyl 2-(5-chloroquinolin-8-yloxy)acetate,
1-methyl-3-phenylpropyl 2-(5-chloroquinolin-8-yloxy)acetate.
1-methyl-2-(4-methylphenoxy)ethyl 2-(5-chloroquinolin-8-yloxy)acetate.

**36.** A composition according to claim 34, which comprises as antidote methallyl 2-(5-chloroquinolin-8-yloxy)acetate,
2-phenoxyethyl 2-(5-chloroquinolin-8-yloxy)acetate,
1-methyisopentyl 2-(5-chloroquinolin-8-yloxy)acetate or
1-methylhexyl 2-(5-chloroquinolin-8-yloxy)acetate.

**37.** A compound selected from the group comprising
R-1-methylisopentyl 2-(5-chloroquinolin-8-yloxy)acetate,
S-1-methylisopentyl 2-(5-chloroquinolin-8-yloxy)acetate,

R-1-methylhexyl 2-(5-chloroquinolin-8-yloxy)acetate,
S-1-methylhexyl 2-(5-chloroquinolin-8-yloxy)acetate,
1-methylisohexyl 2-(5-chloroquinolin-8-yloxy)acetate,
1-phenylisobutyl 2-(5-chloroquinolin-8-yloxy)acetate,
1-phenylethyl 2-(5-chloroquinolin-8-yloxy)acetate,
1-methyl-2-(4-ethylphenoxy)ethyl 2-(5-chloroquinolin-8-yloxy)acetate,
1-methyl-2-phenylethyl 2-(5-chloroquinolin-8-yloxy)acetate,
1-phenylpropyl 2-(5-chloroquinolin-8-yloxy)acetate,
1-methyl-2-phenoxyethyl 2-(5-chloroquinolin-8-yloxy)acetate,
1-methyl-3-phenylpropyl 2-(5-chloroquinolin-8-yloxy)acetate and
1-methyl-2-(4-methylphenoxy)ethyl 2-(5-chloroquinolin-8-yloxy)acetate.

**38.** A process for producing the compositions according to claim 33, which comprises thoroughly mixing together the active ingredients of the formulae I and II and optionally a carrier and/or a surfactant.

**39.** A process for producing the compounds according to claim 37, which comprises reacting 5-chloro-8-hydroxyquinoline, in the presence of an acid-binding agent, with a compound selected from the group comprising
R-1-methylisopentyl bromoacetate,
S-1-methylisopentyl bromoacetate,
R-1-methylhexyl bromoacetate,
S-1-methylhexyl bromoacetate,
1-methylisohexyl bromoacetate,
1-phenylisobutyl bromoacetate,
1-phenylethyl bromoacetate,
1-methyl-2-(4-ethylphenoxy)ethyl bromoacetate,
1-methyl-2-phenylethyl bromoacetate,
1-phenylpropyl bromoacetate,
1-methyl-2-phenoxyethyl bromoacetate,
1-methyl-3-phenylpropyl bromoacetate and
1-methyl-2-(4-methylphenoxy) ethyl bromoacetate.

## Revendications

**1.** Procédé pour la protection de plantes cultivables contre les actions nuisibles de dérivés d'acide 2-[4-(5-chloro-3-fluoropyridin-2-yloxy)-phénoxy]-propionique a activité herbicide de formule II

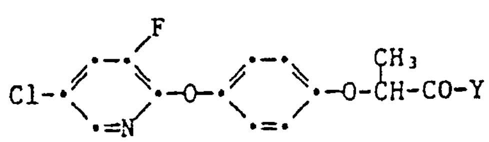

(II)

où Y désigne un groupe $-NR^{16}R^{17}$, $-O-R^{18}$, $-S-R^{18}$ ou $-O-N=CR^{19}R^{20}$.
$R^{16}$ et $R^{17}$ représentent, indépendamment l'un de l'autre, l'hydrogène ou un radical alcoxy en $C_1$-$C_8$, alkyle en $C_1$-$C_8$, phényle ou benzyle,
$R^{16}$ et $R^{17}$ forment ensemble avec l'atome d'azote qui les porte un hétérocycle azoté saturé à 5-6 chaînons, qui peut être interrompu par un atome d'oxygène ou de soufre,
$R^{18}$ représente l'hydrogène ou l'équivalent d'un ion de métal alcalin, de métal alcalino-terreux, de cuivre ou de fer ; un reste d'alkyl($C_1$-$C_4$)ammonium ou d'hydroxyalkyl($C_1$-$C_4$)ammonium quaternaire ; un reste alkyle en $C_1$-$C_9$ éventuellement substitué une ou plusieurs fois par des radicaux amino, halogéno, hydroxyle, cyano, nitro, phényle, alcoxy en $C_1$-$C_4$ polyéthoxy ayant 2 à 6 motifs oxyde d'éthylène, $-COOR^{21}$ , $-COSR^{21}$, $-CONH_2$-, -CON(alcoxy en $C_1$-$C_4$)-alkyle en $C_1$-$C_4$-, -CO-N-di-alkyle en $C_1$-$C_4$, -CONH-alkyle en $C_1$-$C_4$, -N(alcoxy en $C_1$-$C_4$)-alkyle en $C_1$-$C_4$ ou di-alkyl($C_1$-$C_4$)amino ; un rsete alcényle en $c_3$-$c_9$ éventuellement substitué par un halogène ou un radical alcoxy en $C_1$-$C_4$ ; un reste alcynyle en $C_3$-$C_9$ éventuellement substitué par un halogène ou un radical alcoxy en $C_1$-$C_4$ ; un radical cycloalkyle en $C_3$-$C_9$ ; ou un radical phényle éventuellement substitué par un groupe cyano, alkyle en $C_1$-$C_4$, alcoxy en $C_1$-$C_4$, acétyle, $-COOR^{21}$, $-COSR^{21}$, $-CONH_2$, -CON(alcoxy en $C_1$-$C_4$)-alkyle en $C_1$-$C_4$,

-CO-N-di-alkyle en $C_1$-$C_4$ ou -CONH-alkyle en $C_1$-$C_4$,
$R^{19}$ et $R^{20}$ représentent, indépendamment l'un de l'autre, un radical alkyle en $C_1$-$C_4$ ou ensemble une chaîne alkylène à 3-6 chaînons et
$R^{21}$ représente l'hydrogène ou un radical alkyle en $C_1$-$C_6$,halogénoalkyle en $C_1$-$C_6$, alcoxyalkyle en $C_2$-$C_6$, alcényle en $C_3$-$C_6$, halogénoalcényle en $C_3$-$C_6$, alcynyle en $C_3$-$C_6$ ou halogénoalcynyle en $C_3$-$C_6$,
caractérisé en ce qu'on traite les plantes cultivées, des parties de ces plantes ou des sols destinés à la culture des plantes cultivables avec un composé de formule I

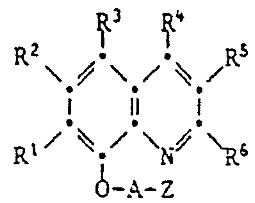

(I),

ou $R^1$, $R^2$ et $R^3$ représentent, indépendamment les uns des autres, l'hydrogène, un halogène ou un radical nitro, cyano, alkyle en $C_1$-$C_3$ ou alcoxy en $C_1$-$C_3$,
$R^4$, $R^5$ et $R^6$ représentent, indépendamment les uns des autres, l'hydrogène, un halogène ou un radical alkyle en $C_1$-$C_3$,
A est l'un des groupes $-CH_2-$, $-CH_2-CH_2-$ ou $-CH(CH_3)-$ et Z représente a) un radical cyano ou amidoxime, qui peut être acylé sur l'atome d'oxygène, ou b) un groupe carboxyle ou un sel de celui-ci, un groupe mercaptocarbonyle ou un sel de celui-ci, un groupe ester d'acide carboxylique, un groupe thioester d'acide carboxylique, un groupe amide d'acide carboxylique substitué ou non, un dérivé cyclisé, substitué ou non, d'un groupe amide d'acide carboxylique ou un groupe hydrazide d'acide carboxylique, ou
A et Z forment ensemble un cycle tétrahydrofurann-2-one substitué ou non,
y compris leurs sels d'addition avec un acide et leurs complexes métalliques, ou un agent contenant l'un de ces composés.

2. Procédé selon la revendication 1, caractérisé en ce utilise un composé de formule I dans lequel :
$R^1$, $R^2$ et $R^3$ représentent, indépendamment les uns des autres, l'hydrogène, un halogène ou un radical nitro, cyano, alkyle en $C_1$-$C_3$ ou alcoxy en $C_1$-$C_3$,
$R^4$, $R^5$ et $R^6$ représentent, indépendamment les uns des autres, l'hydrogène, un halogène ou un radical alkyle en $C_1$-$C_3$,
A est l'un des groupes $-CH_2-$, $-CH_2-CH_2-$ ou $-CH(CH_3)-$ et Z représente l'un des groupes $-C(NH_2)=N-OH$ ou $-C(NH_2)=N-O-CO-E$, un reste oxazolin-2-yle éventuellement substitué, $-COOR^{12}$, $-COSR^{13}$ ou $-CONR^{14}R^{15}$, dans lesquels E représente les radicaux $-R^7$, $-OR^8$, $-SR^9$ ou $-NR^{10}R^{11}$, où
$R^7$ représente un radical alkyle en $C_1$-$C_7$, non substitué ou substitué par un halogène ou un groupe alcoxy en $C_1$-$C_4$, un radical cycloalkyle en $C_3$-$C_6$, un radical alcényle en $C_2$-$C_4$, un radical phényle, qui est non substitué ou substitué par un halogène ou un groupe nitroou alkyle en $C_1$-$C_3$, un radical benzyle, qui est non substitué ou substitué par un halogène ou un groupe nitro ou alkyle en $C_1$-$C_3$, ou un hétérocycle à 5 à 6 chaînons, qui contient un ou deux hétéroatomes du groupe N, O ou S et qui est non substitué ou substitué par un halogène,
$R^8$, $R^9$ et $R^{10}$ représentent, indépendamment les uns des autres, un radical alkyle en $C_1$-$C_8$, qui est non substitué on substitué par un halogène, un radical alcényle en $C_2$-$C_4$, un radical alcynyle en $C_3$-$C_6$, un radical phényle, qui est non substitué ou substitué par un halogène ou un groupe alkyle en $C_1$-$C_3$, alcoxy en $C_1$-$C_3$, trifluorométhyle ou nitro, ou un radical benzyle, qui est non substitué ou substitué par un halogène ou un groupe nitro,
$R^{11}$ représente l'hydrogène ou un radical alkyle en $C_1$-$C_8$ ou alcoxy en $C_1$-$C_3$, ou
$R^{10}$ et $R^{11}$ forment ensemble avec l'atome d'azote auquel ils sont liés un hétérocycle à 5-6 chaînons, qui peut encore comporter un autre hétéroatome du groupe N, O ou S,
$R^{12}$, $R^{13}$ et $R^{14}$ représentent l'hydrogène ou un reste alkyle, alcényle, alcynyle, cycloalkyle, phényle ou naphtyle éventuellement substitué ou un reste hétérocyclique éventuellement substitué, ou $R^{12}$ et $R^{13}$ est aussi un cation ou $R^{14}$ est aussi un reste alcoxy et $R^{15}$ représente l'hydrogène ou un reste amino ou aminomono- ou disubstitué, ou un reste alkyle, alcényle, cycloalkyle ou phényle éventuellement substitué, ou $R^{14}$ et $R^{15}$ représentent en commun avec l'atome d'azote auquel ils sont liés un reste

hétérocyclique éventuellement substitué, ou A et Z forment ensemble un cycle tétrahydrofurann-2-one éventuellement substitué, y compris leurs sels d'addition avec un acide et leurs complexes métalliques, ou un agent contenant l'un de ces composés.

3. Procédé selon la revendication 1, caractérisé en ce qu'on utilise un composé de formule I dans lequel $R^1$, $R^2$, $R^4$, $R^5$ et $R^6$ représentent l'hydrogène, $R^3$ est du chlore ou de l'hydrogène et le reste -A-Z représente un groupe $-CH_2-COOR^{16}$ ou $-CH(CH_3)-COOR^{16}$, où $R^{16}$ désigne un radical alkyle en $C_1$-$C_{12}$, alcényle en $C_3$-$C_6$, phényl-alkyle en $C_2$-$C_4$ ou phénoxy-alkyle en $C_1$-$C_4$.

4. Procédé selon la revendication 1, caractérisé en ce qu'on utilise de
l'ester isopropylique de l'acide 2-quinol-8-yloxy-acétique,
l'ester n-dodécylique de l'acide 2-(5-chloroquinol-8-yloxy)-acétique,
l'ester n-butylique de l'acide 2-(5-chloroquinol-8-yloxy)-acétique,
l'ester n-octylique de l'acide 2-(5-chloroquinol-8-yloxy)-acétique,
l'ester s-octylique de l'acide 2-(5-chloroquinol-8-yloxy)-thioacétique,
l'ester s-butylique de l'acide 2-quinol-8-yloxy-acétique,
l'ester 2-buténylique de l'acide 2-(5-chloroquinol-8-yloxy)-acétique,
l'ester méthallylique de l'acide 2-(5-chloroquinol-8-yloxy)-acétique,
l'ester 2-isopropyloxyéthylique de l'acide 2-(5-chloroquinol-8-yloxy)-acétique,
l'ester 2-phénoxyéthylique de l'acide 2-(5-chloroquinol-8-yloxy)-acétique,
l'ester 1-méthylbutylique de l'acide 2-(5-chloroquinol-8-yloxy)-acétique,
l'ester cyclohexylique de l'acide 2-(5-chloroquinol-8-yloxy)-acétique,
l'ester s-butylique de l'acide 2-(5-chloroquinol-8-yloxy)-acétique,
l'ester 2-méthylpentylique de l'acide 2-(5-chloroquinol-8-yloxy)-acétique,
l'ester n-butylique de l'acide 2-(5-chloroquinol-8-yloxy)-thioacétique,
l'ester 3,6-dioxadécylique de l'acide 2-(5-chloroquinol-8-yloxy)-acétique,
l'ester 3-méthoxybutylique de l'acide 2-(5-chloroquinol-8-yloxy)-acétique,
l' ester 1-éthylbutylique de l'acide 2-(5-chloroquinol-8-yloxy)-acétique,
l'ester 2-éthylbutylique de l'acide 2-(5-chloroquinol-8-yloxy)-acétique,
l'ester 1-méthylisopentylique de l'acide 2-(5-chloroquinol-8-yloxy)-acétique,
l'ester n-undécylique de l'acide 2-(5-chloroquinol-8-yloxy)-acétique,
l'ester 2-méthylbutylique de l'acide 2-(5-chloroquinol-8-yloxy)-acétique,
l'ester s-butylique de l'acide 2-(5-chloroquinol-8-yloxy)-thioacétique,
l'ester 3,6-dioxaheptylique de l'acide 2-(5-chloroquinol-8-yloxy)-acétique,
l'ester n-heptylique de l'acide 2-(5-chloroquinol-8-yloxy)-acétique,
l'ester n-dodécylique de l'acide 2-(5-chloroquinol-8-yloxy)-thioacétique,
l'ester n-décylique de l'acide 2-(5-chloroquinol-8-yloxy)-thioacétique,
l'ester 1-propylpropargylique de l'acide 2-(5-chloroquinol-8-yloxy)-acétique,
l'ester 1-méthylisobutylique de l'acide 2-(5-chloroquinol-8-yloxy)-acétique,
l'ester tert.butylique de l'acide 2-(5-chloroquinol-8-yloxy)-thioacétique,
l'ester néopentylique de l'acide 2-(5-chloroquinol-8-yloxy)-acétique,
l'ester n-propylique de l'acide 2-(5-chloroquinol-8-yloxy)-thioacétique,
l'ester 1-méthylhexylique de l'acide 2-(5-chloroquinol-8-yloxy)-acétique,
l'ester éthylique de l'acide 2-(5-chloroquinol-8-yloxy)-thioacétique,
l'ester 2-éthylhexylique de l'acide 2-(5-chloroquinol-8-yloxy)-acétique,
l'ester i-butylique de l'acide 2-(5-chloroquinol-8-yloxy)-thioacétique,
l'ester n-décylique de l'acide 2-quinol-8-yloxy-thioacétique,
l'ester 1-pentylique de l'acide 2-(5-chloroquinol-8-yloxy)-acétique,
l'ester 1-éthylpentylique de l'acide 2-(5-chloroquinol-8-yloxy)-acétique,
l'ester 1-propylbutylique de l'acide 2-(5-chloroquinol-8-yloxy)-acétique,
l'ester n-hexylique de l'acide 2-(5-chloroquinol-8-yloxy)-thioacétique,
l'ester n-hexylique de l'acide 2-(5-chloroquinol-8-yloxy)-acétique,
l'ester i-propylique de l'acide 2-(5-chloroquinol-8-yloxy)-thioacétique,
l'ester 1-pentallylique de l'acide 2-(5-chloroquinol-8-yloxy)-acétique,
l'ester 1-méthylpentylique de l'acide 2-(5-chloroquinol-8-yloxy)-acétique,
l'ester 1,1-diméthylpropargylique de l'acide 2-(5-chloroquinol-8-yloxy)-acétique,
l'ester 1-éthyl-1-méthylpropargylique de l'acide 2-(5-chloroquinol-8-yloxy)-acétique,
l'ester n-butyloxycarbonylméthylique de l'acide 2-(5-chloroquinol-8-yloxy)-acétique,

l'ester 1-n-butyloxycarbonyléthylique de l'acide 2-(5-chloroquinol-8-yloxy)-acétique,
l'ester 1-méthylisohexylique de l'acide 2-(5-chloroquinol-8-yloxy)-acétique,
l'ester 1-phénylisobutylique de l'acide 2-(5-chloroquinol-8-yloxy)-acétique,
l'ester 1-méthyl-2-(2-méthylphénoxy)-éthylique de l'acide 2-(5-chloroquinol-8-yloxy)-acétique,
l'ester 1-phényléthylique de l'acide 2-(5-chloroquinol-8-yloxy)-acétique,
l'ester 1-méthyl-2-(4-éthylphénoxy)-éthylique de l'acide 2-(5-chloroquinol-8-yloxy)-acétique,
l'ester 1-méthyl-2-phényléthylique de l'acide 2-(5-chloroquinol-8-yloxy)-acétique,
l'ester 1-méthyl-2-(2-isopropylphénoxy)-éthylique de 'lacide 2-(5-chloroquinol-8-yloxy)-acétique,
l'ester 1-phénylpropylique de l'acide 2-(5-chloroquinol-8-yloxy)-acétique,
l'ester 1-méthyl-2-(2-éthylphénoxy)-éthylique de l'acide 2-(5-chloroquinol-8-yloxy)-acétique,
l'ester 1-méthyl-2-(3-éthylphénoxy)-éthylique de l'acide 2-(5-chloroquinol-8-yloxy)-acétique,
l'ester 1-éthyl-2-phénoxyéthylique de l'acide 2-(5-chloroquinol-8-yloxy)-acétique,
l'ester 1-méthyl-3-phénylpropylique de l'acide 2-(5-chloroquinol-8-yloxy)-acétique,
l'ester 1-méthyl-2-(3-méthylphénoxy)-éthylique de l'acide 2-(5-chloroquinol-8-yloxy)-acétique,
l'ester 1-méthyl-2-(4-isopropylphénoxy)-éthylique de l'acide 2-(5-chloroquinol-8-yloxy)-acétique ou
l'ester 1-méthyl-2-(4-méthylphénoxy)-éthylique de l'acide 2-(5-chloroquinol-8-yloxy)-acétique
ou un agent contenant l'un de ces composés.

5. Procédé selon la revendication 1, caractérisé en ce qu'on utilise :
l'ester méthallylique de l'acide 2-(5-chloroquinol-8-yloxy)-acétique,
l'ester 2-phénoxyéthylique de l'acide 2-(5-chloroquinol-8-yloxy)-acétique,
l'ester 1-méthylbutylique de l'acide 2-(5-chloroquinol-8-yloxy)-acétique,
l'ester 1-éthylbutylique de l'acide 2-(5-chloroquinol-8-yloxy)-acétique,
l'ester 1-méthylisopentylique de l'acide 2-(5-chloroquinol-8-yloxy)-acétique,
l'ester 1-méthylhexylique de l'acide 2-(5-chloroquinol-8-yloxy)-acétique,
l'ester 1-éthylpentylique de l'acide 2-(5-chloroquinol-8-yloxy)-acétique,
l'ester 1-propylbutylique de l'acide 2-(5-chloroquinol-8-yloxy)-acétique,
l'ester 1-pentylallylique de l'acide 2-(5-chloroquinol-8-yloxy)-acétique, l'ester 1-méthylpentylique de l'acide 2-(5-chloroquinol-8-yloxy)-acétique,
l'ester 1-méthylisohexylique de l'acide 2-(5-chloroquinol-8-yloxy)-acétique,
l'ester 1-phénylisobutylique de l'acide 2-(5-chloroquinol-8-yloxy)-acétique,
l'ester 1-phényléthylique de l'acide 2-(5-chloroquinol-8-yloxy)-acétique,
l'ester 1-méthyl-2-(4-éthylphénoxy)-éthylique de l'acide 2-(5-chloroquinol-8-yloxy)-acétique,
l'ester 1-méthyl-2-phényléthylique de l'acide 2-(5-chloroquinol-8-yloxy)-acétique,
l'ester 1-phénylpropylique de l'acide 2-(5-chloroquinol-8-yloxy)-acétique,
l'ester 1-méthyl-2-phénoxyéthylique de l'acide 2-(5-chloroquinol-8 yloxy)-acétique,
l'ester 1-méthyl-3-phénylpropylique de l'acide 2-(5-chloroquinol-8-yloxy)-acétique,
l'ester 1-méthyl-2-(4-méthylphénoxy)-éthylique de l'acide 2-(5-chloroquinol-8-yloxy)-acétique ou
un agent contenant un de ces composés.

6. Procédé selon la revendication 1, caractérisé en ce qu'on utilise l'ester méthallylique de l'acide 2-(5-chloroquinol-8-yloxy)-acétique, l'ester 2-phénoxyéthylique de l'acide 2-(5-chloroquinol-8-yloxy)-acétique, l'ester 1-méthylisopentylique de l'acide 2-(5-chloroquinol-8-yloxy)-acétique ou l'ester 1-méthylhexylique de l'acide 2-(5-chloroquinol-8-yloxy)-acétique ou un agent contenant un de ces composés.

7. Procédé selon la revendication 1, caractérisé en ce qu'on utilise l'ester 1-méthylisopentylique de l'acide 2-(5-chloroquinol-8-yloxy)-acétique ou un agent contenant ce composé.

8. Procédé selon la revendication 1, caractérisé en ce qu'on utilise l'ester méthallylique de l'acide 2-(5-chloroquinol-8-yloxy)-acétique ou un agent contenant ce composé.

9. Procédé selon la revendication 1, caractérisé en ce qu'on utilise l'ester 2-phénoxyéthylique de l'acide 2-(5-chloroquinol-8-yloxy)-acétique ou un agent contenant ce composé.

10. Procédé selon la revendication 1, caractérisé en ce qu'on utilise l'ester 1-méthylhexylique de l'acide 2-(5-chloroquinol-8-yloxy)-acétique ou un agent contenant ce composé.

11. Procédé selon la revendication 1 pour la protection de plantes cultivables contre les actions nuisibles

d'herbicides de formule II, où Y représente les groupes -O-$R^{18}$, -S-$R^{18}$, ou -O-N = $CR^{19}R^{20}$, tandis que $R^{18}$ représnte l'hydrogène ou un radical alkyle en $C_1$-$C_4$, alcényle en $C_3$-$C_4$, alcynyle en $C_3$-$C_4$ ou un radical alkyle en $C_1$-$C_4$ substitué par un radical alcoxycarbonyle en $C_1$-$C_4$ ou par un radical di-alkyl-$C_1$-$C_4$)amino et
$R^{19}$ et $R^{20}$ représentent, indépendamment l'un de l'autre, un radical alkyle en $C_1$-$C_4$ ou
$R^{19}$ et $R^{20}$ représentent ensemble une chaîne alkylène en $c_4$-$c_7$.

**12.** Procédé selon la revendication 11, caractérisé en ce que Y représente un radical méthoxy, éthoxy, propyloxy, isopropyloxy, butyloxy, diméthylaminoéthoxy, propargyloxy, 1-cyano-1-méthyléthoxy, méthoxycarbonylméthylthio, 1-éthoxycarbonyléthoxy, butyloxycarbonyle, -O-N = $C(CH_3)_2$, -O-N = $C(CH_3)$-$C_2H_5$ ou -O-N = $C(CH_2)_5$.

**13.** Procédé selon la revendication 1 pour la protection des plantes cultivables contre les actions nuisibles de l'ester méthylique de l'acide 2-[4-(5-chloro-3-fluoropyridin-2-yloxy)-phénoxy]-propionique.

**14.** Procédé selon la revendication 1 pour la protection des plantes cultivables contre les actions nuisibles de l'ester propargylique de l'acide 2-[4-(5-chloro-3-fluoropyridin-2-yloxy)-phénoxy]-propionique.

**15.** Procédé selon la revendication 1 pour la protection des plantes cultivables contre les actions nuisibles de l'ester L-méthoxycarbonylméthylique de l'acide 2-[4-(5-chloro-3-fluoropyridin-2-yloxy)-phénoxyl]-thiopropionique.

**16.** Procédé selon la revendication 1 pour la protection des plantes cultivables contre les actions nuisibles de l'ester 1-éthoxycarbonyléthylique de l'acide 2-[4-(5-chloro-3-fluoropyridin-2-yloxy)-phénoxy]-propionique.

**17.** Procédé selon la revendication 1 pour la protection des plantes cultivables contre les actions nuisibles de l'ester méthylique de l'acide 2-[4-(5-chloro-3-fluoropyridin-2-yloxy)-phénoxy]-propionique, l'ester propargylique de l'acide 2-[4-(5-chloro-3-fluoropyridin-2-yloxy)-phénoxy]-propionique, l'ester L-méthoxycarbonylméthylique de l'acide 2-[4-(5-chloro-3-fluoropyridin-2-yloxy)-phénoxy]-thiopropionique ou l'ester 1-éthoxycarbonyléthylique de l'acide 2-[4-(5-chloro-3-fluoropyridin-2-yloxy)-phénoxy]-propionique, caractérisé en ce qu'on traite les plantes cultivables, des parties de ces plantes ou des sols destinés à la culture des plantes cultivables avec de
l'ester isopropylique de l'acide 2-quinol-8-yloxy-acétique,
l'ester n-dodécylique de l'acide 2-(5-chloroquinol-8-yloxy)-acétique,
l'ester n-butylique de l'acide 2-(5-chloroquinol-8-yloxy)-acétique,
l'ester n-octylique de l'acide 2-(5-chloroquinol-8-yloxy)-acétique,
l'ester s-butylique de l'acide 2-quinol-8-yloxy-acétique,
l'ester n-octylique de l'acide 2-(5-chloroquinol-8-yloxy)-thioacétique,
l'ester 2-buténylique de l'acide 2-(5-chloroquinol-8-yloxy)-acétique,
l'ester méthallylique de l'acide 2-(5-chloroquinol-8-yloxy)-acétique,
l'ester 2-isopropyloxyéthylique de l'acide 2-(5-chloroquinol-8-yloxy)-acétique,
l'ester 2-phénoxyéthylique de l'acide 2-(5-chloroquinol-8-yloxy)-acétique,
l'ester 1-méthylbutylique de l'acide 2-(5-chloroquinol-8-yloxy)-acétique,
l'ester cyclohexylique de l'acide 2-(5-chloroquinol-8-yloxy)-acétique,
l'ester s-butylique de l'acide 2-(5-chloroquinol-8-yloxy)-acétique,
l'ester 2-méthylpentylique de l'acide 2-(5-chloroquinol-8-yloxy)-acétique,
l'ester n-butylique de l'acide 2-(5-chloroquinol-8-yloxy)-thioacétique,
l'ester 3,6-dioxadécylique de l'acide 2-(5-chloroquinol-8-yloxy)-acétique,
l'ester 3-méthoxybutylique de l'acide 2-(5-chloroquinol-8-yloxy)-acétique,
l'ester 1-éthylbutylique de l'acide 2-(5-chloroquinol-8-yloxy)-acétique,
l'ester 2-éthylbutylique de l'acide 2-(5-chloroquinol-8-yloxy)-acétique,
l'ester 1-méthylisopentylique de l'acide 2-(5-chloroquinol-8-yloxy)-acétique,
l'ester n-undécylique de l'acide 2-(5-chloroquinol-8-yloxy)-acétique,
l'ester 2-méthylbutylique de l'acide 2-(5-chloroquinol-8-yloxy)-acétique,
l'ester s-butylique de l'acide 2-(5-chloroquinol-8-yloxy)-thioacétique,
l'ester 3,6-dioxaheptylique de l'acide 2-(5-chloroquinol-8-yloxy)-acétique,
l'ester n-heptylique de l'acide 2-(5-chloroquinol-8-yloxy)-acétique,

l'ester n-dodécylique de l'acide 2-(5-chloroquinol-8-yloxy)-thioacétique,
l'ester n-décylique de l'acide 2-(5-chloroquinol-8-yloxy)-thioacétique,
l'ester 1-propylpropargylique de l'acide 2-(5-chloroquinol-8-yloxy)-acétique,
l'ester 1-méthylisobutylique de l'acide 2-(5-chloroquinol-8-yloxy)-acétique,
l'ester tert.butylique de l'acide 2-(5-chloroquinol-8-yloxy)-thioacétique,
l'ester néopentylique de l'acide 2-(5-chloroquinol-8-yloxy)-acétique,
l'ester n-propylique de l'acide 2-(5-chloroquinol-8-yloxy)-thioacétique,
l'ester 1-méthylhexylique de l'acide 2-(5-chloroquinol-8-yloxy)-acétique,
l'ester éthylique de l'acide 2-(5-chloroquinol-8-yloxy)-thioacétique,
l'ester 2-éthylhexylique de l'acide 2-(5-chloroquinol-8-yloxy)-acétique,
l'ester i-butylique de l'acide 2-(5-chloroquinol-8-yloxy)-thioacétique,
l'ester n-décylique de l'acide 2-quinol-yloxy-thioacétique,
l'ester i-pentylique de l'acide 2-(5-chloroquinol-8-yloxy)-acétique,
l'ester 1-éthylpentylique de l'acide 2-(5-chloroquinol-8-yloxy)-acétique,
l'ester 1-propylbutylique de l'acide 2-(5-chloroquinol-8-yloxy)-acétique,
l'ester n-hexylique de l'acide 2-(5-chloroquinol-8-yloxy)-thioacétique,
l'ester n-hexylique de l'acide 2-(5-chloroquinol-8-yloxy)-acétique,
l'ester i-propylique de l'acide 2-(5-chloroquinol-8-yloxy)-thioacétique,
l'ester 1-pentylallylique de l'acide 2-(5-chloroquinol-8-yloxy)-acétique,
l'ester 1-méthylpentylique de l'acide 2-(5-chloroquinol-8-yloxy)-acétique,
l'ester 1,1-diméthylpropargylique de l'acide 2-(5-chloroquinol-8-yloxy)-acétique,
l'ester 1-éthyl-1-méthylpropargylique de l'acide 2-(5-chloroquinol-8-yloxy)-acétique,
l'ester n-butyloxycarbonylméthylique de l'acide 2-(5-chloroquinol-8-yloxy)-acétique,
l'ester 1-n-butyloxycarbonyléthylique de l'acide 2-(5-chloroquinol-8-yloxy)-acétique,
l'ester 1-méthylisohexylique de l'acide 2-(5-chloroquinol-8-yloxy)-acétique,
l'ester 1-phénylisobutylique de l'acide 2-(5-chloroquinol-8-yloxy)-acétique,
l'ester 1-méthyl-2-(2-méthylphénoxy)-éthylique de l'acide 2-(5-chloroquinol-8-yloxy)-acétique,
l'ester 1-phényléthylique de l'acide 2-(5-chloroquinol-8-yloxy)-acétique,
l'ester 1-méthyl-2-(4-éthylphénoxy)-éthylique de l'acide 2-(5-chloroquinol-8-yloxy)-acétique,
l'ester 1-méthyl-2-phényléthylique de l'acide 2-(5-chloroquinol)-8-yloxy)-acétique,
l'ester 1-méthyl-2-(2-isopropylphénoxy)-éthylique de l'acide 2-(5-chloroquinol-8-yloxy)-acétique,
l'ester 1-phénylpropylique de l'acide 2-(5-chloroquinol-8-yloxy)-acétique,
l'ester 1-méthyl-2-(2-éthylphénoxy)-éthylique de l'acide 2-(5-chloroquinol-8-yloxy)-acétique,
l'ester 1-méthyl-2-(3-éthylphénoxy)-éthylique de l'acide 2-(5-chloroquinol-8-yloxy)-acétique,
l'ester 1-méthyl-2-phénoxyéthylique de l'acide 2-(5-chloroquinol-8-yloxy)-acétique,
l'ester 1-méthyl-3-phénylpropylique de l'acide 2-(5-chloroquinol-8-yloxy)-acétique,
l'ester 1-méthyl-2-(3-méthylphénoxy)-éthylique de l'acide 2-(5-chloroquinol-8-yloxy)-acétique,
l'ester 1-méthyl-2-(4-isopropylphénoxy)-éthylique de l'acide 2-(5-chloroquinol-8-yloxy)-acétique ou
l'ester 1-méthyl-2-(4-méthylphénoxy)-éthylique de l'acide 2-(5-chloroquinol-8-yloxy)-acétique
ou un agent contenant un de ces composés.

**18.** Procédé selon la revendication 17, caractérisé en ce qu'on traite les plantes cultivables, des parties de ces plantes ou des sols destinés à la culture des plantes cultivables avec de
l'ester méthallylique de l'acide 2-(5-chloroquinol-8-yloxy)-acétique,
l'ester 2-phénoxyéhtylique de l'acide 2-(5-chloroquinol-8-yloxy)-acétique,
l'ester 1-méthylbutylique de l'acide 2-(5-chloroquinol-8 yloxy)-acétique,
l'ester 1-éthylbutylique de l'acide 2-(5-chloroquinol-8-yloxy)-acétique,
l'ester 1-méthylisopentylique de l'acide 2-(5-chloroquinol-8-yloxy)-acétique,
l'ester 1-méthylhexylique de l'acide 2-(5-chloroquinol-8-yloxy)-acétique,
l'ester 1-éthylpentylique de l'acide 2-(5-chloroquinol-8-yloxy)-acétique,
l'ester 1-propylbutylique de l'acide 2-(5-chloroquinol-8-yloxy)-acétique,
l'ester 1-pentylallylique de l'acide 2-(5-chloroquinol-8-yloxy)-acétique,
l'ester 1-méthylpentylique de l'acide 2-(5-chloroquinol-8-yloxy)-acétique,
l'ester 1-méthylisohexylique de l'acide 2-(5-chloroquinol-8-yloxy)-acétique,
l'ester 1-phénylisobutylique de l'acide 2-(5-chloroquinol-8-yloxy)-acétique,
l'ester 1-phényléthylique de l'acide 2-(5-chloroquinol-8-yloxy)-acétique,
l'ester 1-méthyl-2-(4-éthylphénoxy)-éthylique de l'acide 2-(5-chloroquinol-8-yloxy)-acétique,
l'ester 1-méthyl-2-phényléthylique de l'acide 2-(5-chloroquinol-8-yloxy)-acétique,

l'ester 1-phénylpropylique de l'acide 2-(5-chloroquinol-86yloxy)-acétique,
l'ester 1-méthyl-2-phénoxyéthylique de l'acide 2-(5-chloroquinol-8-yloxy)-acétique,
l'ester 1-méthyl-3-phénylpropylique de l'acide 2-(5-chloroquinol-8-yloxy)-acétique ou l'ester 1-méthyl-2-(4-méthylphényl)-éthylique de l'acide 2-(5-chloroquinol-8-yloxy)-acétique ou
un agent contenant un de ces composés.

**19.** Procédé selon la revendication 17, caractérisé en ce qu'on traite les plantes cultivables, des parties de ces plantes ou des sols destinés à la culture des plantes cultivables avec l'ester 1-méthylisopentylique de l'acide 2-(5-chloroquinol-8-yloxy)-acétique, l'ester 2-phénoxyéthylique de l'acide 2-(5-chloroquinol-8-yloxy)-acétique, l'ester 1-méthylhexylique de l'acide 2-(5-chloroquinol-8-yloxy)-acétique ou l'ester méthallylique de l'acide 2-(5-chloroquinol-8-yloxy)-acétique ou un agent contenant un de ces composés.

**20.** Procédé selon la revendication 1 pour la protection des céréales.

**21.** Procédé selon la revendication 20 pour la protection du froment, de l'orge, du seigle et du riz.

**22.** Procédé selon la revendication 1, caractérisé en ce qu'on traite des stocks de plantes cultivables ou des surfaces cultivables pour plantes cultivées avec 0,01 à 10 kg/ha d'un composé de formule I selon la revendication 1.

**23.** Procédé selon la revendication 22, caractérisé en ce qu'on traite des plantes cultivables ou des surfaces cultivables pour plantes cultivées avec 0,05 à 0,5 kg/ha d'un composé de formule I selon la revendication 1.

**24.** Procédé selon la revendication 1, caractérisé en ce qu'on traite des semences des plantes cultivables avec un composé de formule I selon la revendication 1.

**25.** Procédé selon la revendication 24, caractérisé en ce qu'on traite des semences de la plante cultivable avec 0,01 à 10 g/kg de semences d'un composé de formule I selon la revendication 1.

**26.** Procédé selon la revendication 25, caractérisé en ce qu'on traite des semences des plantes cultivables avec 0,05 à 2 g/kg de semences d'un composé de formule I selon la revendication 1.

**27.** Procédé selon la revendication 24 pour la protection des plantes cultivables contre les actions nuisibles de l'ester méthylique de l'acide 2-[4-(5-chloro-3-fluoropyridin-2-yloxy)-phénoxy]-propionique, de l'ester propargylique de l'acide 2-[4-(5-chloro-3-fluoropyridin-2-yloxy)-phénoxy]-propionique, de l'ester L-méthoxycarbonylméthylique de l'acide 2-[4-(5-chloro-3-fluoropyridin-2-yloxy)-phénoxy]-thiopropionique ou de l'ester 1-éthoxycarbonyléthylique de l'acide 2-[4-(5-chloro-3-fluoropyridin-2-yloxy)-phénoxy]-propionique, caractérisé en ce qu'on traite les semences des plantes cultivables avec de
l'ester isopropylique de l'acide 2-quinol-8-yloxy-acétique,
l'ester n-dodécylique de l'acide 2-(5-chloroquinol-8-yloxy)-acétique,
l'ester n-butylique de l'acide 2-(5-chloroquinol-8-yloxy)-acétique,
l'ester n-octylique de l'acide 2-(5-chloroquinol-8-yloxy)-acétique,
l'ester s-butylique de l'acide 2-quinol-8-yloxy-acétique,
l'ester n-octylique de l'acide 2-(5-chloroquinol-8-yloxy)-thioacétique,
l'ester 2-buténylique de l'acide 2-(5-chloroquinol-8-yloxy)-acétique,
l'ester méthallylique de l'acide 2-(5-chloroquinol-8-yloxy)-acétique,
l'ester 2-isopropyloxyéthylique de l'acide 2-(5-chloroquinol-8-yloxy)acétique,
l'ester 2-phénoxyéthylique de l'acide 2-(5-chloroquinol-8-yloxy)-acétique,
l'ester 1-méthylbutylique de l'acide 2-(5-chloroquinol-8-yloxy)-acétique,
l'ester cyclohexylique de l'acide 2-(5-chloroquinol-8-yloxy)-acétique,
l'ester s-butylique de l'acide 2-(5-chloroquinol-8-yloxy)-acétique,
l'ester 2-méthylpentylique de l'acide 2-(5-chloroquinol-8-yloxy)-acétique,
l'ester n-butylique de l'acide 2-(5-chloroquinol-8-yloxy)-thioacétique,
l'ester 3,6-dioxadécylique de l'acide 2-(5-chloroquinol-8-yloxy)-acétique,
l'ester 3-méthoxybutylique de l'acide 2-(5-chloroquinol-8-yloxy)-acétique,
l'ester 1-éthylbutylique de l'acide 2-(5-chloroquinol-8-yloxy)-acétique,
l'ester 2-éthylbutylique de l'acide 2-(5-chloroquinol-8-yloxy)-acétique,

l'ester 1-méthylisopentylique de l'acide 2-(5-chloroquinol-8-yloxy)-acétique,
l'ester n-undécylique de l'acide 2-(5-chloroquinol-8-yloxy)-acétique,
l'ester 2-méthylbutylique de l'acide 2-(5-chloroquinol-8-yloxy)-acétique,
l'ester s-butylique de l'acide 2-(5-chloroquinol-8-yloxy)-thioacétique,
l'ester 3,6-dioxaheptylique de l'acide 2-(5-chloroquinol-8-yloxy)-acétique,
l'ester n-heptylique de l'acide 2-(5-chloroquinol-8-yloxy)-acétique,
l'ester n-dodécylique de l'acide 2-(5-chloroquinol-8-yloxy)-thioacétique,
l'ester n-décylique de l'acide 2-(5-chloroquinol-8-yloxy)-thioacétique,
l'ester 1-propylpropargylique de l'acide 2-(5-chloroquinol-8-yloxy)-acétique,
l'ester 1-méthylisobutylique de l'acide 2-(5-chloroquinol-8-yloxy)-acétique,
l'ester tert.butylique de l'acide 2-(5-chloroquinol-8-yloxy)-thioacétique,
l'ester néopentylique de l'acide 2-(5-chloroquinol-8-yloxy)-acétique,
l'ester n-propylique de l'acide 2-(5-chloroquinol-8-yloxy)-thioacétique,
l'ester 1-méthylhexylique de l'acide 2-(5-chloroquinol-8-yloxy)-acétique,
l'ester éthylique de l'acide 2-(5-chloroquinol-8-yloxy)-thioacétique,
l'ester 2-éthylhexylique de l'acide 2-(5-chloroquinol-8-yloxy)-acétique,
l'ester i-butylique de l'acide 2-(5-chloroquinol-8-yloxy)-thioacétique,
l'ester n-décylique de l'acide 2-quinol-8-yloxy-thioacétique,
l'ester i-pentylique de l'acide 2-(5-chloroquinol-8-yloxy)-acétique,
l'ester 1-éthylpentylique de l'acide 2-(5-chloroquinol-8-yloxy)-acétique,
l'ester 1-propylbutylique de l'acide 2-(5-chloroquinol-8-yloxy)-acétique,
l'ester n-hexylique de l'acide 2-(5-chloroquinol-8-yloxy)-thioacétique,
l'ester n-hexylique de l'acide 2-(5-chloroquinol-8-yloxy)-acétique,
l'ester i-propylique de l'acide 2-(5-chloroquinol-8-yloxy)-thioacétique,
1'ester 1-pentylallylique de l'acide 2-(5-chloroquinol-8-yloxy)-acétique,
l'ester 1-méthylpentylique de l'acide 2-(5-chloroquinol-8-yloxy)-acétique,
l'ester 1,1-diméthylpropargylique de l'acide 2-(5-chloroquinol-8-yloxy)-acétique,
l'ester 1-éthyl-1-méthylpropargylique de l'acide 2-(5-chloroquinol-8-yloxy)-acétique,
l'ester n-butyloxycarbonylméthylique de l'acide 2-(5-chloroquinol-8-yloxy)-acétique,
l'ester 1-n-butyloxycarbonyléthylique de l'acide 2-(5-chloroquinol-8-yloxy)-acétique,
l'ester 1-méthylisohexylique de l'acide 2-(5-chloroquinol-8-yloxy)-acétique,
l'ester 1-phénylisobutylique de l'acide 2-(5-chloroquinol-8-yloxy)-acétique,
l'ester 1-méthyl-2-méthylphénoxy)-éthylique de l'acide 2-(5-chloroquinol-8-yloxy)-acétique,
l'ester 1-phényléthylique de l'acide 2-(5-chloroquinol-8-yloxy)-acétique,
l'ester 1-méthyl-2-(4-éthylphénoxy)-éthylique de l'acide 2-(5-chloroquinol-8-yloxy)-acétique,
l'ester 1-éthyl-2-phényléthylique de l'acide 2-(5-chloroquinol-8-yloxy)-acétique,
l'ester 1-méthyl-2-(2-isopropylphénoxy)-éthylique de l'acide 2-(5-chloroquinol-8-yloxy)-acétique,
l'ester 1-phénylpropylique de l'acide 2-(5-chloroquinol-8-yloxy)-acétique,
l'ester 1-méthyl-2-(2-éthylphénoxy)-éthylique de l'acide 2-(5-chloroquinol-8-yloxy)-acétique,
l'ester 1-méthyl-2-(3-éthylphénoxy)-éthylique de l'acide 2-(56chloroquinol-8-yloxy)-acétique,
l'ester 1-méthyl-2-phénoxyéthylique de l'acide 2-(5-chloroquinol-8-yloxy)-acétique,
l'ester 1-méthyl-3-phénylpropylique de l'acide 2-(5-chloroquinol-8-yloxy)-acétique,
l'ester 1-méthyl-2-(3-méthylphénoxy)-éthylique de l'acide 2-(5-chloroquinol-8-yloxy)-acétique,
l'ester 1-méthyl-2-(4-isopropylphenoxy)-éthylique de l'acide 2-(5-chloroquinol-8-yloxy)-acétique ou
l'ester 1-méthyl-2-(4-méthylphénoxy)-éthylique de l'acide 2-(5-chloroquinol-8-yloxy)-acétique ou
un agent contenant un de ces composés.

**28.** Procédé selon la revendication 27, caractérisé en ce que l'on traite les semences des plantes cultivables avec de l'ester méthallylique de l'acide 2-(5-chloroquinol-8-yloxy) acétique,
l'ester 2-phénoxyéthylique de l'acide 2-(5-chloroquinol-8-yloxy)-acétique,
l'ester 1-méthylbutylique de l'acide 2-(5-chloroquinol-8-yloxy)-acétique,
l'ester 1-éthylbutylique de l'acide 2-(5-chloroquinol-8-yloxy)-acétique,
l'ester 1-méthylisopentylique de l'acide 2-(5-chloroquinol-8-yloxy)-acétique,
l'ester 1-méthylhexylique de l'acide 2-(5-chloroquinol-8-yloxy)-acétique,
l'ester 1-éthylpentylique de l'acide 2-(5-chloroquinol-8-yloxy)-acétique,
l'ester 1-propylbutylique de l'acide 2-(5-chloroquinol-8-yloxy)-acétique,
l'ester 1-pentylallylique de l'acide 2-(5-chloroquinol-8-yloxy)-acétique,
l'ester 1-méthylpentylique de l'acide 2-(5-chloroquinol-8-yloxy)-acétique, l'ester 1-méthylisohexylique

de l'acide 2-(5-chloroquinol-8-yloxy)-acétique,
l'ester 1-phénylisobutylique de l'acide 2-(5-chloroquinol-8-yloxy)-acétique,
l'ester 1-phényléthylique de l'acide 2-(5-chloroquinol-8-yloxy)-acétique,
l'ester 1-méthyl-2-phényléthylique de l'acide 2-(5-chloroquinol-8-yloxy)-acétique,
l'ester 1-méthyl-2-(4-éthylphénoxy)-éthylique de l'acide 2-(5-chloroquinol-8-yloxy)-acétique,
l'ester 1-phénylpropylique de l'acide 2-(5-chloroquinol-8-yloxy)-acétique,
l'ester 1-méthyl-2-phénoxyéthylique de l'acide 2-(5-chloroquinol-8-yloxy)-acétique,
l'ester 1-méthyl-3-phénylpropylique de l'acide 2-(5-chloroquinol-8-yloxy)-acétique ou
l'ester 1-méthyl-2-(4-méthylphénoxy)-éthylique de l'acide 2-(5-chloroquinol-8-yloxy)-acétique ou
un agent contenant un de ces composés.

**29.** Procédé selon la revendication 27, caractérisé en ce qu'on traite les semences des plantes cultivables avec de l'ester 1-méthylisopentylique de l'acide 2-(5-chloroquinol-8-yloxy)-acétique, de l'ester 2-phénoxyéthylique de l'acide 2-(5-chloroquinol-8-yloxy)-acétique, de l'ester 1-méthylhexylique de l'acide 2-(5-chloroquinol-8-yloxy)-acétique ou de l'ester méthallylique de l'acide 2-(5-chloroquinol-8-yloxy)-acétique ou un agent contenant un de ces composés.

**30.** Procédé selon la revendication 27 pour la protection des céréales.

**31.** Utilisation de composés de formule I selon la revendication 1 pour la protection de plantes cultivables contre les actions nuisibles de composés de formule II selon la revendication 1.

**32.** Procédé pour la lutte sélective contre les mauvaises herbes dans des plantes cultivables, caractérisé en ce qu'on traite les plantes cultivables, des parties des plantes cultivables ou des surfaces ou des surfaces cultivables pour plantes cultivées avec un antidote de formule I et un herbicide de formule II selon la revendication 1.

**33.** Agent herbicide, caractérisé en ce qu'il contient, outre un agent à activité herbicide de formule II selon la revendication 1, un antidote de formule I selon la revendication 1.

**34.** Agent selon la revendication 33, caractérisé en ce que, outre un herbicide choisi parmi l'ester méthylique de l'acide 2-[4-(5-chloro-3-fluoropyridin-2-yloxy)-phénoxy]-propionique, l'ester propargylique de l'acide 2-[4-(5-chloro-3-fluoropyridin-2-yloxy)-phénoxy]-propionique, l'ester L-méthoxycarbonylméthylique de l'acide 2-[4(5-chloro-3-fluoropyridin-2-yloxy)-phénoxy]-thiopropionique ou l'ester 1-éthoxycarbonyléthylique de l'acide 2-[4-(5-chloro-3-fluoropyridin-2-yloxy)-phénoxy]-propionique, il contient un antidote de la série
ester isopropylique de l'acide 2-quinol-8-yloxy-acétique,
ester n-dodécylique de l'acide 2-(5-chloroquinol-8-yloxy)-acétique,
ester n-butylique de l'acide 2-(5-chloroquinol-8-yloxy)-acétique,
ester n-octylique de l'acide 2-(5-chloroquinol-8-yloxy)-acétique,
ester s-butylique de l'acide 2-quinol-8-yloxy-acétique,
ester n-octylique de l'acide 2-(5-chloroquinol-8-yloxy)-thioacétique,
ester 2-buténylique de l'acide 2-(5-chloroquinol-8-yloxy)-acétique,
ester méthallylique de l'acide 2-(5-chloroquinol-8-yloxy)-acétique,
ester 2-isopropyloxyéthylique de l'acide 2-(5-chloroquinol-8-yloxy)-acétique,
ester 2-phénoxyéthylique de l'acide 2-(5-chloroquinol-8-yloxy)-acétique,
ester 1-éthylbutylique de l'acide 2-(5-chloroquinol-8-yloxy)-acétique,
ester cyclohexylique de l'acide 2-(5-chloroquinol-8-yloxy)-acétique,
ester s-butylique de l'acide 2-(5-chloroquinol-8-yloxy)-acétique,
ester 2-méthylpentylique de l'acide 2-(5-chloroquinol-8-yloxy)-acétique,
ester n-butylique de l'acide 2-(5-chloroquinol-8-yloxy)-thioacétique,
ester 3,6-dioxadécylique de l'acide 2-(5-chloroquinol-8-yloxy)-acétique,
ester 3-méthoxybutylique de l'acide 2-(5-chloroquinol-8-yloxy)-acétique,
ester 1-éthylbutylique de l'acide 2-(5-chloroquinol-8-yloxy)-acétique,
ester 2-éthylbutylique de l'acide 2-(5-chloroquinol-8-yloxy)-acétique,
ester 1-méthylisopentylique de l'acide 2-(5-chloroquinol-8-yloxy)-acétique,
ester n-undécylique de l'acide 2-(5-chloroquinol-8-yloxy)-acétique,
ester 2-méthylbutylique de l'acide 2-(5-chloroquinol-8-yloxy)-acétique,

ester s-butylique de l'acide 2-(5-chloroquinol-8-yloxy)-thioacétique,
ester 3,6-dioxaheptylique de l'acide 2-(5-chloroquinol-8-yloxy)-acétique,
ester n-heptylique de l'acide 2-(5-chloroquinol-8-yloxy)-acétique,
ester n-dodécylique de l'acide 2-(5-chloroquinol-8-yloxy)-thioacétique,
ester n-décylique de l'acide 2-(5-chloroquinol-8-yloxy)-thioacétique,
ester 1-propylpropargylique de l'acide 2-(5-chloroquinol-8-yloxy)-acétique,
ester 1-méthylisobutylique de l'acide 2-(5-chloroquinol-8-yloxy)-acétique,
ester tert.butylique de l'acide 2-(5-chloroquinol-8-yloxy)-thioacétique,
ester néopentylique de l'acide 2-(5-chloroquinol-8-yloxy)-acétique,
ester n-propylique de l'acide 2-(5-chloroquinol-8-yloxy)-thioacétique,
ester 1-méthylhexylique de l'acide 2-(5-chloroquinol-8-yloxy)-acétique,
ester éthylique de l'acide 2-(5-chloroquinol-8-yloxy)-thioacétique,
ester 2-éthylhexylique de l'acide 2-(5-chloroquinol-8-yloxy)-acétique,
ester i-butylique de l'acide 2-(5-chloroquinol-8-yloxy)-thioacétique,
ester n-décylique de l'acide 2-quinol-8-yloxy-thioacétique,
ester i-pentylique de l'acide 2-(5-chloroquinol-8-yloxy)-acétique,
ester 1-éthylpentylique de l'acide 2-(5-chloroquinol-8-yloxy)-acétique,
ester 1-propylbutylique de l'acide 2-(5-chloroquinol-8-yloxy)-acétique,
ester n-hexylique de l'acide 2-(5-chloroquinol-8-yloxy)-thioacétique,
ester n-hexylique de l'acide 2-(5-chloroquinol-8-yloxy)-acétique,
ester i-propylique de l'acide 2-(5-chloroquinol-8-yloxy)-thioacétique,
ester 1-pentylallylique de l'acide 2-(5-chloroquinol-8-yloxy)-acétique,
ester 1-méthylpentylique de l'acide 2-(5-chloroquinol-8-yloxy)-acétique,
ester 1,1-diméthylpropargylique de l'acide 2-(5-chloroquinol-8-yloxy)-acétique,
ester 1-éthyl-1-méthylpropargylique de l'acide 2-(5-chloroquinol-8-yloxy)-acétique,
ester n-butyloxycarbonylméthylique de l'acide 2-(5-chloroquinol-8-yloxy)-acétique,
ester 1-n-butyloxycarbonyléthylique de l'acide 2-(5-chloroquinol-8-yloxy)-acétique,
ester 1-méthylisohexylique de l'acide 2-(5-chloroquinol-8-yloxy)-acétique,
ester 1-phénylisobutylique de l'acide 2-(5-chloroquinol-8-yloxy)-acétique,
ester 1-méthyl-2-(2-méthylphénoxy)-éthylique de l'acide 2-(5-chloroquinol-8-yloxy)-acétique,
ester 1-phényléthylique de l'acide 2-(5-chloroquinol-8-yloxy)-acétique,
ester 1-méthyl-2-(4-éthylphénoxy)-éthylique de l'acide 2-(5-chloroquinol-8-yloxy)-acétique,
ester 1-méthyl-2-phényléthylique de l'acide 2-(5-chloroquinol-8-yloxy)-acétique,
ester 1-méthyl-2-(2-isopropylphénoxy)-éthylique de l'acide 2-(5-chloroquinol-8-yloxy)-acétique,
ester 1-phénylpropylique de l'acide 2-(5-chloroquinol-8-yloxy)-acétique,
ester 1-méthyl-2-(2-éthylphénoxy)-éthylique de l'acide 2-(5-chloroquinol-8-yloxy)-acétique,
ester 1-méthyl-2-(3-éthylphénoxy)-éthylique de l'acide 2-(5-chloroquinol-8-yloxy)-acétique,
ester 1-méthyl-2-phénoxyéthylique de l'acide 2-(5-chloroquinol-8-yloxy)-acétique,
ester 1-méthyl-3-phénylpropylique de l'acide 2-(5-chlrooquinol-8-yloxy)-acétique,
ester 1-méthyl-2-(3-méthylphénoxy)-éthylique de l'acide 2-(5-chloro-8-yloxy)-acétique,
ester 1-méthyl-2-(4-isopropylphénoxy)-éthylique de l'acide 2-(5-chloroquinol-8-yloxy)-acétique ou
ester 1-méthyl-2-(4-méthylphénoxy)-éthylique de l'acide 2-(5-chloroquinol-8-yloxy)-acétique.

**35.** Agent selon la revendication 34, caractérisé en ce qu'il contient un antidote de la série
ester méthallylique de l'acide 2-(5-chloroquinol-8-yloxy)-acétique,
ester 2-phénoxyéthylique de l'acide 2-(5-chloroquinol-8-yloxy)-acétique,
ester 1-méthylbutylique de l'acide 2-(5-chloroquinol-8-yloxy)-acétique,
ester 1-éthylbutylique de l'acide 2-(5-chloroquinol-8-yloxy)-acétique,
ester 1-méthylisopentylique de l'acide 2-(5-chloroquinol-8-yloxy)-acétique,
ester 1-méthylhexylique de l'acide 2-(5-chloroquinol-8-yloxy)-acétique,
ester 1-éthylpentylique de l'acide 2-(5-chloroquinol-8-yloxy)-acétique,
ester 1-propylbutylique de l'acide 2-(5-chloroquinol-8-yloxy)-acétique,
ester 1-pentylallylique de l'acide 2-(5-chloroquinol-8-yloxy)-acétique,
ester 1-méthylpentylique de l'acide 2-(5-chloroquinol-8-yloxy)-acétique,
ester 1-méthylisohexylique de l'acide 2-(5-chloroquinol-8-yloxy)-acétique,
ester 1-phénylisobutylique de l'acide 2-(5-chloroquinol-8-yloxy)-acétique,
ester 1-phényléthylique de l'acide 2-(5-chloroquinol-8-yloxy)-acétique,
ester 1-méthyl-2-(4-éthylphénoxy)-éthylique de l'acide 2-(5-chloroquinol-8-yloxy)-acétique,

ester 1-méthyl-2-(4-éthylphénoxy)-éthylique de l'acide 2-(5-chloroquinol-8-yloxy)-acétique,
ester 1-méthyl-2-phényléthylique de l'acide 2-(5-chloroquinol-8-yloxy)-acétique,
ester 1-phénylpropylique de l'acide 2-(5-chloroquinol-8-yloxy)-acétique,
ester 1-méthyl-2-phénoxyéthylique de l'acide 2-(5-chloroquinol-8-yloxy)-acétique,
ester 1-méthyl-3-phénylpropylique de l'acide 2-(5-chloroquinol-8-yloxy)-acétique ou ester 1-méthyl-2-(4-méthylphénoxy)-éthylique de l'acide 2-(5-chloroquinol-8-yloxy)-acétique.

**36.** Agent selon la revendication 34, caractérisé en ce qu'il contient comme antidote
l'ester méthallylique de l'acide 2-(5-chloroquinol-8-yloxy)-acétique,
l'ester 2-phénoxyéthylique de l'acide 2-(5-chloroquinol-8-yloxy)-acétique, l'ester 1-méthylisopentylique de l'acide 2-(5-chloroquinol-8-yloxy)-acétique ou
l'ester 1-méthylhexylique de l'acide 2-(5-chloroquinol-8-yloxy)-acétique.

**37.** Composé choisi parmi
l'ester D-1-méthylisopentylique de l'acide 2-(5-chloroquinol-8-yloxy)-acétique,
l'ester L-1-méthylisopentylique de l'acide 2-(5-chloroquinol-8-yloxy)-acétique,
l'ester D-1-méthylhexylique de l'acide 2-(5-chloroquinol-8-yloxy)-acétique,
l'ester L-1-méthylhexylique de l'acide 2-(5-chloroquinol-8-yloxy)-acétique,
l'ester 1-méthylisohexylique de l'acide 2-(5-chloroquinol-8-yloxy)-acétique,
l'ester 1-phénylisobutylique de l'acide 2-(5-chloroquinol-8-yloxy)-acétique, l'ester 1-phényléthylique de l'acide 2-(5-chloroquinol-8-yloxy)-acétique,
l'ester 1-méthyl-2-(4-éthylphénoxy)-éthylique de l'acide 2-(5-chloroquinol-8-yloxy)-acétique,
l'ester 1-méthyl-2-phényléthylique de l'acide 2-(5-chloroquinol-8-yloxy)-acétique,
l'ester 1-phénylpropylique de l'acide 2-(5-chloroquinol-8-yloxy)-acétique,
l'ester 1-méthyl-2-phénoxyéthylique de l'acide 2-(5-chloroquinol-8-yloxy)-acétique,
l'ester 1-méthyl-3-phénylpropylique de l'acide 2-(5-chloroquinol-8-yloxy)-acétique et
l'ester 1-méthyl-2-(4-méthylphénoxy)-éthylique de l'acide 2-(5-chloroquinol-8-yloxy)-acétique.

**38.** Procédé pour la préparation de l'agent selon la revendication 33, caractérisé en ce qu'on mélange intimement les constituants de formule I et II entre eux et éventuellement avec une matière de support ou vectrice et/ou avec un agent de surface.

**39.** Procédé pour la préparation des composés selon la revendication 37, caractérisé en ce qu'on fait réagir de la 5-chloro-8-hydroxyquinoléine en présence d'un agent à liaison acide avec un composé de la série
ester D-1-méthylisopentylique de l'acide bromoacétique,
ester L-1-méthylisopentylique de l'acide bromoacétique,
ester D-1-méthylhexylique de l'acide bromoacétique,
ester L-1-méthylhexylique de l'acide bromoacétique,
ester 1-méthylisohexylique de l'acide bromoacétique,
ester 1-phénylisobutylique de l'acide bromoacétique,
ester 1-phényléthylique de l'acide bromoacétique,
ester 1-méthyl-2-(4-éthylphénoxy)-éthylique de l'acide bromoacétique,
ester 1-méthyl-2-phényléthylique de l'acide bromoacétique,
ester 1-phénylpropylique de l'acide bromoacétique,
ester 1-méthyl-2-phénoxyéthylique de l'acide bromoacétique,
ester 1-méthyl-3-phénylpropylique de l'acide bromoacétique et ester 1-méthyl-2-(4-méthylphénoxy)-éthylique de l'acide bromoacétique.